# EUROPEAN PATENT SPECIFICATION

(11) **EP 4 403 645 B1**
(45) Date of publication and mention of the grant of the patent: **21.10.2026**
(21) Application number: 22870362.5
(22) Date of filing: 16.09.2022
(51) Int. Cl.: C12Q 1/6818

(54) **DETECTION OF TARGET NUCLEIC ACID SEQUENCE BY USING SYNTHETIC NON-NATURAL BASE-BEARING TAG OLIGONUCLEOTIDE**
NACHWEIS EINER ZIELNUKLEINSÄURESEQUENZ UNTER VERWENDUNG EINES SYNTHETISCHEN, EINE NICHTNATÜRLICHE BASE TRAGENDEN MARKEROLIGONUKLEOTIDS
DÉTECTION D'UNE SÉQUENCE D'ACIDE NUCLÉIQUE CIBLE À L'AIDE D'UN OLIGONUCLÉOTIDE MARQUEUR PORTEUR DE BASE NON NATURELLE SYNTHÉTIQUE

(30) Priority: 17.09.2021 KR 20210125068
(43) Date of publication of application: 24.07.2024
(73) Proprietor: Seegene, Inc., Seoul 05548 (KR)
(72) Inventor: PARK, Jihoon, Seoul 05548 (KR); KIM, Soyoung, Seoul 05548 (KR); KIM, Dong-Min, Seoul 05548 (KR); KO, Ohsung, Seoul 05548 (KR); PARK, Yuram, Seoul 05548 (KR)
(74) Representative: Dehns
(86) International application number: PCT/KR2022/013921
(87) International publication number: WO 2023/043280

(56) References cited:
- WO-A2-2004/065550
- CN-A- 104 903 465
- KR-A- 20170 142 128
- KR-B1- 101 462 192
- KR-B1- 101 832 867
- KR-B1- 102 265 313
- US-A1- 2016 312 271

## Description

### Technical Field

The present disclosure relates to detection of a target nucleic acid sequence using a tag oligonucleotide comprising a synthetic unnatural base.

### Background Art

For detection of target nucleic acid sequences, real-time detection methods are widely used to detect target nucleic acid sequences with monitoring target amplification in a real-time manner. The real-time detection methods generally use labeled probes or primers specifically hybridized with target nucleic acid sequences. The exemplified methods by use of hybridization between labeled probes and target nucleic acid sequences include Molecular beacon method using dual-labeled probes with hairpin structure (Tyagi et al, Nature Biotechnology v.14 MARCH 1996), HyBeacon method (French DJ et al., Mol. Cell Probes, 15(6):363-374(2001)), Hybridization probe method using two probes labeled each of donor and acceptor (Bernard et al, 147-148 Clin Chem 2000; 46) and Lux method using single-labeled oligonucleotides (U.S. Pat. No. 7,537,886). TaqMan method (U.S. Pat. Nos. 5,210,015 and 5,538,848) using dual-labeled probes and its cleavage by 5'-nuclease activity of DNA polymerase is also widely employed in the art.

The exemplified methods using labeled primers include Sunrise primer method (Nazarenko et al, 2516-2521 Nucleic Acids Research, 1997, v.25 no.12, and U.S. Pat. No. 6,117,635), Scorpion primer method (Whitcombe et al, 804-807, Nature Biotechnology v.17 AUGUST 1999 and U.S. Pat. No. 6,326,145) and TSG primer method (WO 2011/078441).

As alternative approaches, real-time detection methods using duplexes formed depending on the presence of target nucleic acid sequences have been proposed: Invader assay (U.S. Pat. Nos. 5,691,142, 6,358,691 and 6,194,149), PTOCE (PTO Cleavage and Extension) method (WO 2012/096523), PCE-SH (PTO Cleavage and Extension-Dependent Signaling Oligonucleotide Hybridization) method (WO 2013/115442), PCE-SC (PTO Cleavage and Extension-dependent Signaling Oligonucleotide Cleavage) (WO 2013/157821), PCE-NH (PTO Cleavage and Extension-Dependent Non-Hybridization) method (WO 2014/104818), PCE-IH (PTO Cleavage and Extension-dependent Immobilized Oligonucleotide Hybridization) method (WO 2015/008985), and PTO Cleavage and Extension-dependent extension (PTOCE-E) method (WO 2019/066461). Further PTOCE methods are disclosed by CN 104903465A and US2016/312271A1.

As another method, a real-time detection method using a dual quenching assay combined with a melting curve analysis has been proposed (WO 2016/101959).

The real-time detection method using the duplex and/or the real-time detection method using the dual quenching assay as described above uses a tag oligonucleotide (*e*.*g*., a probing and tagging oligonucleotide (PTO)). The tag oligonucleotide is cleaved dependent on the presence of a target nucleic acid sequence to release a fragment *(e.g.,* a PTO fragment). The method then uses hybridization between the fragment and a fragment-hybridizing oligonucleotide (*e*.*g*., capturing and templating oligonucleotide (CTO), capturing and quenching oligonucleotide (CQO), *etc*.) that can be hybridized with the fragment. The method has the advantage in that the sequences of the fragment and the fragment-hybridizing oligonucleotide can be artificially and freely designed without consideration of a target nucleic acid sequence.

However, since the fragment is a part of the tag oligonucleotide and has a relatively short length compared to other oligonucleotides used in the target nucleic acid detection method, the fragment may exhibit low hybridization efficiency with the fragment-hybridizing oligonucleotide under certain reaction conditions. In addition, although the fragment is designed not to be hybridized with the target nucleic acid sequence as well as the non-target nucleic acid sequence, the fragment may be unexpectedly hybridized with an unknown non-target nucleic acid sequence present in the sample. The low hybridization efficiency between the fragment and the fragment-hybridizing oligonucleotide may eventually lead to a decrease in the signal indicative of the presence of the target nucleic acid sequence, and the hybridization between the fragment and the non-target nucleic acid sequence may also consume the fragment or interfere with the hybridization between the fragment and the fragment-hybridizing oligonucleotide, thereby resulting in a decrease in the signal indicating the presence of the target nucleic acid sequence.

Accordingly, in order to increase the signal indicative of the presence of a target nucleic acid sequence, there is a need to develop a new method that can improve the binding affinity and/or specificity between the released fragment and the fragment-hybridizing oligonucleotide.

### Disclosure of the Invention

### Technical Problem

The present inventors have made intensive efforts to improve signal detection in a method for detecting a target nucleic acid by using a tag oligonucleotide and a fragment-hybridizing oligonucleotide. In particular, the present inventors have tried to improve the signal indicative of the presence of a target nucleic acid sequence by improving the binding affinity or specificity between a target-dependent fragment that is cleaved and released dependent on the presence of the target nucleic acid sequence and the fragment-hybridizing oligonucleotide hybridizable with the target-dependent fragment. As a result, the present inventors have confirmed that binding affinity and/or specificity between the released fragment and the fragment-hybridizing oligonucleotide may be increased by introducing a synthetic unnatural base pair to the fragment-hybridizing oligonucleotide and a tagging portion of the tag oligonucleotide involved in the hybridization between the target-dependent fragment and the fragment-hybridizing oligonucleotide. The protocol of the present disclosure increases the signal indicative of the presence of the target nucleic acid sequence by improving the binding affinity or specificity in the hybridization between the target-dependent fragment and the fragment-hybridizing oligonucleotide, thereby allowing the detection of a plurality of target sequences with more improved sensitivity.

Accordingly, the purpose of the present disclosure is to provide a method for detecting a target nucleic acid sequence.

Another purpose of the present disclosure is to provide a composition for detecting a target nucleic acid sequence.

Other purposes and advantages of the present disclosure will become more apparent by the following detailed description along with the appended claims.

### Solution to Problem

According to an aspect of the present disclosure, there is provided a method for detecting a target nucleic acid sequence, the method including the steps of:
(a) reacting the target nucleic acid sequence with a tag oligonucleotide (TO);
   wherein the TO includes (i) a tagging portion including a non-hybridizing nucleotide sequence with the target nucleic acid sequence and (ii) a targeting portion including a hybridizing nucleotide sequence with the target nucleic acid sequence; wherein the tagging portion includes a first synthetic unnatural base within 5 nucleotides from the 3'-end of the tagging portion; wherein the reacting involves hybridization of the TO with the target nucleic acid sequence and cleavage of the TO depending on the presence of the target nucleic acid sequence to release a target-dependent fragment including the tagging portion or a part of the tagging portion of the TO; wherein the target-dependent fragment includes the first synthetic unnatural base; wherein the cleavage of the TO is performed by an enzyme having nuclease activity;
(b) reacting the target-dependent fragment with a fragment-hybridizing oligonucleotide (FHO) to form a duplex;
   wherein the FHO is (i) a first form of FHO including, in a 3' to 5' direction, a capturing portion having a hybridizing nucleotide sequence with the target-dependent fragment and a templating portion having a non-hybridizing nucleotide sequence with the target-dependent fragment and the targeting portion of the TO or (ii) a second form of FHO including a capturing portion having a hybridizing nucleotide sequence with the target-dependent fragment; wherein the capturing portion of the FHO includes a second synthetic unnatural base capable of base pairing with the first synthetic unnatural base in the target-dependent fragment,
   (i) when the FHO is the first form of FHO, the target-dependent fragment is hybridized with the capturing portion of the FHO and is extended to generate an extended strand including an extended sequence complementary to the templating portion of the FHO, thereby inducing the formation of a first duplex between the extended strand and the FHO, or (ii) when the FHO is the second form of FHO, the target-dependent fragment is hybridized with the capturing portion of FHO to form a second duplex; and
(c) detecting the presence of the extended strand in the first duplex or the presence of the second duplex;
wherein the presence of the extended strand in the first duplex or the presence of the second duplex is indicative of the presence of the target nucleic acid sequence.

The present inventors have studied and made intensive efforts to improve signal detection in a method for detecting a target nucleic acid by using a tag oligonucleotide and a fragment-hybridizing oligonucleotide.

In particular, the present inventors have tried to improve the signal indicative of the presence of a target nucleic acid sequence by improving the binding affinity or specificity between a target-dependent fragment that is cleaved and released dependent on the presence of the target nucleic acid sequence and a fragment-hybridizing oligonucleotide hybridizable with the target-dependent fragment. As a result, the present inventors have confirmed that binding affinity or specificity between the released fragment and the fragment-hybridizing oligonucleotide may be increased by introducing a synthetic unnatural base pair to the fragment-hybridizing oligonucleotide and a tagging portion of the tag oligonucleotide involved in the hybridization between the target-dependent fragment and the fragment-hybridizing oligonucleotide. The protocol of the present disclosure increases the signal indicative of the presence of the target nucleic acid sequence by improving the binding affinity or specificity in the hybridization between the target-dependent fragment and the fragment-hybridizing oligonucleotide, thereby allowing the detection of a plurality of target sequences with more improved accuracy.

According to an embodiment of the present disclosure, the first synthetic unnatural base is located at a site of 2 to 5 nucleotides apart from the 3'-end of the tagging portion of the TO.

According to an embodiment of the present disclosure, the tagging portion of the TO further includes 1 to 5 additional synthetic unnatural bases.

According to an embodiment of the present disclosure, the first synthetic unnatural base and the 1 to 5 additional synthetic unnatural bases are positioned 1 to 10 nucleotides apart from each other.

According to an embodiment of the present disclosure, the first synthetic unnatural base is selected from the group consisting of S, Z, V, K, Pa. Pn, Px, Q, MMO2, 5FM, NaM, B, P, J, X, Ds, Dss and 5SICS.

According to an embodiment of the present disclosure, the first synthetic unnatural base is any base of a pair selected from the group consisting of S-B base pair, Z-P base pair, V-J base pair, K-X base pair, Pa-Ds base pair, Pa-Q base pair, Pn-Ds base pair, Pn-Dss base pair, Px-Ds base pair, MMO2-5SICS base pair, 5FM-5SICS base pair, and NaM-5SICS base pair.

According to an embodiment of the present disclosure, the tagging portion of the TO is 5 to 50 nucleotides in length.

According to an embodiment of the present disclosure, the tagging portion of the TO is 9 to 14 nucleotides in length.

According to an embodiment of the present disclosure, the enzyme having nuclease activity is an enzyme having 5' nuclease activity.

According to an embodiment of the present disclosure, the method is performed in the presence of an upstream primer including a hybridizing nucleotide sequence with the target nucleic acid sequence, and the upstream primer is located upstream the 5' direction of the TO.

According to an embodiment of the present disclosure, the step (a) includes a reaction in which the upstream primer is extended by a template-dependent nucleic acid polymerase to generate an extended strand, and the extended strand induces cleavage of the TO by the enzyme having nuclease activity, thereby releasing the target-dependent fragment including the tagging portion or a part of the tagging portion of the TO.

According to an embodiment of the present disclosure, the template-dependent nucleic acid polymerase is identical to the enzyme having the nuclease activity.

According to an embodiment of the present disclosure, the TO is a probe.

According to an embodiment of the present disclosure, the method is performed in the presence of a downstream primer.

According to an embodiment of the present disclosure, the first duplex provides a detectable signal by (i) at least one label linked to the target-dependent fragment and/or the first form of FHO, (ii) a label incorporated into the first duplex during the extension reaction, (iii) a label incorporated into the first duplex during the extension reaction and at least one label linked to the first form of FHO or (iv) an intercalating label, and the presence of the extended strand in step (c) is detected by measuring a signal provided from the first duplex.

According to an embodiment of the present disclosure, the first duplex includes a cleavage site to be cleaved by a nucleolytic enzyme, the first duplex provides a detectable signal by cleavage at the cleavage site, and the presence of the extended strand in step (c) is detected by measuring a signal provided from cleavage of the first duplex.

According to an embodiment of the present disclosure, the method additionally uses a signaling oligonucleotide (SO) including a hybridizing nucleotide sequence with the extended strand and a label, a duplex between the SO and the extended strand provides a detectable signal and the presence of the extended strand in step (c) is detected by measuring a signal provided from the duplex between the SO and the extended strand.

According to an embodiment of the present disclosure, the method additionally uses a signaling oligonucleotide (SO) including a hybridizing nucleotide sequence with the extended strand and a label, a duplex between the SO and the extended strand includes a cleavage site to be cleaved by a nucleolytic enzyme, the duplex between the SO and the extended strand provides a detectable signal by cleavage at the cleavage site, and the presence of the extended strand in step (c) is detected by measuring a signal provided from cleavage of the duplex between the SO and the extended strand.

According to an embodiment of the present disclosure, the method additionally uses a hybridizing oligonucleotide (HO) including a hybridizing nucleotide sequence with the first form of FHO and a label, a duplex between the HO and the first form of FHO provides a detectable signal and the presence of the extended strand in step (c) is detected by measuring a signal provided from the duplex between the HO and the first form of FHO, the presence of the signal from the duplex between the HO and the first form of FHO indicates the absence of the extended strand.

According to an embodiment of the present disclosure, the method additionally uses an immobilizing oligonucleotide (IO) immobilized on a solid substrate, which includes a hybridizing nucleotide sequence with the extended strand, the extended strand includes a label, a duplex between the extended strand and the IO provides a detectable signal on the solid substrate, and the presence of the extended strand in step (c) is detected by measuring a signal provided from the duplex between the extended strand and the IO.

According to an embodiment of the present disclosure, step (c) includes the steps of: (c-1) hybridizing the extended strand with a third form of FHO, wherein the third form of FHO includes in a 3' to 5' direction (i) a capturing portion including a hybridizing nucleotide sequence with the extended strand and (ii) a templating portion including a non-hybridizing nucleotide sequence with the extended strand, and the extended strand is hybridized with the capturing portion of the third form of FHO; (c-2) performing an extension reaction using the resultant of the step (c-1) and a template-dependent nucleic acid polymerase, wherein the extended strand hybridized with the capturing portion of the third form of FHO is further extended to generate a second extended strand including a second extended sequence complementary to the templating portion of the third form of FHO, thereby forming a third duplex between the second extended strand and the third form of FHO; and (c-3) detecting the presence of the second extended strand, whereby the presence of the second extended strand indicates the presence of the extended strand.

According to an embodiment of the present disclosure, the first or second form of FHO is immobilized on the solid substrate.

According to an embodiment of the present disclosure, the method uses additional SO, HO or third form of FHO immobilized on the solid substrate.

According to an embodiment of the present disclosure, the TO and/or the FHO is blocked at its 3'-end to prohibit its extension.

According to an embodiment of the present disclosure, when the method forms the second duplex, a reporter molecule and a first quencher molecule are linked to the TO, and a second quencher molecule is linked to the second form of FHO, the TO is cleaved depending on the presence of the target nucleic acid sequence during the step (a) to release a target-dependent fragment including the reporter molecule, the reporter molecule included in the target-dependent fragment and the second quencher molecule linked to the second form of FHO provide a signal detectable by the formation and dissociation of a second duplex between the target-dependent fragment and the second form of FHO depending on temperature, and the presence of the second duplex in step (c) is detected by measuring a signal provided from the second duplex.

According to an embodiment of the present disclosure, a Tm value of the first or second duplex is 0.5 to 10°C higher than a Tm value of a duplex in which the first and second synthetic unnatural bases included in the first or second duplex are substituted with natural bases.

According to an embodiment of the present disclosure, all or some of the steps (a)-(c) are repeated with denaturation.

According to another aspect of the present disclosure, there is provided a composition for detecting a target nucleic acid sequence, the composition including:
(a) a tag oligonucleotide (TO);
   wherein the TO includes (i) a tagging portion including a non-hybridizing nucleotide sequence with the target nucleic acid sequence and (ii) a targeting portion including a hybridizing nucleotide sequence with the target nucleic acid sequence; wherein the tagging portion includes a first synthetic unnatural base within 5 nucleotides from the 3'-end of the tagging portion; the TO is cleaved depending on the presence of the target nucleic acid sequence to release a target-dependent fragment including the tagging portion or a part of the tagging portion of the TO; wherein the target-dependent fragment includes the first synthetic unnatural base;
(b) a fragment-hybridizing oligonucleotide (FHO);
   wherein the FHO is (i) a first form of FHO including, in a 3' to 5' direction, a capturing portion having a hybridizing nucleotide sequence with the target-dependent fragment and a templating portion having a non-hybridizing nucleotide sequence with the target-dependent fragment and the targeting portion of the TO or (ii) a second form of FHO including a capturing portion having a hybridizing nucleotide sequence with the target-dependent fragment; wherein the capturing portion of the FHO includes a second synthetic unnatural base capable of base pairing with the first synthetic unnatural base in the target-dependent fragment; and
(c) an enzyme having nuclease activity.

According to an embodiment of the present disclosure, the composition further includes an upstream primer including a hybridizing nucleotide sequence with the target nucleic acid sequence, and the upstream primer is located upstream the 5' direction of the TO.

According to an embodiment of the present disclosure, the composition further includes a template-dependent nucleic acid polymerase.

According to an embodiment of the present disclosure, the composition further includes a downstream primer.

### Advantageous Effects of Invention

The features and advantages of the present disclosure are summarized as follows:
(a) According to the present disclosure, when the TO and FHO comprising an unnatural base pair in the tagging portion of the TO and the capturing portion of the FHO are used, a hybrid (duplex) between the target-dependent fragment and the FHO has a high Tm and improved binding affinity. This can increase the extension efficiency of the target-dependent fragment hybridized with the capturing portion of the FHO, resulting in an increase in the signal indicative of the presence of the target nucleic acid sequence.
(b) According to the present disclosure, when the TO and FHO comprising an unnatural base pair in the tagging portion of the TO and the capturing portion of the FHO are used, the binding specificity of the duplex between the target-dependent fragment and the FHO is improved. This can prevent the target-dependent fragment from hybridizing with the non-target nucleic acid sequence, resulting in an increase in the signal indicative of the presence of the target nucleic acid sequence.
(c) According to the present disclosure, when an unnatural base pair is included in the tagging portion of the TO and the capturing portion of the FHO, the tagging portion of the TO can have a reduced length while maintaining its Tm. The tagging portion of the TO having the reduced length maintains the binding affinity with the capturing portion of the FHO, and when hybridizing with the non-target nucleic acid sequence, the Tm and binding affinity of the tagging portion of the TO is greatly reduced, and accordingly, the target-dependent fragment is prevented from hybridizing with the non-target nucleic acid sequence, resulting in an increase in the signal indicative of the presence of the target nucleic acid sequence.
(d) Conventional method using the TO not comprising an unnatural base upon detection of a plurality of target nucleic acid sequences, had difficulty in ensuring that tagging portions of the plurality of TOs are non-complementariy to all target nucleic acid sequences due to the diversity of the plurality of target nucleic acid sequences. However, the method of the present disclosure has the advantage of ensuring non-complementarity of tagging portion of TOs to all target nucleic acid sequences, regardless of their sequences, by introducing an unnatural base pair to the tagging portion of the TO and the capturing portion of the FHO.

### Brief Description of Drawings

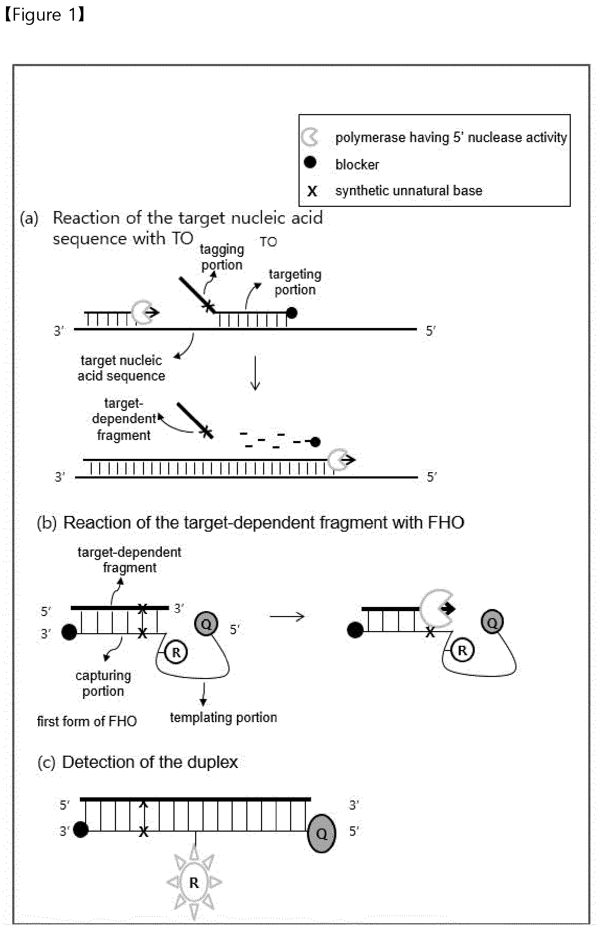

FIG. 1 schematically shows an example of a method for detecting a target nucleic acid by using a first form of FHO and a TO comprising a synthetic unnatural base pair.

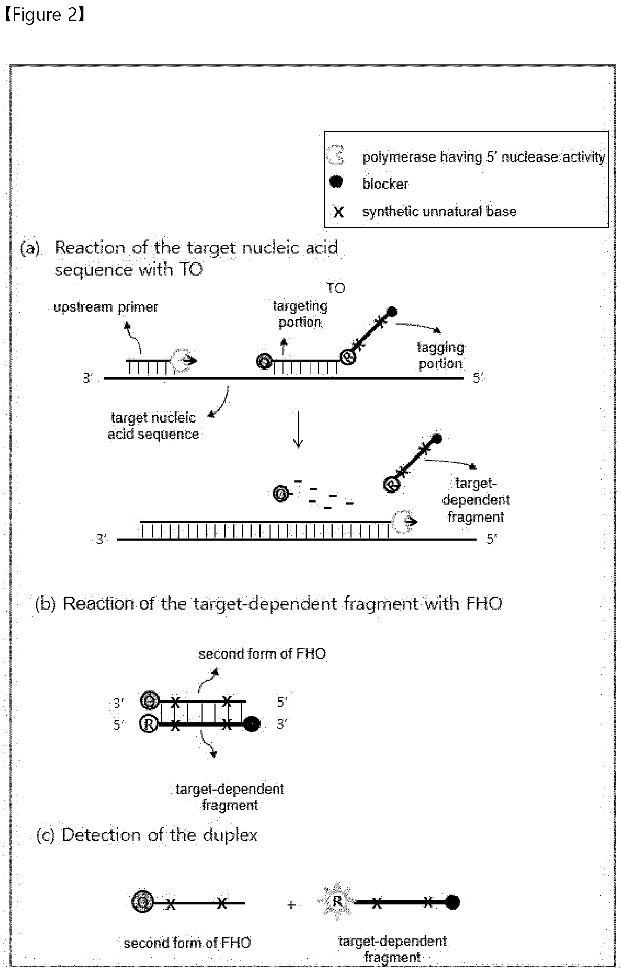

FIG. 2 schematically shows an example of a method for detecting a target nucleic acid by using a second form of FHO and a TO comprising a synthetic unnatural base pair.
FIGS. 3 and 4 show the results of real-time PCR of Example 2.

### Best Mode for Carrying out the Invention

The present inventors have tried to improve the signal indicative of the presence of a target nucleic acid sequence by improving the binding affinity or specificity between a target-dependent fragment that is cleaved and released dependent on the presence of the target nucleic acid sequence and a fragment-hybridizing oligonucleotide hybridizable with the target-dependent fragment. As a result, the present inventors have confirmed that binding affinity or specificity between the released fragment and the fragment-hybridizing oligonucleotide may be increased by introducing a synthetic unnatural base pair to the fragment-hybridizing oligonucleotide and a tagging portion of the tag oligonucleotide involved in the hybridization between the target-dependent fragment and the fragment-hybridizing oligonucleotide.

In an aspect of the present disclosure, there is provided a method for detecting a target nucleic acid sequence, comprising the steps of:
(a) reacting the target nucleic acid sequence with a tag oligonucleotide (TO);
   wherein the TO comprises (i) a tagging portion comprising a non-hybridizing nucleotide sequence with the target nucleic acid sequence and (ii) a targeting portion comprising a hybridizing nucleotide sequence with the target nucleic acid sequence;
   wherein the tagging portion comprises a first synthetic unnatural base within 5 nucleotides from the 3'-end of the tagging portion;
   wherein the reacting involves hybridization of the TO with the target nucleic acid sequence and cleavage of the TO depending on the presence of the target nucleic acid sequence to release a target-dependent fragment comprising the tagging portion or a part of the tagging portion of the TO;
   wherein the target-dependent fragment comprises the first synthetic unnatural base;
   wherein the cleavage of the TO is performed by an enzyme having nuclease activity;
(b) reacting the target-dependent fragment with a fragment-hybridizing oligonucleotide (FHO) to form a duplex;
   wherein the FHO is (i) a first form of FHO comprising, in a 3' to 5' direction, a capturing portion having a hybridizing nucleotide sequence with the target-dependent fragment and a templating portion having a non-hybridizing nucleotide sequence with the target-dependent fragment and the targeting portion of the TO or (ii) a second form of FHO comprising a capturing portion having a hybridizing nucleotide sequence with the target-dependent fragment;
   wherein the capturing portion of the FHO comprises a second synthetic unnatural base capable of base pairing with the first synthetic unnatural base in the target-dependent fragment,

   (i) when the FHO is the first form of FHO, the target-dependent fragment is hybridized with the capturing portion of the FHO and is extended to generatean extended strand comprising an extension sequence complementary to the templating portion of the FHO, thereby inducing the formation of a first duplex between the extended strand and the FHO, or
   (ii) when the FHO is the second form of FHO, the target-dependent fragment is hybridized with the capturing portion of FHO to form a second duplex; and
(c) detecting the presence of the extended strand in the first duplex or the presence of the second duplex;
wherein the presence of the extended strand in the first duplex or the presence of the second duplex is indicative of the presence of the target nucleic acid sequence.

In the description of the components of the present disclosure, the terms, such as first, second, A, B, (a), (b), (i), and (ii) may be used herein. These terms are merely used to distinguish one component from other components, and the nature, an order, a sequence, or the like of the corresponding component are not limited by the terms.

The present disclosure will be described in detail according to each step as follows:

### Step (a): Reaction of Target Nucleic Acid Sequence with Tag Oligonucleotide

According to the present disclosure, a target nucleic acid sequence is first reacted with a tag oligonucleotide (TO).

As used herein, the term "target nucleic acid," "target nucleic acid sequence," or "target sequence" refers to a sequence to be finally detected, and is annealed or hybridized with a primer or probe under specific hybridizing, annealing, or amplifying conditions.

As used herein, the term "probe" refers to a single-stranded nucleic acid molecule comprising a hybridizing portion(s) to a target nucleic acid sequence.

As used herein, the term "primer" may refer to an oligonucleotide, which acts as an initiation point of synthesis under conditions in which the synthesis of a primer extension product complementary to a target nucleic acid sequence (template) is induced, *i*.*e*., under the presence of nucleotides and an agent for polymerization such as a DNA polymerase, as well as a suitable temperature and pH.

In particular, the probe and primer are single-stranded deoxyribonucleotide molecules. The probe or primer used in the present disclosure may comprise naturally occurring dNMPs *(i.e.,* dAMPs, dGMPs, dCMPs, and dTMPs), modified nucleotides, or unnatural nucleotides. In addition, the probe or primer may also comprise ribonucleotides.

The primer should have a length sufficient to prime the synthesis of the extension product in the presence of an agent for polymerization. The suitable length of the primer is determined according to various factors, such as temperature, application field, and a source of the primer. The term "annealing" or "priming" refers to the apposition of an oligodeoxynucleotide or a nucleic acid to a template nucleic acid, and the apposition allows the polymerase to polymerize the nucleotides to generate a nucleic acid molecule complementary to the template nucleic acid or a part thereof.

As used herein, the term "hybridization" refers to the formation of a double-stranded polynucleotide from two single-stranded polynucleotides through non-covalent binding between complementary nucleotide sequences under predetermined hybridization conditions or stringent conditions.

In particular, the expression herein that one oligonucleotide "comprises a hybridizing nucleotide sequence" with another oligonucleotide means that all or a part of the one oligonucleotide comprises a complementary nucleotide sequence necessary for hybridizing with all or a part of the other oligonucleotide.

In addition, when referring to hybridization of a portion in one oligonucleotide with another oligonucleotide herein, the portion in one oligonucleotide may be regarded as an individual oligonucleotide to express hybridization between different oligonucleotides.

The hybridization may occur when two nucleic acid sequences are perfectly complementary (perfect matched) or substantially complementary to each other with some mismatches (e.g., 1-4 mismatches) at a hybridization occurrence portion (a double-strand formation portion). The degree of complementarity required for hybridization may vary depending on the hybridization reaction conditions, and may be controlled by, particularly, temperature.

The terms "annealing" and "hybridization" are not different from each other, and are used interchangeably in the present specification.

As used herein, the term "complementary" means that the primer or probe is sufficiently complementary to selectively hybridize with a target nucleic acid sequence under predetermined annealing or stringent conditions, and the term encompasses both "substantially complementary" and "perfectly complementary," and specifically, means perfectly complementary.

On the other hand, the term "non-complementary" as used herein means that the primer or probe is sufficiently non-complementary so that it is not selectively hybridized with a specific sequence under predetermined annealing or stringent conditions, and the term encompasses both "substantially non-complementary" and "perfectly non-complementary," and specifically, means perfectly non-complementary.

The TO comprises (i) a tagging portion comprising a non-hybridizing nucleotide sequence with the target nucleic acid sequence and (ii) a targeting portion comprising a hybridizing nucleotide sequence with the target nucleic acid sequence.

The "tagging portion" comprising a nucleotide sequence that does not hybridize with a target nucleic acid sequence is also called a "tag sequence," a "flap sequence," or a "tail sequence." The tagging portion of the TO may have an arbitrary sequence as long as it does not hybridize with the target nucleic acid sequence.

In an embodiment, the tagging portion of the TO may be located at the 5'-end, the 3'-end, or the center of the targeting portion of the TO. In a specific embodiment, the tagging portion of the TO may be located at the 5'-end of the targeting portion of the TO.

In an embodiment, the tagging portion of the TO may comprise one or more synthetic unnatural bases that exhibit binding affinity different from a Watson-Crick base-pairing that is generated between naturally occurring bases such as guanine (G), cytosine (C) adenine (A), thymine (T), and uracil (U).

In an embodiment, the tagging portion of the TO comprises a first synthetic unnatural base within 5 nucleotides from the 3'-end of the tagging portion.

In an embodiment, the first synthetic unnatural base may be selected from the group consisting of S, Z, V, K, Pa. Pn, Px, Q, MMO2, 5FM, NaM, B, P, J, X, Ds, Dss and 5SICS, but is not limited thereto.

In an embodiment, the first synthetic unnatural base may be located within 5 nucleotides from the 3'-end of the tagging portion (specifically, the 5'-tagging portion) of the TO. Specifically, the first synthetic unnatural base may be located within 5 nucleotides, within 4 nucleotides, within 3 nucleotides, or within 2 nucleotides from the 3'-end of the tagging portion of the TO.

In an embodiment, the first synthetic unnatural base is located at a site of 2 to 5 nucleotides apart from the 3'-end of the tagging portion of the TO. For example, when the tagging portion of the TO is composed of n nucleotides, a site 2 nucleotides apart from the 3'-end of the tagging portion of the TO indicates an *n*-1^{th} nucleotide in the 5' to 3' direction in the tagging portion, and a site 5 nucleotides apart from the 3'-end of the tagging portion of the TO indicates an *n*-4^{th} nucleotide.

The present inventors have found that including the synthetic unnatural base at the site of the first nucleotide from the 3'-end of the tagging portion (specifically, the 5'-tagging portion) of the TO(*i.e*., the *n*^{th} nucleotide in the 5' to 3' direction in the tagging portion consisting of the n nucleotides) leads to reduced sensitivity in the detection of step (c), in particular, reduced sensitivity in the detection of the extended strand in the first duplex. This finding may be due to, when the synthetic unnatural base is present at the site of the first nucleotide from the 3'-end, (i) a phenomenon that the cleavage efficiency is reduced due to the unnatural base located at the cleavage site of nuclease, (ii) a phenomenon that the extension efficiency is reduced because the unnatural base located at the 3'-end of the target-dependent fragment hybridized with the FHO, *i.e.,* at the initiation site of the extension of the polymerase is not easily recognized as a general substrate in the extension reaction of the polymerase, and (iii) a phenomenon that the synthetic unnatural base at the site of the first nucleotide from the 3'-end exhibits stronger repulsive force against the opposite natural base in the target nucleic acid sequence compared to the repulsive force between natural bases, and thus the 5'-end of the targeting portion is also separated from the target nucleic acid sequence together with the 3'-tagging portion *(i.e.,* the synthetic unnatural base at the site of the first nucleotide from the 3'-end is not hybridized with the target nucleic acid sequence), therefore, the TO cleavage site by the enzyme having nuclease activity is changed or the sequence at the 3'-end of the target-dependent fragment is changed, so that the extension efficiency of the target-dependent fragment hybridized with the FHO is reduced.

In an embodiment, the first synthetic unnatural base may be located at a second nucleotide to a fifth nucleotide, a second nucleotide to a fourth nucleotide, or a second nucleotide to a third nucleotide from the 3'-end of the tagging portion of the TO.

As used herein, the term "unnatural base" refers to derivatives of natural bases such as adenine (A), guanine (G), thymine (T), cytosine (C), and uracil (U) capable of forming base pairs. As used herein, the term "unnatural base" comprises a base having a base pair pattern different from a natural base as a mother compound, and is described in, for example, U.S. Pat. Nos. 5,432,272, 5,965,364, 6,001,983, and 6,037,120. Base pairs between the unnatural bases may contain two or three hydrogen bonds, similar to natural bases. In addition, the base pairs between the unnatural bases are formed in a specific manner.

Specific examples of the unnatural bases comprise base pair combinations of the following bases: iso-C/iso-G, iso-dC/iso-dG, K/X, H/J, M/N and 5FM-5SICS (*see* U.S. Pat. No. 7,422,850).

In an embodiment, the tagging portion of the TO may further comprise 1 to 5 synthetic unnatural bases, and specifically, may further comprise 1, 2, 3, 4, or 5 synthetic unnatural bases.

In an embodiment, when the tagging portion of the TO comprises additional synthetic unnatural bases in addition to the first synthetic unnatural base, the additional synthetic unnatural bases are located at sites other than the first nucleotide from the 3'-end of the tagging portion. For example, when the tagging portion of the TO consists of *n* nucleotides, the additional synthetic unnatural base may not be located at the site of the *n*^{th} nucleotide in the 5' to 3' direction in the tagging portion.

In an embodiment, when the tagging portion of the TO comprises additional synthetic unnatural bases in addition to the first synthetic unnatural base, the first synthetic unnatural base and the additional synthetic unnatural bases may be continuously located.

In an embodiment, when the tagging portion of the TO comprises additional synthetic unnatural bases in addition to the first synthetic unnatural base, the first synthetic unnatural base and the additional synthetic unnatural bases may be spaced apart from each other by 1 to 20 nucleotides, specifically, by 1 to 15 nucleotides, 1 to 10 nucleotides, 1 to 5 nucleotides, 2 to 15 nucleotides, 2 to 10 nucleotides, or 2 to 5 nucleotides, and more specifically, by 1 nucleotide, 2 nucleotides, 3 nucleotides, 4 nucleotides, or 5 nucleotides.

In an embodiment, all or some of the first synthetic unnatural base and the additional synthetic unnatural bases may be the same. For example, the first synthetic unnatural base and the additional synthetic unnatural bases may all be the same unnatural base. As another example, some of the first synthetic unnatural base and the additional synthetic unnatural bases may be the same kind of unnatural base (*e.g.,* S) and the others may be different kinds of unnatural base (*e.g.,* Z).

In an embodiment, the first synthetic unnatural base and the additional synthetic unnatural bases may all be different.

The TO does not require any specific length. For example, the length of the TO may be 15 to 150 nucleotides, 15 to 100 nucleotides, 15 to 80 nucleotides, 15 to 60 nucleotides, 15 to 40 nucleotides, 20 to 150 nucleotides, 20 to 100 nucleotides, 20 to 80 nucleotides, 20 to 60 nucleotides, 20 to 50 nucleotides, 30 to 150 nucleotides, 30 to 100 nucleotides, 30 to 80 nucleotides, 30 to 60 nucleotides, 30 to 50 nucleotides, 35 to 100 nucleotides, 35 to 80 nucleotides, 35 to 60 nucleotides, or 35 to 50 nucleotides. The targeting portion of the TO may have any length as long as it is specifically hybridized with the target nucleic acid sequence. For example, the targeting portion of the TO may be 10 to 100 nucleotides in length, 10 to 80 nucleotides, 10 to 50 nucleotides, 10 to 40 nucleotides, 10 to 30 nucleotides, 15 to 100 nucleotides, 15 to 80 nucleotides, 15 to 50 nucleotides, 15 to 40 nucleotides, 15 to 30 nucleotides, 20 to 100 nucleotides, 20 to 80 nucleotides, 20 to 50 nucleotides, 20 to 40 nucleotides, or 20 to 30 nucleotides. The tagging portion may have any length as long as it is specifically hybridized with the capturing portion of the FHO and/or extended. For example, the tagging portion of the TO may be 5 to 50 nucleotides in length, 5 to 40 nucleotides, 5 to 30 nucleotides, 5 to 20 nucleotides, 5 to 15 nucleotides, 10 to 50 nucleotides, 10 to 40 nucleotides, 10 to 30 nucleotides, 10 to 20 nucleotides, 10 to 15 nucleotides, 15 to 50 nucleotides, 15 to 40 nucleotides, 15 to 30 nucleotides, or 15 to 20 nucleotides.

In a specific embodiment, the tagging portion of the TO is 9 to 14 nucleotides in length.

In an embodiment, the TO may be a primer or a probe, and in a specific embodiment, the TO may be a probe.

In an embodiment, the 3'-end of the TO may have a 3'-OH terminal.

In an embodiment, the 3'-end of the TO may be "blocked" and its extension may be prevented.

The blocking may be achieved according to a conventional method. For example, the blocking may be performed by adding a chemical moiety such as biotin, label, a phosphate group, an alkyl group, a non-nucleotide linker, a phosphorothioate or alkane-diol residue to the 3'-hydroxyl group of the last nucleotide. Alternatively, the blocking may be performed by removing the 3'-hydroxyl group of the last nucleotide or by using a nucleotide without the 3'-hydroxyl group, such as dideoxynucleotide.

In an embodiment, the reaction in step (a) involves hybridization of the TO with the target nucleic acid sequence and cleavage of the TO depending on the presence of the target nucleic acid sequence to release a target-dependent fragment comprising the tagging portion or a part of the tagging portion of the TO. In a specific embodiment, the reaction in step (a) involves a reaction in which the TO is hybridized with the target nucleic acid sequence, and then the TO is cleaved depending on the presence of the target nucleic acid sequence to release a target-dependent fragment comprising the tagging portion or a part of the tagging portion of the TO. In particular, the hybridization of the target nucleic acid sequence and the TO may be performed under stringent conditions in which the targeting portion of the TO is hybridized with the target nucleic acid sequence and the tagging portion is not hybridized with the target nucleic acid sequence.

In an embodiment, the cleavage of the TO may be performed by an enzyme having nuclease activity. For example, the cleavage of the TO may be performed by using a polymerase having nuclease activity or may be performed by using a separate nuclease, but is not limited thereto. The nuclease may be a naturally occurring, unmodified, or modified nuclease.

In an embodiment, the TO hybridized with the target nucleic acid sequence is cleaved by an enzyme having nuclease activity under conditions for the cleavage of the TO to release a target-dependent fragment comprising the tagging portion or a part of the tagging portion of the TO.

As used herein, the term "conditions for the cleavage of the TO" refers to conditions sufficient for the cleavage of the TO hybridized with the target nucleic acid sequence by an enzyme having nuclease activity, such as temperature, pH, ionic strength, buffer, length and sequence of an oligonucleotide, and an enzyme. For example, when the Taq DNA polymerase is used as an enzyme having 5' nuclease activity, conditions for the cleavage of the TO comprise Tris-HCl buffer, KCl, MgCl₂, and temperature.

When the TO is hybridized with the target nucleic acid sequence, the targeting portion of the TO is involved in the hybridization, but the tagging portion of the TO is not hybridized with the target nucleic acid sequence and forms a single strand. As such, the oligonucleotide comprising both single-stranded and double-stranded structures may be cleaved by using an enzyme having nuclease activity by means of various techniques known in the art. Examples of the enzymes include, for example, 5'-nuclease and 3'-nuclease, in particular, nucleic acid polymerase having 5'-nuclease activity, nucleic acid polymerase having 3'-nuclease activity, or FEN nuclease.

In an embodiment, the enzyme having nuclease activity may be an enzyme having 5'-nuclease activity. For example, it may be a DNA polymerase having 5'-nuclease activity or FEN nuclease, specifically, a thermostable DNA polymerase having 5'-nuclease activity or FEN nuclease.

In an embodiment, the DNA polymerase having 5' nuclease activity is a thermostable DNA polymerase obtained from a variety of bacterial species, including *Thermus aquaticus* (Taq), *Thermus thermophilus* (Tth), *Thermus filiformis, Thermis flavus, Thermococcus literalis, Thermus antranikianii, Thermus caldophilus, Thermus chliarophilus, Thermus flavus, Thermus igniterrae, Thermus lacteus, Thermus oshimai, Thermus ruber, Thermus rubens, Thermus scotoductus, Thermus silvanus, Thermus species Z05, Thermus species sps 17, Thermus thermophilus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosiphoafricanus, Pyrococcus woesei, Pyrococcus horikoshii, Pyrococcus abyssi, Pyrodictium occultum, Aquifex pyrophilus,* and *Aquifex aeolieus.* Specifically, the thermostable DNA polymerase is a Taq polymerase.

Alternatively, the present disclosure may use a DNA polymerase having 5'-nuclease activity that has been modified to have less polymerase activity.

In an embodiment, the flap endonuclease (FEN nuclease) may be a 5' flap-specific nuclease. For example, the FEN nuclease includes an FEN nuclease obtained from various bacterial species, including *Sulfolobus solfataricus, Pyrobaculum aerophilum, Thermococcus litoralis, Archaeaglobus veneficus, Archaeaglobus profundus, Acidianus brierlyi, Acidianus ambivalens, Desulfurococcus amylolyticus, Desulfurococcus mobilis, Pyrodictium brockii, Thermococcus gorgonarius, Thermococcus zilligii, Methanopyrus kandleri, Methanococcus igneus, Pyrococcus horikoshii, Aeropyrum pernix* and *Archaeaglobus veneficus.*

In an embodiment, step (a) may be performed in the presence of an upstream oligonucleotide comprising a hybridizing nucleotide sequence with the target nucleic acid sequence. The upstream oligonucleotide is positioned upstream in a 5' direction relative to the TO.

The upstream oligonucleotide used in the present disclosure refers to an oligonucleotide located upstream on the basis of the TO. When the target nucleic acid sequence is a double strand, the upstream oligonucleotide and the TO are hybridized with one strand of the double strand, and the TO is located downstream of the upstream oligonucleotide. The upstream oligonucleotide is hybridized with a particular portion in the 3'-direction compared to a portion of the target nucleic acid strand with which the TO is hybridized.

In an embodiment, step (a) may comprise a reaction of hybridizing the upstream oligonucleotide with the target nucleic acid sequence.

In an embodiment, step (a) may comprise a reaction in which the upstream oligonucleotide induces the cleavage of the TO by the enzyme having nuclease activity to release the target-dependent fragment comprising the tagging portion or a part of the tagging portion of the TO.

The induction of the TO cleavage by the upstream oligonucleotide may be achieved in two ways: (i) upstream oligonucleotide extension-independent cleavage induction; and (ii) upstream oligonucleotide extension-dependent cleavage induction.

When the upstream oligonucleotide is located adjacent to the TO such that it is sufficient induce the cleavage reaction of the TO by an enzyme having 5' nuclease activity, the enzyme that binds to the upstream oligonucleotide cleaves the TO without an extended reaction. On the other hand, when the upstream oligonucleotide is located away from the TO, an enzyme having polymerase activity (*e*.*g*., a template-dependent polymerase) promotes the extension of the upstream oligonucleotide (*e*.*g*., an upstream primer), and an enzyme having 5' nuclease activity bound to the extension product cleaves the TO.

Accordingly, the upstream oligonucleotide may be located relatively with respect to the TO in two ways. The upstream oligonucleotide may be located adjacent to the TO such that it is sufficient induce the cleavage of the TO in an extension-independent manner. Alternatively, the upstream oligonucleotide may be located away from the TO such that it is sufficient induce the TO cleavage in an extension-dependent manner.

The term "adjacent" used herein to refer to positions or locations of an upstream oligonucleotide means that the upstream oligonucleotide is located immediately near the targeting portion of the TO to form a nick. The term also means that the upstream oligonucleotide is located 1 to 30 nucleotides, 1 to 20 nucleotides or 1 to 15 nucleotides apart from the targeting portion of the TO.

The term "located away from (distant)" used herein to refer to a positions or locations of an upstream oligonucleotide comprises any positions or locations sufficient to ensure an extension reaction.

In an embodiment, the upstream oligonucleotide may be an upstream primer or an upstream probe. The upstream primer is suitable for extension-independent cleavage induction or extension-dependent cleavage, and the upstream probe is suitable for extension-independent cleavage induction. In particular, when the upstream oligonucleotide is an upstream primer, it may also be referred to as a forward primer.

In an embodiment, when the cleavage of the TO is induced in an extension-dependent manner of the upstream oligonucleotide, step (a) may comprise a reaction in which the upstream oligonucleotide is hybridized with the target nucleic acid sequence and is extended to produce an extended strand thereof. The extended strand of the upstream oligonucleotide may induce the cleavage of the TO by the enzyme having nuclease activity to release the target-dependent fragment comprising the tagging portion or a part of the tagging portion of the TO. The reaction of generating an extended strand of the upstream oligonucleotide may be carried out by a template-dependent nucleic acid polymerase.

The hybridization of the TO and the upstream oligonucleotide with the target nucleic acid sequence may be carried out under suitable hybridization conditions that are generally determined by an optimization procedure. The conditions such as temperature, concentration of components, number of hybridization and washing, buffer components, their pH and ionic strength may vary depending on various factors including the length of oligonucleotide (upstream oligonucleotide and TO), GC content, and a target nucleotide sequence. For example, when a relatively short oligonucleotide is used, it is preferable to select a low stringent condition. Detailed conditions for hybridization can be found in Joseph Sambrook, et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999).

In an embodiment, when the TO comprises a 5'-tagging portion and a 3'-targeting portion, optionally, the upstream oligonucleotide may have a partial-overlapped sequence with a 5'-part of the 3'-targeting portion of the TO. For example, the overlapped sequence is 1 to 10 nucleotides in length, specifically 1 to 5 nucleotides, and more specifically 1 to 3 nucleotides. When the upstream oligonucleotide has a partial-overlapped sequence with a 5'-part of the 3'-targeting portion of the TO, the 3'-targeting portion of the TO is partially cleaved with the 5'-tagging portion in the cleavage reaction. In addition, the overlapped sequence ensures that the 3'-targeting portion is cleaved at a specific desired site.

According to an embodiment, the upstream primer induces the cleavage of the TO by an enzyme having 5' nuclease activity through its extended strand.

As long as the upstream oligonucleotide induces the cleavage of the TO hybridized with the target nucleic acid sequence to release a target-dependent fragment comprising the tagging portion or a part of the tagging portion of the TO, conventional techniques related to the cleavage by the upstream oligonucleotide may be applied to the present disclosure. For example, U.S. Pat. Nos. 5,210,015, 5,487,972, 5,691,142, 5,994,069, 7,381,532, and U.S. Patent Application Publication No. 2008-0241838 may be applied to the present invention.

The cleavage site of the TO varies depending on the type of upstream oligonucleotide (upstream probe or upstream primer), the hybridization location of the upstream oligonucleotide, and the cleavage conditions (*see* U.S. Pat. No. 5,210,015, 5,487,972, 5,691,142, 5,994,069 and 7,381,532, or U.S. Patent Application Publication No. 2008-0241838).

In an embodiment, the cleavage site of the TO may vary depending on the location of the tagging portion, for example, whether the tagging portion is located at the 5'-, 3'-, or the center of the targeting portion. For example, when the TO comprises the 5'-tagging portion and 3'-targeting portion, there may be three cleavage sites in the cleavage reaction. The first cleavage site is a junction site between the hybridization portion (3'-targeting portion) and the non-hybridization portion (5'-tagging portion) of the TO. The second cleavage site is a site located several nucleotides in the 3'-direction apart from the 3'-end of the tagging portion of the TO. The second cleavage site may be located at a part of the 5'-end of the 3'-targeting portion of the TO. The third cleavage site is a site located several nucleotides in the 5'-direction apart from the 3'-end of the 5'-tagging portion of the TO. As another example, when the TO comprises the 5'-targeting portion and the 3'-tagging portion, the cleavage site may be (i) a junction site between the hybridization portion (5'-targeting portion) and the non-hybridization portion (3'-tagging portion) of the TO, (ii) a site located several nucleotides in the 5'-direction apart from the 5'-end of the tagging portion of the TO (i.e., a site at a part of the 3'-end of the 5'-targeting portion of the TO), and/or (iii) a site located several nucleotides in the 3'-direction apart from the 5'-end of the 3'-tagging portion of the TO.

In an embodiment, the method may be carried out in the presence of a downstream primer. The downstream primer may also be referred to as a reverse primer. The downstream primer is hybridized with another nucleic acid strand complementary to the target nucleic acid strand with which the upstream primer and the TO are hybridized. In particular, the downstream primer is hybridized with a particular portion in the 3'-direction compared to a portion of another nucleic acid strand complementary to a portion of the target nucleic acid strand with which the TO is hybridized. The downstream primer further generates a target nucleic acid sequence with which the TO can be hybridized to improve the detection sensitivity of the present invention.

In an embodiment, when the upstream primer and the downstream primer are used, a template-dependent nucleic acid polymerase may be further used to extend the primer.

In an embodiment, the template-dependent nucleic acid polymerase may be identical to the enzyme having nuclease activity.

In an embodiment, the template-dependent polymerase may be different from the enzyme having nuclease activity.

The target-dependent fragment released by the cleavage reaction of the TO comprises the one or more synthetic unnatural bases.

The term "target-dependent fragment comprising the tagging portion or a part of the tagging portion of the TO" in the context of refer to the cleavage of the TO by an enzyme having nuclease activity is used to mean that (i) a tagging portion, (ii) a part of the tagging portion, (iii) a 5'-tagging portion and a part of the 5'-end of a 3'-targeting portion, and (iv) the 3'-end of a 5'-targeting portion and a 3'-tagging portion are included.

The term "part" used herein, such as a part of the tagging portion of the TO, a part of the targeting portion, and a part of the capturing portion of the FHO, when referring to the TO or FHO, means a nucleotide sequence consisting of 1 to 40, 1 to 30, 1 to 20, 1 to 15, 1 to 10, or 1 to 5 nucleotides, and specifically, may be 1, 2, 3, or 4 nucleotides.

### Step (b): Formation of Duplex

Then, the target-dependent fragment, which is cleaved and released from the TO dependent on the presence of the target nucleic acid sequence, reacts with a fragment-hybridizing oligonucleotide (FHO) to form a duplex.

In an embodiment, the FHO may be (i) a first form of FHO comprising, in a 3' to 5' direction, a capturing portion having a hybridizing nucleotide sequence with the target-dependent fragment and a templating portion having a non-hybridizing nucleotide sequence with the target-dependent fragment and the targeting portion of the TO or (ii) a second form of FHO comprising a capturing portion having a hybridizing nucleotide sequence with the target-dependent fragment.

In an embodiment, the capturing portion of the first form of FHO or the second form of FHO comprises a second synthetic unnatural base capable of base pairing with the first synthetic unnatural base in the target-dependent fragment.

The unnatural base pair used in the present disclosure means a base pair formed between unnatural bases. Specifically, the unnatural base pair used in the present disclosure means an unnatural base pair formed between synthetic unnatural bases exhibiting binding affinity different from that of Watson-Crick base-pairing.

In an embodiment, the unnatural base pairs formed between the synthetic unnatural bases used in the present disclosure have a higher binding affinity than the natural base pairs formed between the natural bases (Zunyi Yang, et al., Nucleic Acids Research, Vol. 34, No. 21, 2006), thereby increasing the binding affinity between the target-dependent fragment comprising the first synthetic unnatural base, and the first form of FHO or the second form of FHO comprising the second synthetic unnatural base. Under predetermined hybridization conditions, the synthetic unnatural base may form an unnatural base pair with a synthetic unnatural base complementary thereto. In addition, the synthetic unnatural base may comprise an unnatural base pair that does not follow the Watson-Crick pairing rules. For example, the synthetic unnatural base may be used to encompass those exhibiting binding affinity different from that of a natural base pair by a binding method (*e.g*., non-hydrogen bond, *etc*.) different from that of the natural base pair.

In general, the Watson-Crick pairing rules follow two complementarity rules: (i) size complementarity (pair of large-sized purine and small-sized pyrimidine); and (ii) hydrogen bond complementarity (pair of hydrogen bond donor and hydrogen bond acceptor) (U.S. Pat. 10,865,431).

As used herein, the expression "unnatural base pair exhibits binding affinity different from that of the Watson-Crick base pair" means that the number of hydrogen bonds, the strength of hydrogen bond, and the like exhibited by the unnatural base pair are different from those exhibited by the Watson-Crick base-pair between natural bases such as G-C and A-T (U), and thus the unnatural base pair exhibits the binding affinity different from that of the natural base pair.

In an embodiment, the unnatural base pair comprises a Watson-Crick base pair generated between synthetic unnatural bases exhibiting binding affinity different from the Watson-Crick base-pairing generated between natural bases such as G-C and A-T (U).

Examples of the unnatural base pairs may comprise four types of mutually exclusive pairs formed from eight types of unnatural bases as disclosed in U.S. Pat. 5,432,272, 5,965,364, 6,001,983, 6,037,120, 6,140,496, 6,627,456, and 6,617,106. The eight types of unnatural bases are classified according to the heterocyclic ring system and the hydrogen bonding pattern and comprise pyAAD(S), pyDDA(Z), pyADD(V), pyDAD(K), puDDA(B), puAAD(P), puDAA(J) and puADA(X). Here, py refers to a pyrimidine-like heterocyclic ring system, pu refers to a purine-like heterocyclic ring system, D refers to a hydrogen bond donor group, and A refers to a hydrogen bond acceptor group. For example, C-G, which is an example of a natural base pair, may be indicated as a pyDAA-puADD pair. The four types of mutually exclusive pairs comprise a pyAAD-puDDA(S-B) pair, a pyDDA-puAAD(Z-P) pair, a pyADD-puDAA(V-J) pair, and a pyDAD-puADA(K-X) pair. The eight types of unnatural bases may comprise various structures as long as they satisfy the presence or absence of a heterocyclic ring system (pyrimidine-like or purine-like) and the presence or absence of a hydrogen bond donor group and acceptor group for each type.

For example, a representative example of the S-B pair is isoC-isoG, wherein the isoC refers to isocytosine or deoxyisocytosine having a structure of pyAAD; isoG refers to isoguanosine or deoxyisoguanosine having a structure of puDDA. A detailed description of isoC-isoG base pair can be found in C. Y. Switzer, et al., Biochemistry, vol. 32, no. 39, pp. 10489-10496, 1993; J. A. Piccirilli et al., Nature, vol. 343, no. 6253, pp. 33-37, 1990; M. J. Moser et al., Clinical Chemistry, vol. 49, no. 3, pp. 407-414, 2003; Johnson SC et al., Nucleic Acids Res 2004, 32(6): 1937-1941.

As a representative example of Z-P, Z may be 6-amino-5-nitro-3-(1'-β-D-2'-deoxyribofuranosyl)-2(1H)-pyridone, and P may be 2-amino-8-(2'-deoxy-β-D-erythro-pentofuranosyl)-imidazo[1,2-*a*]-1,3,5-triazin-4(8H)-one. The Z-P base pairs exhibit 99.8% unnatural base pair selectivity per replication and 0.2% misincorporation opposite the natural base per amplification in PCR using Taq DNA polymerase (Yang et al., 2007, 2011; Robert W. Molt. Jr. et al., Nucleic Acids Res., 2017, 45(7): 3643-3653).

As a representative example of V-J, V may be a 6-aminopyrazin-2-one ring, and J may be 2'-deoxy-5-aza7-deazaisoguanosine; and as a representative example ofK-X, K may be 2,4-diaminopyrimidine, and X may be 2'-deoxyxanthosine.

In an embodiment, the unnatural base pair may also comprise an unnatural base pair based on a non-hydrogen bond, which is different from a Watson-Crick base pair based on a hydrogen bond. For example, the unnatural base pair may comprise an unnatural base pair based on hydrophobic and shape complementary interaction (*e*.*g*., Q-Pa, Ds-Pa, Ds-Pn, and Dss-Pn) and an unnatural base pair based on hydrophobic and packing interaction (*e*.*g*., 5SICS-MMO2, 5SICS-5FM, and 5SICS-NaM). A detailed description thereof can be found in Filip Wojciechowski et al., Chem. Soc. Rev., 2011, 40, 5669-5679.

In an embodiment, the unnatural base pair may be selected from the group consisting of S-B base pair, Z-P base pair, V-J base pair, K-X base pair, Pa-Ds base pair, Pa-Q base pair, Pn-Ds base pair, Pn-Dss base pair, Px-Ds base pair, MMO2-5SICS base pair, 5FM-5SICS base pair, and NaM-5SICS base pair.

As used herein, Pa refers to pyrrole-2-carbaldehyde, Ds refers to 7-(2-thienyl)-imidazo[4,5-b]pyridine, Q refers to 9-methyl-1-H-imidzo[(4,5-*b*)]pyridine, Pn refers to 2-nitropyrrole, Dss refers to 7-(2,2-bithien-5-yl)-imidazo[4,5-*b*]pyridine, Px refers to 2-nitro-4-propynylpyrrole, MMO2 refers to 2-methoxy 4-methyl benzene, 5SICS refers to 5-Sulfoisocarbostyril, 5FM refers to 5-fluoro-4-methyl-2-methoxybenzene, and NaM refers to 2-methoxy naphthalene.

In an embodiment, the first synthetic unnatural base may be any base of a pair selected from the group consisting of S-B base pair, Z-P base pair, V-J base pair, K-X base pair, Pa-Ds base pair, Pa-Q base pair, Pn-Ds base pair, Pn-Dss base pair, Px-Ds base pair, MMO2-5SICS base pair, 5FM-5SICS base pair, and NaM-5SICS base pair.

In an embodiment, the first synthetic unnatural base may be selected from two unnatural bases constituting the unnatural base pair to be used, based on selectivity. Specifically, an unnatural base having high selectivity in the unnatural base pair (*i.e*., an unnatural base having a low frequency of base pairing with another base comprising a natural base other than a complementary unnatural base) may be used as the first synthetic unnatural base, and the remaining base may be used as the second synthetic unnatural base. For example, S (*e.g.,* iso-C) having a relatively high selectivity in an S-B base pair may be used as the first synthetic unnatural base, and B (*e.g.,* iso-G) having a relatively low selectivity may be used as the second synthetic unnatural base.

In an embodiment, the target-dependent fragment and the capturing portion of the FHO may comprise one or more S-B base pairs.

As an example, the target-dependent fragment may comprise at least one S, and the capturing portion of the FHO may comprise B, which is complementary to the opposite S. As another example, the target-dependent fragment may comprise at least one B, and the capturing portion of the FHO may comprise S, which is complementary to the opposite B.

In an embodiment, the target dependent-fragment and the capturing portion of the FHO comprise one or more Z-P base pairs.

As an example, the target-dependent fragment may comprise at least one Z, and the capturing portion of the FHO may comprise P, which is complementary to the opposite Z. As another example, the target-dependent fragment may comprise at least one P, and the capturing portion of the FHO may comprise Z, which is complementary to the opposite P.

In an embodiment, the target dependent-fragment and the capturing portion of the FHO comprise one or more V-J base pairs.

As an example, the target-dependent fragment may comprise at least one V, and the capturing portion of the FHO may comprise J, which is complementary to the opposite V. As another example, the target-dependent fragment may comprise at least one J, and the capturing portion of the FHO may comprise V, which is complementary to the opposite J.

In an embodiment, the target dependent-fragment and the capturing portion of the FHO comprise one or more K-X base pairs.

As an example, the target-dependent fragment may comprise at least one K, and the capturing portion of the FHO may comprise X, which is complementary to the opposite K. As another example, the target-dependent fragment may comprise at least one X, and the capturing portion of the FHO may comprise K, which is complementary to the opposite X.

In an embodiment, the target dependent-fragment and the capturing portion of the FHO comprise one or more Ds-Px base pairs.

The Ds-Px base pair is a third base pair and exhibits high efficiency and selectivity in PCR amplification (Michiko Kimoto et al., Journal of Nucleic Acids, Vol. 2012). The selectivity of Ds-Px base pair was reported to be 99.77%-99.92% by exonuclease-proficient Deep Vent DNA polymerase (Rie Yamashige et al., Nucleic Acids Research, 2011, 40(6):2793-2806).

As an example, the target-dependent fragment may comprise at least one Ds and the capturing portion of the FHO may comprise Px, which is complementary to the opposite Ds. As another example, the target-dependent fragment may comprise at least one Px and the capturing portion of the FHO may comprise Ds, which is complementary to the opposite Px.

In an embodiment, the target dependent-fragment and the capturing portion of the FHO comprise one or more 5SICS-MMO2 base pairs.

The 5SICS-MMO2 base pair exhibits 99.9% selectivity in PCR using OneTaq (a mixture of Taq and exonuclease-proficient Deep Vent DNA polymerase) (Malyshev et al., 2009, 2012). The rate of misincorporation of 5SICS-MMO2 base pairs is 0.01-0.1% per replication per base.

As an example, the target-dependent fragment may comprise at least one 5SICS and the capturing portion of the FHO may comprise MMO2, which is complementary to the opposite 5SICS. As another example, the target-dependent fragment comprises at least one MMO2 and the capturing portion of the FHO comprises 5SICS, which is complementary to the opposite MMO2.

In an embodiment, the reaction in step (b) comprises a reaction in which the target-dependent fragment is hybridized with the capturing portion of the FHO.

The hybridization of the target-dependent fragment and the capturing portion of the FHO may be carried out under suitable hybridization conditions that are generally determined by an optimization procedure. In particular, the first synthetic unnatural base and/or the second synthetic unnatural base included in the target-dependent fragment and the capturing portion of the FHO may be carried out under conditions that the first synthetic unnatural base and the second synthetic unnatural base pair with each other, but not with any of natural bases. Since the first synthetic unnatural base constituting the unnatural base pair is hybridized only with the second synthetic unnatural base, the introduction of the unnatural base pair into the tagging portion of the TO and the capturing portion of the FHO increases the binding specificity of the target-dependent fragment to the FHO and reduces the likelihood that the target-dependent fragment is hybridized with a non-target nucleic acid sequence other than the FHO. This may eventually increase a signal indicative of the presence of the target nucleic acid sequence, and may increase detection sensitivity.

In addition, the unnatural base pairs included in the target-dependent fragment and the capturing portion of the FHO increase the melting temperature of the hybrid between the target-dependent fragment and the capturing portion of the FHO, that is, improve the binding affinity between the target-dependent fragment and the capturing portion of the FHO.

Thus, the hybrid between the capturing portion of the FHO and the target-dependent fragment comprising the unnatural base pair has a higher melting temperature and binding affinity than the hybrid between the capturing portion of the FHO and the target-dependent fragment not comprising the unnatural base pair.

The increased binding affinity between and the capturing portion of the FHO and the target-dependent fragment comprising the unnatural base pair may increase the extension efficiency of the target-dependent fragment hybridized with the capturing portion of the FHO, which may, in turn, lead to an increase in the signal indicative of the presence of the target nucleic acid sequence and an increase in the detection sensitivity.

Meanwhile, the introduction of the unnatural base pair into the tagging portion of the TO and the capturing portion of the FHO may reduce the length while maintaining the Tm of the tagging portion of the TO. That is, the tagging portion of the TO comprising the first synthetic unnatural base may have a shorter length than the tagging portion of the conventional TO not comprising the synthetic unnatural base.

Since the hybridization efficiency between the shortened tagging portion of the TO and the non-target nucleic acid sequence is more affected by mismatch as compared with the hybridization efficiency between the long tagging portion of the TO and the non-target nucleic acid sequence, the hybridization of the short target-dependent fragment with the non-target nucleic acid sequence is less likely to be occur than the hybridization of the long target-dependent fragment with the non-target nucleic acid sequence. Thus, using the short tagging portion of the TO may prevent the hybridization with a non-target, which may lead to an increase in the signal indicative of the presence of the target nucleic acid sequence.

According to an embodiment, a Tm value of the first or second duplex is 0.5 to 10°C higher than a Tm value of a duplex in which the first and second synthetic unnatural bases included in the first or second duplex are substituted with natural bases.

In an embodiment, the reaction in step (b) may comprise an extension reaction by a template-dependent nucleic acid polymerase.

In an embodiment, when the FHO is the first form of FHO, the target-dependent fragment is hybridized with the capturing portion of the first form of the FHO and is extended to generate an extended strand comprising an extended sequence complementary to the templating portion of the FHO, thereby inducing the formation of a first duplex between the extended strand and the first form of FHO. The uncleaved TO hybridized with the capturing portion of the first form of FHO is not extended, and thus does not generate an extended strand.

The target-dependent fragment hybridized with the capturing portion of the first form of FHO may be extended along the templating portion of the first form of FHO as a template by the activity of the template-dependent nucleic acid polymerase.

In an embodiment, the TO and/or the FHO is blocked at its 3'-end to prohibit its extension.

The terms "extended sequence," "extended strand," and "first duplex" in the context of the extension reaction of the target-dependent fragment have the following meanings:
As used herein, the term "extended sequence" refers to a sequence newly extended through an extension reaction from the target-dependent fragment hybridized with the capturing portion of the first form of FHO. In addition, the extended sequence refers to a portion excluding the target-dependent fragment from the extended strand which will be described below.

As used herein, the term "extended strand" refers to a sequence encompassing the target-dependent fragment and the extended sequence. In addition, the extended strand refers to a portion excluding the first form of FHO in the first duplex which will be described below.

As used herein, the term "first duplex" refers to a hybrid or a duplex (through complementarity between sequences) between the extended strand and the first form of FHO. In other words, the first duplex refers to a duplex between the first form of FHO and the extended strand composed of the target-dependent fragment and the extended sequence.

The template-dependent nucleic acid polymerase used in step (b) includes any nucleic acid polymerase, including, for example, "Klenow" fragment of *Escherichia coli* DNA polymerase I, thermostable DNA polymerase, and bacteriophage T7 DNA polymerase. In particular, the template-dependent nucleic acid polymerase is a thermostable DNA polymerase obtained from a variety of bacterial species, including *Thermus aquaticus*(Taq), *Thermus thermophilus(Tth), Thermus filiformis, Thermis flavus, Thermococcus literalis, Thermus antranikianii, Thermus caldophilus, Thermus chliarophilus, Thermus flavus, Thermus igniterrae, Thermus lacteus, Thermus oshimai, Thermus ruber, Thermus rubens, Thermus scotoductus, Thermus silvanus, Thermus species* Z05, *Thermus species sps 17, Thermus thermophilus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosiphoafricanus, Pyrococcus furiosus* (Pfu), *Pyrococcus woesei, Pyrococcus horikoshii, Pyrococcus abyssi, Pyrodictium occultum, Aquifex pyrophilus* and *Aquifex aeolieus.* More specifically, the template-dependent nucleic acid polymerase is a Taq polymerase.

In an embodiment, the template-dependent nucleic acid polymerase comprises a reverse transcriptase.

In an embodiment, when the FHO is the second form of FHO, the target-dependent fragment is hybridized with the capturing portion of the second form of FHO to form a second duplex.

In an embodiment, the method makes it possible to form the second duplex by hybridization between the second form of the FHO and the target-dependent fragment released by the cleavage of the TO in step (a).

In an embodiment, according to the method, the tagging portion of the TO is first hybridized with the second form of the FHO to form a double-stranded form, followed by a cleavage reaction of the TO by an enzyme having nuclease activity to form a second duplex.

In an embodiment, the second form of FHO may be linked to the end of the tagging portion of the TO. For example, the tagging portion of the TO and the second form of FHO may be included in a single oligonucleotide. In this case, the hybridization between the tagging portion of the TO and the second form of FHO may form a hairpin structure.

The term "hairpin" refers to a structure of an oligonucleotide having a double-stranded "stem" region and a single-stranded "loop" region.

As used herein, the term "second duplex" refers to a hybrid or a duplex (through complementarity between sequences) between the target-dependent fragment and the capturing portion of the second form of FHO. The hybrid or duplex between the tagging portion of the uncleaved TO and the capturing portion of the second form of FHO is not included in the second duplex.

In an embodiment, the enzyme having nuclease activity used in step (a) (in particular, the enzyme having 5' nuclease activity), the template-dependent nucleic acid polymerase for extending the upstream primer in step (a), and the template-dependent nucleic acid polymerase for extending the target-dependent fragment in step (b) may all be different, or may be partially identical or all identical.

### Step (c): Detection of Presence of Extended Strand in First Duplex or Presence of Second Duplex

Finally, the presence of the extended strand in the first duplex or the presence of the second duplex is detected. The presence of the extended strand in the first duplex or the presence of the second duplex is indicative of the presence of the target nucleic acid sequence.

In an embodiment, the target nucleic acid sequence may be detected by detecting a signal indicative of the presence of the target nucleic acid sequence.

As used herein, the term "signal indicative of the presence of the target nucleic acid sequence" refers to a signal having an intensity exceeding an intensity of a background signal or an intensity of a signal that may be provided in the absence of the target nucleic acid, or refers to a signal having an intensity after subtracting the intensity of a background signal or the intensity of a signal that may be provided in the absence of the target nucleic acid sequence from the intensity of the provided signal.

In an embodiment, the signal indicative of the presence of the target nucleic acid sequence comprises all signals capable of indicating the presence of the extended strand in the first duplex or the presence of the second duplex. For example, the signal indicative of the presence of the target nucleic acid sequence comprises a signal from a label (signal generation or extinguishment), a change in the signal from the label (signal increase or decrease), a melting curve, a melting pattern, and a melting temperature (or Tm value).

In an embodiment, step (c) may be achieved by detecting a signal indicative of the presence of the extended strand in the first duplex or a signal indicative of the presence of the second duplex.

In an embodiment, the method may provide a signal from a label in a manner dependent on the formation of a duplex. By detecting the signal, the presence of the target nucleic acid sequence may be detected. The duplex comprises not only the first or second duplex, but also a third duplex formed dependent on the presence of the first or second duplex.

According to an embodiment, the provision of the signal comprises "signal generation or extinguishment" and "signal increase or decrease" from the label.

As used herein, the term "duplex" may be used to encompass a duplex in associated form and a duplex in disassociated form. That is, the duplex may refer to two single-stranded nucleic acid molecules that comprise partially or entirely complementary sequences to each other and thus may be hybridized with each other under hybridization conditions to have a structure of duplex (hybrid). For example, depending on the temperature, the duplexes may exist in associated form, or may exist in dissociated form, or some of them may exist in associated form and the rest may exist in dissociated form.

In an embodiment, the method may provide a signal in a manner dependent on the association or dissociation of a duplex, and detect the signal, thereby detecting the presence of the target nucleic acid sequence. For example, the duplex in associated form may extinguish (or emit) signals, and the duplex in dissociated form may emit (or extinguish) signals, so that the duplex is capable of providing different signals in the associated state and the dissociated state, thereby detecting the presence of a target nucleic acid sequence using the duplex.

The term "association" or "dissociation" has the same meaning as the term "hybridization" or "denaturation".

In a specific embodiment, the method may provide a signal in a manner dependent on the cleavage of a duplex, and detect the signal to detect the presence of the target nucleic acid sequence. The cleaved duplex comprises a first duplex or a second duplex, as well as a third duplex formed dependent on the presence of the first duplex or the second duplex.

In an embodiment, the label may be a single label and/or an interactive label.

As a representative example of an interactive labeling system, a fluorescence resonance energy transfer (FRET) labeling system comprises a fluorescent reporter molecule (donor molecule) and a quencher molecule (acceptor molecule). In FRET, the energy donor may be fluorescent, but the energy acceptor may be fluorescent or non-fluorescent. In another form of an interactive labeling system, the energy donor is non-fluorescent, for example, chromophore, and the energy acceptor is fluorescent. In another form of an interactive labeling system, the energy donor is luminescent, such as bioluminescent, chemiluminescent or electrochemiluminescent, and the acceptor is fluorescent. Donor molecules and acceptor molecules may be described as reporter molecules and quencher molecules, respectively, in the present disclosure. The interactive dual labels comprise a label pair that provides a detectable signal on the basis of contact-mediated quenching (Salvatore et al., Nucleic Acids Research, 2002 (30) no.21 e122, and Johansson et al., J. AM. CHEM. SOC 2002 (124) pp. 6950-6956). In the present disclosure, the interactive labeling system comprises any systems that induce a signal change due to an interaction between at least two molecules (*e*.*g*., dyes).

Reporter molecules and quencher molecules useful as interactive-dual labels may comprise any molecules known in the art. Examples thereof are as follows: Cy2^{™}(506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), Magnesium Green^{™}(531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™}(576), Pyronin Y(580), Rhodamine B (580), TAMRA (582), Rhodamine Red^{™} (590), Cy3.5^{™} (596), ROX (608), Calcium Crimson^{™} (615), Alexa^{™} 594 (615), Texas Red(615), Nile Red (628), YO-PRO^{™}-3 (631), YOYO^{™}-3 (631), Rphycocyanin (642), C-Phycocyanin (648), TO-PRO^{™}-3 (660), TOTO3 (660), DiD DilC(5) (665), Cy5^{™}(670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705), and Quasar 705 (610). The number in parentheses is the maximum emission wavelength expressed in nanometers. Specifically, the reporter molecule and the quencher molecule comprise JOE, FAM, TAMRA, ROX and fluorescein-based labels.

Suitable reporter-quencher pairs are disclosed in various documents as follows: Pesce et al., editors, Fluorescence Spectroscopy (Marcel Dekker, New York, 1971); White et al., Fluorescence assay: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996); US Pat. Nos. 3,996,345 and 4,351,760.

It is noteworthy that in the present invention, a non-fluorescent black quencher molecule capable of quenching fluorescence of a wide wavelength or a specific wavelength can be used. Examples of non-fluorescent black quencher molecules are BHQ and DABCYL.

In the FRET label applied to the present disclosure, the reporter comprises a donor of FRET and the quencher comprises the remaining partner (acceptor) of FRET. For example, a fluorescein dye is used as a reporter and a rhodamine dye is used as a quencher.

The label may be linked to the TO and/or FHO through conventional methods. Specifically, the label is linked to the TO and/or FHO through a spacer containing carbon atoms (*e.g.,* a 3-carbon spacer, a 6-carbon spacer, or a 12-carbon spacer).

In an embodiment, the interactive labels may comprise at least one reporter molecule and at least one quencher molecule. For example, the interactive labels may comprise one reporter molecule and one quencher molecule or comprising one reporter molecule and two quencher molecules.

In an embodiment, the single label should be able to provide different signals depending on whether it is on a double strand or on a single strand. The single label comprises a fluorescent label, a luminescent label, a chemiluminescent label, an electrochemical label, and a metal label. Specifically, the single label comprises a fluorescent label.

The types and preferred binding sites of single fluorescent labels used in the present disclosure are disclosed in U.S. Pat. Nos. 7,537,886 and 7,348,141. Specifically, the single fluorescent label comprises JOE, FAM, TAMRA, ROX, and fluorescein-based label. The labeled nucleotide residue is preferably located at the internal nucleotide residue in the oligonucleotide than at the 5'-end or 3'-end.

The single fluorescent label useful in the present disclosure may be detailed with reference to the description of the reporter and quencher molecules as describe above.

In particular, when the method is carried out in a solid phase using a single label, conventional fluorescent label may be used, and no specific fluorescent label is required as long as it is capable of providing fluorescent signals having different intensities depending on whether it is present on a double strand or on a single strand. The target signal provided from the solid substrate is measured.

When the FHO immobilized on the solid substrate is used, a chemical label (*e.g*., biotin) or an enzyme label (*e*.*g*., alkaline phosphate, peroxidase, β-galactosidase, and β-glucosidase) may be used.

In the present disclosure, a signal related to the formation of a first duplex or a second duplex is measured in order to detect the presence of the target nucleic acid sequence.

In an embodiment, the first duplex or the second duplex has a label, whereby the first duplex or the second duplex provides a signal.

The detection of the presence of the extended strand in the first duplex or the presence of the second duplex in step (c) may be carried out by any of various methods known in the art.

In an embodiment, when the method forms the first duplex, the detection of the presence of the extended strand in the first duplex in step (c) may be carried out by any of various methods known in the art. Specifically, the detection may be carried out by (i) measuring the signal from the first duplex at a predetermined temperature, or (ii) measuring the signal from the first duplex by a melting analysis or a post-melting hybridization analysis.

As used herein, the term "melting analysis" refers to a method for obtaining a signal indicative of the presence of a duplex by melting the duplex, and comprises a method for measuring a signal at two different temperatures, a melting curve analysis, a melting pattern analysis, and a melting peak analysis. Specifically, the melting analysis is a melting curve analysis.

As used herein, the term "post-melting hybridization analysis" refers to a method for obtaining a signal indicative of the presence of a duplex by melting the duplex and then hybridization. For example, the post-melting hybridization analysis may be a hybridization curve analysis.

The melting curve or hybridization curve may be obtained by conventional techniques as described in, for example, U.S. Pat. Nos. 6,174,670 and 5,789,167, Drobyshev, et al., Gene 188:45 (1997); Kochinsky and Mirzabekov, Human Mutation 19:343 (2002); Livehits et al., J. Biomol. Structure Dynam. 11:783 (1994); and Howell et al., Nature Biotechnology 17:87 (1999). For example, the melting curve or hybridization curve may be composed of a graphic plot or display of an output signal change for a parameter of hybridization stringency. The output signal may be plotted directly for the hybridization parameter. Typically, the melting curve or hybridization curve will be plotted, for example, with an output signal, *e.g.,* fluorescence, indicating the degree of the duplex structure (*i.e*., the degree of hybridization) on the Y-axis, and with a hybridization parameter on the X-axis.

A plot of the first derivative of the fluorescence vs. temperature, *i.e.,* a plot of the rate of change in fluorescence vs. temperature (dF/dT vs. T) or (-dF/dT vs. T) provides a melting peak.

The detection method will be schematically described below.

### Detection of First Duplex at Predetermined Temperature

In an embodiment, the detection in step (c) is carried out by measuring the signal from the first duplex between the extended strand and the first form of FHO at a predetermined temperature.

As an example, the presence of the extended strand is detected by measuring a signal from the first duplex at a predetermined temperature sufficient to maintain a double strand of the first duplex (*i.e.,* to allow the first duplex to exist in associated form).

The first duplex may itself provide a signal that can distinguish the formation and non-formation of the first duplex, and the signal is detected at a predetermined temperature at which the first duplex maintains a double-stranded form, thereby determining the presence of the target nucleic acid sequence.

In the present disclosure, a signal related to the formation of the first duplex is measured to detect the presence of the target nucleic acid sequence.

In an embodiment, the first duplex has a label, whereby the first duplex provides a signal.

The underlying principle of providing the signal from the first duplex is as follows: (i) the extension of the target-dependent fragment induces a signal change from the label to provide a signal; or (ii) the hybridization of the target-dependent fragment and the first form of FHO induces a signal change from the label to provide a signal, and the first duplex formed by the extension after the hybridization maintains the signal.

For example, when the immobilized first form of FHO is used, the present disclosure detects multiple target nucleic acid sequences in a more effective manner. The templating portion of the immobilized first form of FHO has a reporter molecule and a quencher molecule. The reporter molecule and the quencher molecule are conformationally in close proximity to each other such that the quencher molecule quenches the signal from the reporter molecule. When the target-dependent fragment is hybridized with the capturing portion of the first form of FHO, the quencher molecule quenches the signal from the reporter molecule. Thereafter, when the target-dependent fragment is extended to form the first duplex, the reporter molecule and the quencher molecule are conformationally separated from each other such that the quencher molecule unquenches the signal from the reporter molecule. Accordingly, the signal indicative of the presence of the target nucleic acid sequence is provided in the extension step.

In a specific embodiment, the tagging portion (and target-dependent fragment) of the TO has a reporter molecule and a quencher molecule. The reporter molecule and the quencher molecule are conformationally in close proximity to each other such that the quencher molecule quenches the signal from the reporter molecule. The TO hybridized with the target nucleic acid sequence is cleaved to release a target-dependent fragment comprising a tagging portion having a reporter molecule and a quencher molecule, and the target-dependent fragment is hybridized with the capturing portion of the first form of FHO. Due to the hybridization, the reporter molecule and the quencher molecule are conformationally separated from each other such that the quencher molecule unquenches the signal from the reporter molecule. The signal is provided in the hybridization step between the target-dependent fragment and the first form of FHO, and the first duplex maintains the signal.

When the tagging portion of the TO (and the target-dependent fragment) has a reporter molecule and a quencher molecule, the duplex between the uncleaved TO and the first form of FHO also unquenches the signal from the reporter molecule like the quencher molecule of the first duplex. In this case, the predetermined temperature (*i*.*e*., the temperature at which the signal is detected) may be determined within a temperature range in which some or all of the duplexes between the uncleaved TO and the first form of FHO are in dissociated form and some or all of the first duplexes are in associated form. For example, the signal may be detected at a temperature at which all of the duplexes between the uncleaved TO and the first form of FHO are in dissociated form, and some or all of the first duplexes are in associated form. In this case, the detected signal is provided from the first duplex and is indicative of the presence of the target nucleic acid sequence. As another example, the signal may be detected at a temperature at which some of the duplexes between the uncleaved TO and the first form of FHO are in associated form and all of the first duplexes are in associated form. In this case, the detected signal is provided from the duplex between the uncleaved TO and the first form of FHO and is indicative of the presence of the target nucleic acid sequence. Specifically, the amount of duplex between the uncleaved TO and the first form of FHO decreases as the first duplex is formed, and the signal provided from the duplex between the uncleaved TO and the first form of FHO also decreases, and the change in the signal (*i.e.,* a decrease in the signal) indicates the presence of the target nucleic acid sequence.

According to an embodiment, the temperature at which the signal is detected is additionally determined in consideration of the Tm value of the duplex.

According to an embodiment, the predetermined temperature is higher than the temperature obtained by subtracting 10°C from the Tm value of the duplex (the higher Tm value among the two forms of duplexes when two forms of duplexes are produced), specifically higher than the temperature obtained by subtracting 5°C from the Tm value of the duplex, more specifically higher than the Tm value of the duplex, and even more specifically higher than the temperature obtained by adding 5°C to the Tm value of the duplex.

### Detection of First Duplex by Melting or Post-Melting Hybridization Analysis

In an embodiment, the detection in step (c) is carried out by measuring the signal from the first duplex between the extended strand and the first form of FHO by a melting analysis or a post-melting hybridization analysis.

According to an embodiment, the detection of the presence of the extended strand in step (c) is carried out through the melting analysis, during which the first duplex is melted in a certain range of temperatures to provide a signal indicative of the presence of the extended strand.

According to an embodiment, the detection of the presence of the extended strand in step (c) is carried out through the post-melting hybridization analysis. For example, the detection of the presence of the extended strand in step (c) is carried out through the hybridization analysis during which the first duplex is melted and the resultant is hybridized in a certain range of temperatures to provide a target signal indicative of the presence of the extended strand.

The detection of the first duplex at the above-described predetermined temperature and the detection of the first duplex by the melting analysis or the post-melting hybridization analysis may be carried out according to various methods as follows: A. detection of the first duplex; B. detection of the cleavage of the first duplex; C. detection of the duplex between the extended strand and the signaling oligonucleotide; D. detection of the cleavage of the duplex between the extended strand and the signaling oligonucleotide; E. detection of the duplex between the first form of FHO and the hybridizing oligonucleotide; F. detection of the duplex between the extended strand and the immobilizing oligonucleotide; and G. detection of a third duplex by using a third form of FHO.

The analysis method will be described in detail below.

### A. Detection of First Duplex

According to an embodiment, the detection in step (c) may be carried out by directly using the signal from the first duplex between the extended strand and the first form of FHO. For example, it may be carried out on the basis of PTOCE assay (*see* WO 2012/096523).

The TO, the first form of FHO, the target-dependent fragment, and the first duplex of the present disclosure may correspond to a probing and tagging oligonucleotide (PTO), a capturing and templating oligonucleotide (CTO), a PTO fragment *(i.e.,* a tagging portion of PTO), and an extended duplex, respectively, in a PTOCE-based method such as PTOCE, PCEC, PCE-SH, PCE-SC, PCE-NH, PCE-IH, and PTOCE-E as described above.

According to an embodiment, the first duplex provides a signal detectable by (i) at least one label linked to the target-dependent fragment and/or the first form of FHO, (ii) a label incorporated into the first duplex during the extension reaction, (iii) at least one label linked to the first form of FHO and a label incorporated into the first duplex during the extension reaction, or (iv) an intercalating label, and the presence of the extended strand in step (c) may be detected by measuring the signal provided from the first duplex.

The details on the principles of PTOCE assay are referred to International Publication WO 2012/096523.

The PTOCE assay-based labeling system may be described as follows:

### (i) At least One Label Linked to Target-dependent Fragment and/or First Form of FHO

According to an embodiment, the signal is provided by (i) at least one label linked to the target-dependent fragment and/or the first form of FHO.

The label comprises interactive dual labels and a single label.

### (i-1) Interactive Dual Labels

In an embodiment, the signal indicative of the presence of the first duplex *(i.e.,* the presence of the target nucleic acid sequence) is provided by an interactive labeling system, and in particular by FRET labeling system (*i.e.,* an interactive dual labeling system).

### Embodiment 1 (Intra-strand Interactive Dual Labels)

In Embodiment 1 of interactive dual labels system, a target-dependent fragment or FHO has interactive dual labels comprising a reporter molecule and a quencher molecule; and depending on the positions of the reporter molecule and quencher molecule, the hybridization between the target-dependent fragment and the first form of FHO, the formation of a first duplex between the extended strand and the first form of FHO, or the melting or post-melting hybridization of the first duplex results in a change in the signal from the interactive dual labels, thereby providing a detectable signal. Embodiment 1 of the interactive dual labels system is referred to as intra-strand interactive dual labels.

### Embodiment 1-1 (Intra-strand Interactive Dual Labels on First Form of FHO)

According to Embodiment 1-1, the first form of FHO has interactive dual labels comprising a reporter molecule and a quencher molecule, and the hybridization between the target-dependent fragment and the first form of FHO, the formation of a first duplex between the extended strand and the first form of FHO, or the melting or post-melting hybridization of the first duplex results in a change in the signal from the interactive dual labels, thereby providing a detectable signal.

According to Embodiment 1-1, when the target-dependent fragment is hybridized with the first form of FHO, when the target-dependent fragment hybridized with the first form of FHO is extended to form a first duplex, or when the first duplex is subjected to melting or post-melting hybridization, a change in a structural distance between the reporter molecule and the quencher molecule is caused and thus a signal (specifically, signal generation) is provided.

For example, before the target-dependent fragment is hybridized with the capturing portion of the first form of FHO, the reporter molecule and the quencher molecule on the first form of FHO are conformationally in close proximity to each other such that the quencher molecule quenches the signal from the reporter molecule; whereas when the target-dependent fragment hybridized with the first form of FHO is extended to form a first duplex, the reporter molecule and the quencher molecule on the first form of FHO are conformationally separated such that the quencher molecule unquenches the signal from the reporter molecule, thereby providing a signal (*i.e.,* a signal indicative of the presence of the target nucleic acid sequence). Further, when the first duplex is melted, the reporter molecule and the quencher molecule are conformationally in close proximity to each other such that the quencher molecule quenches the signal from the reporter molecule, thereby providing a signal; and when the first duplex is hybridized again after the melting, the reporter molecule and the quencher molecule are separated again such that the quencher molecule unquenches the signal from the reporter molecule, thereby providing a signal.

The expression "reporter molecule and quencher molecule are conformationally in close proximity to each other" used herein means that the reporter molecule and the quencher molecule are three-dimensionally in close proximity to each other by a conformational structure of an oligonucleotide (*e*.*g*., TO, a target-dependent fragment, or FHO), such as a random coil and a hairpin structure.

The expression "reporter molecule and quencher molecule are conformationally separated" used herein means that the reporter molecule and the quencher molecule are three-dimensionally spaced apart separated by a conformational change of the target-dependent fragment or the first form of FHO when the double strand is formed.

In an embodiment, the reporter molecule and the quencher molecule may be located at any position of the first form of FHO as long as the signal from the reporter molecule is quenched or unquenched depending on the formation, melting, or post-melting hybridization of the first duplex between the target-dependent fragment and the first form of FHO.

In an embodiment, both the reporter molecule and the quencher molecule are located in the templating portion of the first form of FHO.

In an embodiment, at least one of the reporter molecule and the quencher molecule is located in the templating portion of the first form of FHO.

In an embodiment, both the reporter molecule and the quencher molecule are located in the capturing portion of the first form of FHO.

In an embodiment, the reporter molecule and the quencher molecule are located at the 5'-end and 3'-end of the first form of FHO.

In an embodiment, one of the reporter molecule and the quencher molecule is located at the 5'-end or 1 to 5 nucleotides apart from the 5'-end of the first form of FHO, and the other is located at a site to quench or unquench the signal from the reporter molecule depending on the conformation of the first form of FHO.

In an embodiment, one of the reporter molecule and the quencher molecule is located at the 3'-end or 1 to 5 nucleotides apart from the 3'-end of the first form of FHO, and the other is located at a site to quench or unquench the signal from the reporter molecule depending on the conformation of the first form of FHO.

In an embodiment, the reporter molecule and the quencher molecule are located at most 80 nucleotides, specifically at most 60 nucleotides, more specifically at most 30 nucleotides, and even more specifically at most 25 nucleotides apart from each other. In an embodiment, the reporter molecule and the quencher molecule are located at least 4 nucleotides, specifically at least 6 nucleotides, more specifically at least 10 nucleotides, and even more specifically at least 15 nucleotides apart from each other.

A timing at which a signal is generated (a signal change is caused) may vary depending on the positions of the reporter molecule and the quencher molecule on the first form of FHO.

For example, when both the reporter molecule and the quencher molecule are located in the templating portion of the first form of FHO and the reporter molecule and the quencher molecule are spaced apart enough to allow the quencher molecule to unquench the signal from the reporter molecule upon the formation of the double strand, the formation of the first duplex in step (b) may cause a change in the signal from the interactive dual labels, thereby providing a detectable signal.

As another example, when both the reporter molecule and the quencher molecule are located in the capturing portion of the first form of FHO and the reporter molecule and the quencher molecule are spaced apart enough to allow the quencher molecule to unquench the signal from the reporter molecule upon the formation of the double strand, the hybridization between the target-dependent fragment and the first form of FHO causes a change in the signal from the interactive dual labels to provide a detectable signal, and the formation of the first duplex maintains the signal at a temperature at which the signal is detected.

In Embodiment 1-1, when the capturing portion of the first form of FHO is labeled with interactive dual labels, a duplex may be formed between the uncleaved TO and the first form of FHO to provide a signal. In this case, the signal from the first duplex and the signal from the duplex between the uncleaved TO and the first form of FHO may be distinguished by (i) measuring a signal (a signal provided from the first duplex) at a temperature at which the duplex between the uncleaved TO and the first form of FHO is dissociated, (ii) measuring a change in the signal provided from the duplex between the uncleaved TO and the first form of FHO as the first duplex is formed, and thus the amount of the duplex between the uncleaved TO and the first form of FHO is reduced, or (iii) measuring a difference between the Tm value of the first duplex and the Tm value of the duplex between the uncleaved TO and the first form of FHO in a melting analysis.

### Embodiment 1-2 (Intra-strand Interactive Dual Labels on TO)

According to Embodiment 1-2, the target-dependent fragment has interactive dual labels comprising a reporter molecule and a quencher molecule, and the hybridization between the target-dependent fragment and the first form of FHO, or the melting or post-melting hybridization of the first duplex results in a change in the signal from the interactive dual labels, thereby providing a detectable signal.

A signal is provided upon the hybridization between the target-dependent fragment and the first form of FHO, and the signal is maintained at a temperature at which the signal is detected when the first duplex is formed. Specifically, before the target-dependent fragment is hybridized with the capturing portion of the first form of FHO, the reporter molecule and the quencher molecule on the TO are conformationally in close proximity to each other such that the quencher molecule quenches the signal from the reporter molecule; whereas when the target-dependent fragment is hybridized with the capturing portion of the first form of FHO, the reporter molecule and the quencher molecule on the target-dependent fragment are conformationally separated from each other such that the quencher molecule unquenches the signal from the reporter molecule, thereby providing a signal. In addition, the formation of the first duplex between the extended strand and the first form of FHO maintains the signal. Thereafter, when the first duplex is melted, the reporter molecule and the quencher molecule are conformationally in close proximity to each other such that the quencher molecule quenches the signal from the reporter molecule, thereby providing a signal.

According to an embodiment, the reporter molecule and the quencher molecule may be located at any location of the target-dependent fragment as long as the signal from the reporter molecule is quenched or unquenched depending on the hybridization or melting between the target-dependent fragment and the first form of FHO.

According to an embodiment, one of the reporter molecule and the quencher molecule on the target-dependent fragment is located at its 5'-end or 1 to 5 nucleotides apart from its 5'-end, and the other is located at a site to quench or unquench the signal from the reporter molecule depending on the conformation of the target-dependent fragment.

According to an embodiment, the reporter molecule and the quencher molecule are located at most 50 nucleotides, specifically at most 40 nucleotides, more specifically at most 30 nucleotides, and even more specifically at most 20 nucleotides apart from each other. According to an embodiment, the reporter molecule and the quencher molecule are located at least 4 nucleotides, specifically at least 6 nucleotides, more specifically at least 10 nucleotides, and even more specifically at least 15 nucleotides apart from each other.

In Embodiment 1-2, the duplex between the uncleaved TO and the first form of FHO may be formed, thereby providing a signal. In this case, the signal from the first duplex and the signal from the duplex between the uncleaved TO and the first form of FHO may be distinguished by (i) measuring a signal (a signal provided from the first duplex) at a temperature at which the duplex between the uncleaved TO and the first form of FHO is dissociated, (ii) measuring a change in the signal provided from the duplex between the uncleaved TO and the first form of FHO as the first duplex is formed, and thus the amount of the duplex between the uncleaved TO and the first form of FHO is reduced, or (iii) measuring a difference between the Tm value of the first duplex and the Tm value of the duplex between the uncleaved TO and the first form of FHO in a melting analysis.

### Embodiment 2 (Inter-strand Interactive Dual Label)

According to Embodiment 2, the target-dependent fragment has any one label of interactive dual labels comprising the reporter molecule and the quencher molecule, and the first form of FHO has the other of the interactive dual labels, the hybridization between the target-dependent fragment and the first form of FHO, or the melting or post-melting hybridization of the first duplex results in a change in the signal from the interactive dual labels, thereby providing a detectable signal.

For example, when the target-dependent fragment has a quencher molecule and the first form of FHO has a reporter molecule, the reporter molecule on the first form of FHO is not quenched until the target-dependent fragment is hybridized with the first form of FHO, whereas when the target-dependent fragment is hybridized with the first form of FHO, the signal from the reporter molecule linked to the first form of FHO is quenched by the quencher molecule linked to the target-dependent fragment, thereby providing a signal (specifically, signal extinguishment). In addition, the formation of the first duplex between the extended strand and the first form of FHO maintains the signal. Thereafter, when the first duplex is melted, the reporter molecule and the quencher molecule are separated from each other such that the quencher molecule unquenches the signal from the reporter molecule, thereby providing a signal (specifically, signal generation).

As long as the signal from the reporter molecule is quenched by the quencher molecule in the first duplex, the reporter molecule and the quencher molecule may be located at any site of the target-dependent fragment and the first form of FHO. It is advantageous that the quencher molecule and the reporter molecule are located in close proximity to each other in order to facilitate the quencher molecule quenching the signal from the reporter molecule upon the hybridization of the target-dependent fragment and the first form of FHO.

According to an embodiment, the reporter molecule or quencher molecule on the target-dependent fragment is located at the 5'-end or 5'-end portion thereof.

According to an embodiment, the reporter molecule or quencher molecule on the target-dependent fragment is located at the 3'-end or 3'-end portion thereof.

According to an embodiment, the reporter molecule or quencher molecule on the first form of FHO is located in the capturing portion of the first form of FHO.

According to an embodiment, the reporter molecule or quencher molecule on the first form of FHO is located at the 3'-end thereof or 1 to 5 nucleotides apart from the 3'-end thereof.

In Embodiment 2, the duplex between the uncleaved TO and the first form of FHO may be formed, thereby providing a signal (specifically, signal extinguishment). In this case, the signal from the first duplex and the signal from the duplex between the uncleaved TO and the first form of FHO may be distinguished by (i) measuring a signal (a signal provided from the first duplex) at a temperature at which the duplex between the uncleaved TO and the first form of FHO is dissociated, (ii) measuring a change in the signal provided from the duplex between the uncleaved TO and the first form of FHO as the first duplex is formed, and thus the amount of the duplex between the uncleaved TO and the first form of FHO is reduced, or (iii) measuring a difference between the Tm value of the first duplex and the Tm value of the duplex between the uncleaved TO and the first form of FHO in a melting analysis.

The types and features of the labels to be used may be described with reference to the description of the label system applicable to the present invention as described above.

### (i-2) Single Label

The present disclosure is excellently implemented using a single label system that provides a signal indicative of the presence of the target nucleic acid sequence.

According to an embodiment, the target-dependent fragment or the first form of FHO has a single label, and the hybridization between the target-dependent fragment and the first form of FHO, the formation of the first duplex between the extended strand and the first form of FHO, or the melting or post-melting hybridization of the first duplex results in a change in the signal from the single label, thereby providing the detectable signal.

### Embodiment 1 (Single Label on FHO)

According to Embodiment 1, the first form of FHO has a single label, and the hybridization between the target-dependent fragment and the first form of FHO, the formation of the first duplex between the extended strand and the first form of FHO, or the melting or post-melting hybridization of the first duplex results in a change in the signal from the single label, thereby providing the detectable signal.

In the above embodiment, when the target-dependent fragment is hybridized with the first form of FHO, when the target-dependent fragment hybridized with the first form of FHO is extended to form a first duplex, or when the first duplex is subjected to melting or post-melting hybridization, a fluorescence intensity from a single fluorescent label is increased and thus a signal (specifically, signal changes) is provided.

For example, before the target-dependent fragment is hybridized with the capturing portion of the first form of FHO, a single label on the first form of FHO in the single strand state provides a low signal; whereas when the target-dependent fragment hybridized with the first form of FHO is extended to form a first duplex, a single label on the first duplex in the double strand state provides an increased signal, thereby providing a signal indicative of the presence of the target nucleic acid sequence. Further, when the first duplex is melted, a single label on the extended strand of the single strand state provides a reduced signal, thereby providing a signal indicative of the presence of the target nucleic acid sequence; when hybridized again after the melting, a single label on the first duplex in the double strand state provides an increased signal, thereby providing a signal indicative of the presence of the target nucleic acid sequence.

According to an embodiment, the single label may be located at any position of the first form of FHO as long as the signal intensity from the single label changes depending on the hybridization between the target-dependent fragment and the first form of FHO, the formation of the first duplex, or the melting or post-melting hybridization.

According to an embodiment, the single label is located in the templating portion or the capturing portion of the first form of FHO.

A timing at which a signal is provided (a signal change is caused) may vary depending on the positions of the single label on the first form of FHO.

As an example, when the single label is located in the templating portion of the first form of FHO, the formation of the first duplex may cause a change in the signal from the single label, thereby providing a detectable signal.

As another example, when a single label is located in the capturing portion of the first form of FHO, the hybridization between the target-dependent fragment and the first form of FHO results in a change in the signal from the single label to provide a detectable signal, and the formation of the first duplex maintains the signal at a temperature at which the signal is detected.

In Embodiment 1, when the capturing portion of the first form of FHO is labeled with a single label, a duplex may be formed between the uncleaved TO and the first form of FHO to provide a signal. In this case, the signal from the first duplex and the signal from the duplex between the uncleaved TO and the first form of FHO may be distinguished by (i) measuring a signal (a signal provided from the first duplex) at a temperature at which the duplex between the uncleaved TO and the first form of FHO is dissociated, (ii) measuring a change in the signal provided from the duplex between the uncleaved TO and the first form of FHO as the first duplex is formed, and thus the amount of the duplex between the uncleaved TO and the first form of FHO is reduced, or (iii) measuring a difference between the Tm value of the first duplex and the Tm value of the duplex between the uncleaved TO and the first form of FHO in a melting analysis.

### Embodiment 2 (Single Label on TO)

According to Embodiment 2, the target-dependent fragment has a single label, and the hybridization between the target-dependent fragment and the first form of FHO, or the melting or post-melting hybridization of the first duplex results in a change in the signal from the single label, thereby providing a detectable signal.

A signal is provided upon the hybridization between the target-dependent fragment and the first form of FHO, and the formation of the first duplex maintains the signal at a temperature at which the signal is detected. Specifically, before the target-dependent fragment is hybridized with the capturing portion of the first form of FHO, the single label on the TO *(i.e.,* the single label on the target-dependent fragment) provides a signal with low intensity; whereas when the target-dependent fragment is hybridized with the capturing portion of the first form of FHO, the single label on the target-dependent fragment provides a signal with increased intensity. In addition, the formation of the first duplex between the extended strand and the first form of FHO maintains the increased signal. Then, when the first duplex is melted, the single label provides a low signal, thereby providing a signal (specifically, a change in the signal) indicative of the presence of the target nucleic acid sequence.

According to an embodiment, the single label may be located at any location of the target-dependent fragment as long as the signal intensity from the single label changes depending on the hybridization between the target-dependent fragment and a first form of FHO, the formation of the first duplex, or the melting or post-melting hybridization.

In Embodiment 2, when the target-dependent fragment is labeled with a single label, a duplex may be formed between the uncleaved TO and the first form of FHO to provide a signal. In this case, the signal from the first duplex and the signal from the duplex between the uncleaved TO and the first form of FHO may be distinguished by (i) measuring a signal (a signal provided from the first duplex) at a temperature at which the duplex between the uncleaved TO and the first form of FHO is dissociated, (ii) measuring a change in the signal provided from the duplex between the uncleaved TO and the first form of FHO as the first duplex is formed, and thus the amount of the duplex between the uncleaved TO and the first form of FHO is reduced, or (iii) measuring a difference between the Tm value of the first duplex and the Tm value of the duplex between the uncleaved TO and the first form of FHO in a melting analysis.

### (ii) Label Incorporated into First Duplex during Extension Reaction

The present disclosure may use the label incorporated into the first duplex during the extension reaction in step (b) in order to provide a signal indicative of the presence of the first duplex.

Although the target-dependent fragment or the first form of FHO does not have a label, the first duplex may be successfully labeled using the label incorporated into the first duplex during the extension reaction in step (b).

According to an embodiment, the extension reaction in step (b) is carried out in the presence of a nucleotide having the single label, whereby the single label is incorporated into the first duplex and the formation of the first duplex between the extended strand and the first form of FHO in step (b), or the melting or post-melting of the first duplex results in a change in the signal from the single label, thereby providing the detectable signal.

For example, the target-dependent fragment is hybridized with the capturing portion of the first form of FHO immobilized on the solid substrate and is extended in the presence of a nucleotide labeled with a single fluorescent label to form the first duplex. The fluorescent signal from the first duplex may be detected on the spot of the solid substrate on which the first form of FHO is immobilized. In addition, when the first duplex is melted, the strand having the fluorescent label is released, and the fluorescent signal is no longer detected on the spot. Accordingly, a change in the signal may be provided on the spot by melting the first duplex. That is, the signal indicative of the presence of the extended strand in the first duplex is provided, thereby indicating the presence of the first duplex in step (c).

According to an embodiment, the nucleotide incorporated during the extension reaction have a synthetic unnatural base (*i.e.,* non-natural base), and the first form of FHO has a nucleotide having a complementary synthetic unnatural base with a specific binding affinity to (*i.e.,* capable of base-pairing with) the synthetic unnatural base. Specifically, the nucleotide having the synthetic unnatural base is located at any site in the templating portion of the first form of FHO.

For example, the target-dependent fragment is hybridized with the first form of FHO comprising the nucleotide having a complementary synthetic unnatural base (*e.g.,* iso-dC) to a specific synthetic unnatural base (*e.g.,* iso-dG). The extension is carried out in the presence of the nucleotide having a specific synthetic unnatural base (*e.g.,* iso-dG) labeled with a single fluorescent label to form a first duplex. In the extension reaction, the nucleotide having a specific synthetic unnatural base labeled with the single fluorescent label is incorporated into the site opposite the nucleotide having its complementary synthetic unnatural base on the first form of FHO.

In an embodiment, the synthetic unnatural base labeled with a label incorporated into the first duplex during the extension reaction may be selected from the group consisting of S, Z, V, K, Pa. Pn, Px, Q, MMO2, 5FM, NaM, B, P, J, X, Ds, Dss, and 5SICS.

The labeled synthetic unnatural base and the complementary synthetic unnatural base thereof may form an unnatural base pair, and the unnatural base pair may be, for example, an S-B base pair, a Z-P base pair, a V-J base pair, a K-X base pair, a Pa-Ds base pair, a Pa-Q base pair, a Pn-Ds base pair, a Pn-Dss base pair, a Px-Ds base pair, a MMO2-5SICS base pair, a 5FM-5SICS base pair, or a NaM-5SICS base pair.

In an embodiment, the unnatural base pair generated during the extension reaction may be the same as or different from the unnatural base pair between the first synthetic unnatural base and the second synthetic unnatural base.

The fluorescent signal from the first duplex may be detected on the spot of the solid substrate having the immobilized first form of FHO. In addition, when the first duplex is melted, the strand having the fluorescent label is released, and the fluorescent signal is no longer detected on the spot. Accordingly, a change in the signal may be provided on the spot by the melting of the first duplex. That is, the signal indicative of the presence of the extended strand in the first duplex is provided, thereby indicating the presence of the first duplex in step (c).

When the label incorporated into the first duplex is used during the extension reaction, the label is not included in the duplex between the uncleaved TO and the first form of FHO because the uncleaved TO hybridized with the first form of FHO and is not extended. Accordingly, the duplex between the uncleaved TO and the first form of FHO does not provide a signal.

The types and features of the single label used may be described with reference to the description of the label system using the "target-dependent fragment and/or label linked to the FHO" as described above.

### (iii) At least One Label Linked to Target-dependent Fragment or First Form of FHO and Label Incorporated into First Duplex during Extension Reaction

The present disclosure may utilize a label system using a combination of a label linked to the target-dependent fragment and/or the first form of FHO and a label incorporated into the first duplex during the extension reaction.

The label incorporated into the first duplex during the extension reaction may be a label incorporated into the extended sequence during the extension reaction in which the target-dependent fragment is hybridized with the first form of FHO in step (b) and extended.

In an embodiment, the first form of FHO has any one label of the interactive dual labels comprising the reporter molecule and the quencher molecule, and the extension reaction in step (b) is carried out in the presence of a nucleotide having the other label of the interactive dual labels, whereby the label is incorporated into the extended sequence; the formation of the first duplex between the extended strand and the first form of FHO in step (b), or the melting or post-melting hybridization of the first duplex results in a change in the signal from the interactive dual labels, thereby providing a detectable signal.

In particular, the nucleotide incorporated during the extension reaction have a synthetic unnatural base, and the first form of FHO has a nucleotide having a complementary synthetic unnatural base with a specific binding affinity to the synthetic unnatural base.

In a certain embodiment, the first form of FHO has any one label of the interactive dual labels comprising the reporter molecule and the quencher molecule, the templating portion of the first form of FHO comprises the nucleotide having a synthetic unnatural base, and the extension reaction in step (b) is carried out in the presence of the nucleotide having the other label of the interactive dual labels and a complementary synthetic unnatural base with a specific binding affinity to the synthetic unnatural base, whereby the label is incorporated into the extended sequence; the formation of the first duplex between the extended strand and the first form of FHO in step (b) or the melting or post-melting hybridization of the first duplex results in a change in the signal from the interactive dual labels, thereby providing a detectable signal.

When the first duplex is melted, the reporter molecule and the quencher molecule are separated from each other such that the quencher molecule unquenches the signal from the reporter molecule, thereby providing a signal.

The label site on the first form of FHO and the incorporation site of the incorporated label are determined within a range in which the two labels act as interactive dual labels that induce a change in the signal in the formation and the melting of the first duplex.

In particular, the templating portion of the first form of FHO has the nucleotide having the synthetic unnatural base and the reporter or quencher molecule.

The extension reaction in step (b) is carried out in the presence of the nucleotide having the quencher or reporter molecule and the complementary synthetic unnatural base with a specific binding affinity to the synthetic unnatural base in the first form of FHO. In step (b), the two synthetic unnatural bases of the first duplex form a base pair, and quench a signal from the reporter molecule by the quencher molecule to induce a change in the signal, thereby providing a signal. Alternatively, the target-dependent fragment has the reporter or quencher molecule, and the templating portion of the first form of FHO has the nucleotide having the synthetic unnatural base. The extension reaction in step (b) is carried out in the presence of the nucleotide having the quencher or reporter molecule and the complementary synthetic unnatural base with a specific binding affinity to the synthetic unnatural base in the first form of FHO. The two synthetic unnatural bases of the first duplex in step (b) form a base pair and induce a change in the signal from the reporter molecule by quenching, thereby providing a signal.

As another example, the target-dependent fragment comprising the reporter or quencher molecule is hybridized with the first form of FHO comprising the nucleotide having the complementary synthetic unnatural base (*e.g.,* isodC) with a specific binding affinity to the specific synthetic unnatural base (*e.g*., iso-dG). The extension is carried out in the presence of the nucleotide having the specific synthetic unnatural base (*e.g.,* iso-dG) labeled with the quencher or reporter molecule to form the first duplex, and the signal from the reporter molecule is quenched by the quencher molecule. In the extension reaction, the nucleotide having the specific synthetic unnatural base (*e.g.,* iso-dG) is incorporated into the site opposite the nucleotide having the complementary synthetic unnatural base (*e.g.,* isodC).

When the first duplex is formed in step (b), the reporter molecule and the quencher molecule are conformationally in close proximity to each other such that the quencher molecule quenches the signal from the reporter molecule, thereby providing the signal; when the first duplex is melted, the reporter molecule and the quencher molecule are conformationally separated from each other such that the quencher molecule unquenches the signal from the reporter molecule, thereby providing the signal.

When the label incorporated into the first duplex during the extension reaction is used, the label is not incorporated into the duplex between the uncleaved TO and the first form of FHO because the duplex between the uncleaved TO and the first form of FHO is not extended. Accordingly, the duplex does not provide a signal.

### (iv) Intercalating Label

The present disclosure may use an intercalating label that provides a signal indicative of the presence of the first duplex. Since a double-stranded nucleic acid molecule in a sample may provide a signal, an intercalating label is more useful in a solid-phase reaction using the first form of FHO that is immobilized.

In an embodiment, the signal is provided by the intercalating label, and the hybridization between the target-dependent fragment and the first form of FHO in step (b), the formation of the first duplex between the extended strand and the first form of FHO in step (b), or the melting or post-melting hybridization of the first duplex results in a change in the signal from the intercalating label, thereby providing the detectable signal.

Examples of intercalating labels useful in the present invention include SYBR^{™} Green I, PO-PRO^{™}-1, BO-PRO^{™}-1, SYTO^{™} 43, SYTO^{™} 44, SYTO^{™} 45, SYTOX^{™} Blue, POPO^{™}-1, POPO^{™}-3, BOBO^{™}-1, BOBO^{™}-3, LO-PRO^{™}-1, JO-PRO^{™}-1, YO-PRO^{™} 1, TO-PRO^{™} 1, SYTO^{™} 11, SYTO^{™} 13, SYTO^{™} 15, SYTO^{™} 16, SYTO^{™} 20, SYTO^{™} 23, TOTO^{™}-3, YOYO^{™} 3, GelStar^{™}, and thiazole orange. An intercalating dye specifically intervenes in the double-stranded nucleic acid molecule to provide a signal.

This embodiment may also be applied to other embodiments using melting analysis.

For example, the target-dependent fragment is hybridized with the capturing portion of the first form of FHO immobilized on the solid substrate. The extension is carried out in the presence of the intercalating dye (*e.g*., SYBR^{™} Green) to generate the first duplex comprising the intercalating dye. A fluorescent signal provided from the first duplex on the spot of the solid substrate with the immobilized first form of FHO may be detected using an intercalating fluorescent dye. In addition, when the first duplex is melted, the intercalating fluorescent dye is released, and thus a fluorescent signal is no longer detected on the spot. Thus, the signal is provided, thereby indicating the presence of the first duplex in step (c).

When the intercalating label is used, the duplex between the uncleaved TO and the first form of FHO may be formed, thereby providing a signal. In this case, the signal from the first duplex and the signal from the duplex between the uncleaved TO and the first form of FHO may be distinguished by (i) measuring a signal (a signal provided from the first duplex) at a temperature at which the duplex between the uncleaved TO and the first form of FHO is dissociated, (ii) measuring a change in the signal provided from the duplex between the uncleaved TO and the first form of FHO as the first duplex is formed, and thus the amount of the duplex between the uncleaved TO and the first form of FHO is reduced, or (iii) measuring a difference between the Tm value of the first duplex and the Tm value of the duplex between the uncleaved TO and the first form of FHO in a melting analysis.

### B. Detection of Cleavage of First Duplex

According to an embodiment, the detection in step (c) may be carried out by using the signal from the cleavage of the first duplex between the extended strand and the first form of FHO. For example, it may be carried out on the basis of PCEC assay (*see* WO 2012/134195).

In an embodiment, the first duplex comprises a cleavage site to be cleaved by a nucleolytic enzyme, the first duplex provides a detectable signal by cleavage at the cleavage site, and the presence of the extended strand in step (c) is detected by measuring a signal provided from cleavage of the first duplex.

In an embodiment, the first duplex comprises at least one label. The label provides a detectable signal by the cleavage of the first duplex. For example, in the PCEC assay, the detectable signal may be provided by (i) at least one label linked to the target-dependent fragment and/or the first form of FHO, (ii) a label incorporated into the first duplex during the extension reaction, (iii) at least one label linked to the first form of FHO and the label incorporated into the first duplex during the extension reaction, or (iv) an intercalating label.

In a certain embodiment, the first duplex comprises at least one label, the first duplex provides a detectable signal by (i) cleavage at a cleavage site included in the first duplex to be cleaved by a cleavage enzyme, or (ii) sequential cleavage from the 5'-end of the first form of FHO in the first duplex by a 5'-3' exonuclease, and the presence of the extended strand in step (c) is detected by measuring the signal provided from the cleavage of the first duplex.

In an embodiment, as long as the label induces a change in the signal by the cleavage of the first duplex, the label may be linked to any position of the first duplex.

The details on the principles of PCEC assay are referred to International Publication WO 2012/134195.

The PCEC assay-based labeling system may be described as follows:

### (i) Single Label

The present disclosure may provide a signal for the occurrence of cleavage of the first duplex indicative of the presence of the target nucleic acid sequence by using a single label.

The types and features of the single label are referred to the PTOCE assay section described above.

According to an embodiment, the first form of FHO has the single label, the cleavage of the first duplex generates a cleaved fragment comprising the single label, and the signal from the single label before the first duplex is cleaved is different from the signal from the single label after the first duplex is cleaved, such that a difference between these signals allows the occurrence of cleavage of the first duplex to be detected.

According to an embodiment, the TO has the single label, the cleavage of the first duplex forms a cleaved fragment comprising the single label, and the signal from the single label before the first duplex is cleaved is different from the signal from the single label after the first duplex is cleaved, such that a difference between these signals allows the occurrence of cleavage of the first duplex to be detected.

According to an embodiment, the single label that provides a different signal depending on the cleavage of the first duplex is a single label that emits a fluorescent terbium chelate or polarized fluorescence.

According to an embodiment, the single label is linked to the first form of FHO, particularly, the templating portion of the first form of FHO, and more particularly the 5'-end of the templating portion of the first form of FHO.

According to an embodiment, the single label is linked to the TO, particularly, the tagging portion of the TO. Specifically, the single label is located on the TO such that the target-dependent fragment has the single label.

In an embodiment, when the single label is used in the present disclosure, the present disclosure may be implemented in a solid phase using the immobilized first form of FHO. When the present disclosure is implemented in a solid phase by using a single label, the single label linked to the TO or to the first form of FHO may provide a signal indicative of the occurrence of cleavage of the first duplex.

According to an embodiment, the first form of FHO is immobilized on a solid substrate through the 5'-end or 3'-end thereof.

According to an embodiment, the first form of FHO has the single label, the cleavage of the first duplex forms a cleaved fragment comprising the single label, and the cleaved fragment is released from the solid substrate, resulting in a change in the signal on the solid substrate, thereby providing a signal indicative of the occurrence of cleavage of the first duplex.

More particularly, the first form of FHO is immobilized on the solid substrate through the 3'-end thereof, the TO has the single label, the cleavage of the first duplex forms a cleaved fragment comprising the single label, and the cleaved fragment is released from the solid substrate, resulting in a change in the signal on the solid substrate, thereby providing a signal indicative of the occurrence of cleavage of the first duplex.

When the first form of FHO is immobilized through the 3'-end thereof and a single label is used, the single label is linked to the templating portion of the first form of FHO, and the cleavage site of the nucleic acid cleavage enzyme may be the cleavage site of a 5' to 3' exonuclease, a restriction enzyme, or a ribonuclease.

As an example, the target-dependent fragment is hybridized with the first form of FHO immobilized on the solid substrate through the 3'-end and is extended to form a first duplex, thereby creating the cleavage site of the 5' to 3' exonuclease. The 5' to 3' exonuclease cleaves the cleavage site, and thus the first duplex is cleaved (specifically, sequentially cleaved from the 5'-end of the first form of FHO) to release the fluorescent reporter molecule from the 5'-end of the first form of FHO. When the target nucleic acid sequence is present, fluorescence is reduced or extinguished at a spot comprising the immobilized first form of FHO. When the target nucleic acid sequence is absent, the fluorescence is not reduced nor extinguished at the spot comprising the immobilized first form of FHO.

Alternatively, the first form of FHO is immobilized on the solid substrate through the 5'-end thereof, the TO has the single label, the cleavage of the first duplex generates a cleaved fragment comprising the single label, and the cleaved fragment is released from the solid substrate, resulting in a change in the signal on the solid substrate, thereby providing a signal indicative of the occurrence of cleavage of the first duplex.

As an additional example, the target-dependent fragment is hybridized with the first form of FHO (comprising the sequence recognized by the restriction enzyme in the templating portion) immobilized on the solid substrate through the 5'-end and is extended to form the first duplex, resulting in generation of the cleavage site of the restriction enzyme. The restriction enzyme cleaves the first duplex and releases the fluorescent reporter molecule from the 3'-end of the first form of FHO. When the target nucleic acid sequence is present, fluorescence is reduced or extinguished at a spot comprising the immobilized first form of FHO. When the target nucleic acid sequence is absent, the fluorescence is not reduced nor extinguished at the spot comprising the immobilized first form of FHO.

As an additional example, the target-dependent fragment is hybridized with the first form of FHO (comprising the RNA molecules in the templating portion) immobilized on the solid substrate through the 5'-end and is extended to form the first duplex, creating the cleavage site of RNase H. The RNase H cleaves the first duplex to release a fluorescent reporter molecule from the 3'-end of the first form of FHO. When the target nucleic acid sequence is present, fluorescence is reduced or extinguished at a spot comprising the immobilized first form of FHO. When the target nucleic acid sequence is absent, the fluorescence is not reduced nor extinguished at the spot comprising the immobilized first form of FHO.

When the first form of FHO is immobilized on the solid substrate through the 5'-end and a single label is used, it is preferred that the single label is linked to the first form of FHO or TO and a cleavage site of the nucleic acid cleavage enzyme such as restriction enzyme or ribonuclease is generated.

The single label used in a solid phase reaction does not require specific features. For the solid phase reaction, any single label may be used. This is because, after the cleavage of the first duplex, a difference in a signal depending on the occurrence of cleavage of the first duplex may be induced by the presence or absence of the single label on the solid substrate.

The single label may be linked to the first form of FHO or TO using conventional methods. Specifically, the label is linked to the first form of FHO or TO via a spacer containing at least three carbon atoms (*e.g.,* a 3-carbon spacer, a 6-carbon spacer, or a 12-carbon spacer).

According to an embodiment, the single label linked to the first form of FHO is at the 5'-end or at a site of 1 to 5 nucleotides apart therefrom. Alternatively, the single label linked to the first form of FHO is at the 3'-end or at a site of 1 to 5 nucleotides apart therefrom.

According to a certain embodiment, a single label linked to the TO is at the 5'-end or at a site of 1 to 5 nucleotides apart therefrom.

### (ii) Interactive Dual Label

The present disclosure may provide a signal for the occurrence of cleavage of the first duplex indicative of the presence of the target nucleic acid sequence by using interactive dual labels.

The types and features of the interactive dual labels are referred to the PTOCE assay section described above.

According to an embodiment of the present invention, the interactive dual labels are linked to the first form of FHO.

According to an embodiment of the present invention, the cleavage site to be cleaved by a nucleic acid cleavage enzyme is located between the reporter molecule and the quencher molecule linked to the first form of FHO, the quencher molecule quenches the signal from the reporter molecule before the first duplex is formed, the cleavage of the first duplex separates the reporter molecule and the quencher molecule from each other, and the occurrence of cleavage of the first duplex is detected by measuring the signal from the label.

The interactive label system of the present disclosure is useful in liquid and solid phases.

When the interactive label system is used, specifically, the cleavage site generated in the first duplex may be the cleavage site of the 5' to 3' exonuclease, restriction enzyme or ribonuclease.

As an example, the target-dependent fragment is hybridized with the first form of FHO and is extended to form the first duplex, whereby the cleavage site of the 5' to 3' exonuclease is generated. The cleavage site of the 5' to 3' exonuclease is located between the reporter molecule and the quencher molecule linked to the first form of FHO.

Before the first duplex is formed, the quencher molecule is located at a site suitable for quenching the signal from the reporter molecule.

In particular, quenching occurs when the two labels are in close proximity, depending on the distance on the first form of FHO or are in close proximity in a three-dimensional manner by forming a conformational structure such as a random coil and a hairpin structure.

The 5' to 3' exonuclease cleaves (specifically, sequentially cleaves) the 5'-end of the first duplex and releases the fluorescent reporter molecule, resulting in a change in the signal from the fluorescent reporter molecule. The change in the fluorescent signal is measured to detect the occurrence of cleavage of the first duplex, which determines the presence or absence of the target nucleic acid sequence.

When the quencher molecule is fluorescent, it is preferable to measure the change using the signal from the quencher molecule.

As another example, the target-dependent fragment is hybridized with the first form of FHO and is extended to form the first duplex, whereby the cleavage site of the restriction enzyme is generated. The cleavage site of the restriction enzyme is located between the reporter molecule and the quencher molecule linked to the first form of FHO. The restriction enzyme cleaves the first duplex at the cleavage site and releases the fluorescent reporter molecule, resulting in a change in the signal from the fluorescent reporter molecule. The change in the fluorescent signal is measured to detect the occurrence of cleavage of the first duplex, which determines the presence or absence of the target nucleic acid sequence.

As another example, the target-dependent fragment is hybridized with the first form of FHO (comprising the RNA molecule in the templating portion) and is extended to form the first duplex, thereby generating the cleavage site of RNase. The cleavage site of the RNase is located between the reporter molecule and the quencher molecule linked to the first form of FHO. The RNase cleaves the first duplex at the cleavage site and releases the fluorescent fluorescence reporter molecule, resulting in a change in the signal from the fluorescent reporter molecule. The change in the fluorescent signal is measured to detect the occurrence of cleavage of the first duplex, which determines the presence or absence of the target nucleic acid sequence.

According to an embodiment, at least one of the reporter molecule and the quencher molecule is linked to the templating portion of the first form of FHO, and in particular, the 5'-end of the first form of FHO.

According to an embodiment, both the reporter molecule and the quencher molecule are linked to the templating portion of the first form of FHO.

According to an embodiment, one of the reporter molecule and the quencher molecule is linked to the 5'-end of the first form of FHO, and the other is linked to the 3'-end of the first form of FHO.

According to an embodiment, the reporter molecule or quencher molecule linked to the templating portion of the first form of FHO is located at the 5'-end thereof or at a site of 1 to 5 nucleotides apart from the 5'-end thereof. For example, the quencher molecule may be located at the 5'-end of the templating portion of the first form of FHO, or at a site of 1 to 5 nucleotides apart from the 5'-end of the templating portion of the first form of FHO, and the reporter molecule may be located at a site of 5 to 50 nucleotides apart from the quencher molecule.

According to an embodiment, the interactive dual labels are located at a suitable site to maintain a quenching state between the interactive dual labels when the first duplex is formed and to become an unquenching state between the interactive dual labels when the label is released by the cleavage of the first duplex.

In consideration of real-time signal provision during the cleavage of the first duplex, the reporter molecule and the quencher molecule are located at a site of up to 25 nucleotides apart from each other, specifically up to 20 nucleotides, more specifically up to 15 nucleotides, even more specifically up to 10 nucleotides. According to an embodiment of the present invention, the reporter molecule and the quencher molecule are located at a site of at least 3 nucleotides apart from each other, specifically at least 4 nucleotides, more specifically at least 5 nucleotides, and even more specifically at least 6 nucleotides.

When the first duplex is formed, the reporter molecule and the quencher molecule on the first form of FHO may be conformationally separated such that the quencher molecule unquenches the signal from the reporter molecule. The cleavage of the first duplex may completely separate the reporter molecule from the quencher molecule, thereby making it possible to increase a change in the signal caused by unquenching.

In addition, since a cleaved fragment having the label (*e.g.,* the reporter molecule) is generated, under more flexible or convenient conditions (*e.g*., highly stringent conditions or post-wash conditions in a solid phase), a signal from the label linked to the cleaved fragment may be directly detected to analyze the occurrence of cleavage of the first duplex.

According to an embodiment, one of the interactive dual labels linked to the immobilized first form of FHO remains on the solid substrate after the cleavage of the first duplex.

According to an embodiment, when the first form of FHO immobilized on the solid substrate has the interactive dual labels and the 5' to 3' exonuclease is used as a nucleic acid cleavage enzyme, one of the interactive dual labels may reliably remain on the solid substrate after the cleavage of the first duplex by providing conditions suitable for the separation of the fragment of the first form of FHO from the first duplex or by imparting resistance to the activity of the 5' to 3' exonuclease to the inner nucleotide of the first form of FHO.

According to an embodiment, the resistance to the activity of the 5' to 3' exonuclease is imparted by the nucleotide with a backbone that has a resistance to the activity of the 5' to 3' exonuclease, comprising various phosphorothioate linkages, phosphonate linkages, phosphoroamidate linkages, and 2'-carbohydrate modifications, more particularly, phosphorothioate linkages, alkyl phosphotriester linkages, aryl phosphotriester linkages, alkyl phosphonate linkages, aryl phosphonate linkages, aryl phosphonate linkages, hydrogen phosphonate linkages, alkyl phosphoroamidate linkages, aryl phosphoroamidate linkages, phosphoroselenate linkages, 2'-O-aminopropyl modifications, 2'-O-alkyl modifications, 2'-O-allyl modifications, 2'-O-butyl modifications, α-anomeric oligodeoxynucleotides, and 1-(4'-thio-β-D-ribofuranosyl) modifications.

Reporter molecules and quencher molecules that may be used in the present disclosure are referred to those described in the PTOCE assay.

### (iii) Intercalating Label

According to the present invention, an intercalating label is used to correlate the cleavage reaction of the first duplex with the provision of the signal indicative of the presence of the target nucleic acid sequence.

Since a double-stranded nucleic acid molecule present in a sample can provide a signal, the intercalating label is more useful in a solid phase reaction using the immobilized first duplex.

According to an embodiment, the first duplex is immobilized on the solid substrate through the 5'-end or 3'-end thereof, the intercalating dye is used as a label, the cleavage of the first duplex forms a cleaved fragment comprising the intercalating dye, and the cleaved fragment is released from the solid substrate, resulting in a change in the signal on the solid substrate, thereby providing the signal indicative of the occurrence of cleavage of the first duplex. In this case, the cleavage reaction of the first duplex is preferably carried out using a restriction enzyme or RNase.

According to an embodiment, the cleaved fragment that is not immobilized on the solid substrate is dissociated from the solid substrate.

Examples of intercalating dyes useful in the present disclosure are referred to the disclosure in the PTOCE assay section.

### C. Detection of Duplex between Extended Strand and Signaling Oligonucleotide

According to an embodiment, the detection in step (c) may be carried out by using the signal from the duplex between the extended strand and the signaling oligonucleotide. For example, it may be carried out on the basis of PCE-SH assay (*see* WO 2013/115442).

According to an embodiment, the method additionally uses a signaling oligonucleotide (SO) comprising a hybridizing nucleotide sequence with the extended strand and a label, a duplex between the SO and the extended strand provides a detectable signal and the presence of the extended strand in step (c) is detected by measuring a signal provided from the duplex between the SO and the extended strand.

In the PCE-SH assay, the detectable signal may be provided by (i) a label linked to the SO, (ii) a combination of the label linked to the SO and a label linked to the target-dependent fragment, (iii) a combination of the label linked to the SO and a label incorporated into the extended strand during the extension reaction in step (b), or (iv) a combination of the label linked to the SO and an intercalating label.

The details on the principles of PCE-SH assay are referred to International Publication WO 2013/115442.

A label system useful for the PCE-SH assay may be described as follows:

### (i) Single Label Linked to SO

The present disclosure may provide a signal for the generation of the extended strand indicative of the presence of the target nucleic acid sequence by using a single label.

According to an embodiment, the SO is linked with a single label and the hybridization between the SO and the extended strand results in a change in the signal from the single label, thereby providing the detectable signal.

The types and features of the single label are referred to the disclosure in the PTOCE assay section.

According to an embodiment, the single label on the SO is located at a site of 1 to 15 nucleotides, 1 to 10 nucleotides, or 1 to 5 nucleotides apart from the 5'-end or 3'-end, specifically located at the central portion of the SO.

### (ii) SO Intra-strand Interactive Dual Labels

The types and features of the intra-strand interactive dual labels are referred to the disclosure in the PTOCE assay section.

According to an embodiment, the SO is labeled with the interactive dual labels comprising the reporter molecule and the quencher molecule, and the hybridization between the SO and the extended strand results in a change in the signal from the interactive dual labels, thereby providing a detectable signal. Before the SO is hybridized, the reporter molecule and the quencher molecule on the SO are conformationally in close proximity to each other such that the quencher molecule quenches the signal from the reporter molecule, and when the SO is hybridized, the reporter molecule and the quencher molecule are conformationally separated from each other such that the quencher molecule unquenches the signal from the reporter molecule, resulting in a change in the signal from the interactive dual labels.

According to an embodiment, the reporter molecule and the quencher molecule are located at the 5'-end (or 3'-end) and the 3'-end (or 5'-end) of the SO. According to an embodiment of the present invention, one of the reporter molecule and the quencher molecule linked to the SO is located at the 5'-end or at a site of 1 to 5 nucleotides apart from the 5'-end, and the other is located at a site for quenching or unquenching the signal of the reporter molecule depending on the conformation of the SO.

According to an embodiment, one of the reporter molecule and the quencher molecule linked to the SO is located at the 3'-end or at a site of 1 to 5 nucleotides apart from the 3'-end, and the other is located at a site for quenching or unquenching the signal of the reporter molecule depending on the conformation of the SO.

According to an embodiment, the reporter molecule and the quencher molecule are located apart from each other within 80 nucleotides, specifically within 60 nucleotides, more specifically within 30 nucleotides, and even more specifically within 25 nucleotides. According to an embodiment, the reporter molecule and the quencher molecule are spaced at least 4 nucleotides apart, at least 6 nucleotides, at least 10 nucleotides, or at least 15 nucleotides.

### (iii) Inter-strand Interactive Dual Label

In embodiments using inter-strand interactive dual labels, the extended strand has one of the interactive dual labels comprising the reporter molecule and the quencher molecule, and the SO has the other of the interactive dual labels.

Embodiments using interstrand interactive dual labels may be implemented according to the following three manners:
According to a first manner, the SO comprises one label of the reporter molecule and the quencher molecule of interactive dual labels, the target-dependent fragment comprises the other of the reporter molecule and the quencher molecule, and the extended strand comprises a label derived from the target-dependent fragment; the hybridization between the SO and the extended strand results in a change in the signal from the interactive dual labels, thereby providing a detectable signal.

The label linked to the SO may be the reporter molecule or the quencher molecule, and the label linked to the target-dependent fragment may be the quencher molecule or the reporter molecule.

By determining the site of the label on the TO in consideration of the cleavage site of the TO, the label may be allowed to exist on the target-dependent fragment.

As long as the label interacts with the label linked to the SO to induce a change in the signal in the hybridization with the SO, the label may be linked to any site of the target-dependent fragment (*e.g*., the tagging portion of the TO). As long as the label interacts with the label linked to the target-dependent fragment to induce a change in the signal in the hybridization with the target-dependent fragment, the label may be linked to any site of the SO (*e.g.,* the 5'-end of the SO).

According to a second manner, the SO comprises one label of the reporter molecule and the quencher molecule of interactive dual labels, the templating portion of the first form of FHO comprises the nucleotide having the synthetic unnatural base, and the extension reaction in step (b) is carried out in the presence of the complementary synthetic unnatural base with a specific binding affinity to the synthetic unnatural base and the nucleotide having the other of the reporter molecule and the quencher molecule, whereby the label is incorporated into the extended strand; the hybridization between the SO and the extended strand results in a change in the signal from the interactive dual labels, thereby providing a detectable signal. As long as the label incorporated into the extended strand interacts with the label bound to the SO to induce a change in the signal in the hybridization with the SO, the label may be linked to any site of the extended strand (*e.g.,* the 3'-end of the extended strand). As long as the label interacts with the label incorporated into the extended strand to induce a change in the signal in the hybridization with the extended strand, the label may bind to any site of the SO (*e.g.,* the 5'-end of the SO).

According to a third manner, the SO comprises one label of the reporter molecule and the quencher molecule of interactive dual labels, and the extension reaction in step (b) is carried out in the presence of the nucleotide having the other of the reporter molecule and the quencher molecule, whereby the label is incorporated into the extended strand; the hybridization between the SO and the extended strand results in a change in the signal from the interactive dual labels, thereby providing a detectable signal.

### (iv) Interactive Dual Labels Using Two SOs

In an embodiment of the interactive dual labels using two SOs, the method of the present invention further comprises one additional SO, the additional SO comprises a sequence complementary to the extended strand, and the two SOs are hybridized with the extended strand adjacent to each other; each of the two SOs comprises one label of the reporter molecule and the quencher molecule of the interactive dual labels, and the hybridization between the two SOs and the extended strand results in a change in the signal from the interactive dual labels, thereby providing a detectable signal.

Specifically, at least one SO of the two SOs comprises a portion for hybridizing with the extended sequence newly generated in the extension reaction in step (b).

The principle of the interactive dual labels using two SOs is as follows: the two SOs are hybridized with the extended strand. Because in the hybridization, the two SOs are hybridized with the extended strand adjacent to each other, the reporter molecules and quencher molecules in the two SOs are in close proximity to each other such that the quencher molecule quenches the signal from the reporter molecule, resulting in a change in the signal from the interactive dual labels (*e.g.,* a decrease in the signal from the reporter molecule). Meanwhile, when there is no target nucleic acid sequence, the extended strand is not generated, and thus the two SOs are not hybridized. The reporter molecule and quencher molecule in the two unhybridized SOs are separated from each other to provide the signal from the reporter molecule.

According to an embodiment, the two SOs may be hybridized at any site of the extended strand so long as they are hybridized to the extended strand such that the quencher molecule may be allowed to quench the signal from the reporter molecule. For example, the two SOs may be positioned immediately adjacent to each other, or 1 to 5 nucleotides apart from each other.

According to an embodiment, when the two SOs are hybridized with the extended strand adjacent to each other, the reporter molecule and the quencher molecule may be linked to any site of the two SOs so long as the quencher molecule may be allowed to quench the signal from the reporter molecule. For example, the reporter molecule or the quencher molecule may be linked to the 5'-end or to a site of 1 to 5 nucleotides apart from the 5'-end of one SO, and the quencher molecule or the reporter molecule may be linked to the 3'-end or to a site of 1 to 5 nucleotides apart from the 3'-end of the other SO.

### D. Detection of Cleavage of Duplex between Extended Strand and Signaling Oligonucleotide

According to an embodiment, the detection in step (c) may be carried out using the signal provided from the cleavage of the duplex between the extended strand in the first duplex and the signaling oligonucleotide. For example, it may be carried out according to PCE-SC assay (see WO 2013/157821).

According to an embodiment, the method additionally uses a signaling oligonucleotide (SO) comprising a hybridizing nucleotide sequence with the extended strand and a label, a duplex between the SO and the extended strand comprises a cleavage site to be cleaved by a nucleolytic enzyme, the duplex between the SO and the extended strand provides a detectable signal by cleavage at the cleavage site, and the presence of the extended strand in step (c) is detected by measuring a signal provided from cleavage of the duplex between the SO and the extended strand.

In the PCE-SC assay, the duplex between the SO and the extended strand comprises the interactive dual labels or single label comprising the reporter molecule and the quencher molecule, and a cleavage site to be cleaved by the 5' to 3' exonuclease, ribonuclease or restriction enzyme, wherein the cleavage of the duplex between the SO and the extended strand results in a change in the signal from the interactive dual labels or single label, thereby providing a detectable signal.

The details on the principles of PCE-SC assay are referred to International Publication WO 2013/157821, which is incorporated herein by reference.

A label system useful for the PCE-SC assay may be described as follows:

### (i) Interactive Dual Label

The types and features of interactive dual labels are referred to the disclosure in the PTOCE assay section.

According to an embodiment, the signal indicative of the occurrence of cleavage of the duplex between the extended strand and the SO (*i.e.,* the presence of the target nucleic acid sequence) is provided by the interactive label system, and in particular by a FRET label system (*i.e.,* the interactive dual labels system).

According to an embodiment, the SO has the interactive dual labels comprising the reporter molecule and the quencher molecule, wherein the cleavage site for the nucleic acid cleavage enzyme is located between the reporter molecule and the quencher molecule linked to the SO, the cleavage of the SO in the duplex between the extended strand and the SO separates the reporter molecule and the quencher molecule from each other, and the occurrence of the cleavage reaction for the SO of the duplex between the extended strand and the SO is detected by measuring the signal from the label.

In a certain embodiment, the quencher molecule is located at a site such that the signal from the reporter molecule is quenched before the generation of the duplex between the extended strand and the SO.

In the present disclosure, the interactive label system is used in liquid and solid phases.

When the interactive label system is used, the resulting cleavage site is a cleavage site for the 5' nuclease, restriction enzyme, or ribonuclease.

The nucleic acid cleavage enzyme (*e.g*., 5' nuclease) may cleave the 5'-end portion of the SO of the duplex between the extended strand and the SO to release the reporter molecule, thereby inducing a change in the signal from the reporter molecule. The occurrence of cleavage of the duplex between the extended strand and the SO may be detected by measuring a fluorescent signal for determining the presence of the target nucleic acid sequence.

According to an embodiment, at least one of the reporter molecule and the quencher molecule is linked to the 5'-end of the SO. In a certain embodiment, one of the reporter molecule and the quencher molecule is linked to the 5'-end of the SO, and the other is linked to the 3'-end thereof.

According to an embodiment, one of the reporter molecule and the quencher molecule on the SO is linked to the 5'-end thereof or to a site of 1 to 10 nucleotides apart from the 5'-end thereof, and the other is linked to a site to quench the signal from the reporter molecule before the hybridization of the extended strand and the SO.

According to an embodiment, one of the reporter molecule and the quencher molecule on the SO is linked to the 3'-end thereof or to a site of 1 to 10 nucleotides apart from the 3'-end thereof, and the other is linked to a site to quench the signal from the reporter molecule before the hybridization of the extended strand and the SO.

For example, the reporter molecule on the SO may be linked to the 5'-end thereof or to a site of 1 to 5 nucleotides apart from the 5'-end thereof, and the quencher molecule may be linked to a site of 5 to 80 nucleotides apart from the reporter molecule.

According to an embodiment, the interactive dual labels are linked to a site sufficient to maintain a quenching phenomenon prior to the formation of the duplex between the extended strand and the SO, and induces unquenching upon the cleavage of the duplex between the extended strand and the SO.

When considering real-time signal generation during the cleavage of the duplex between the extended strand and the SO, the reporter molecule and the quencher molecule may be linked to be apart from each other within 80 nucleotides, within 60 nucleotides, within 30 nucleotides, within 25 nucleotides, within 20 nucleotides, within 15 nucleotides, or within 10 nucleotides. According to an embodiment, the reporter molecule and the quencher molecule are separated by at least 3 nucleotides apart, at least 4 nucleotides, at least 5 nucleotides, at least 6 nucleotides, at least 10 nucleotides, or at least 15 nucleotides.

In addition, since a cleaved fragment having a label (*e.g.,* the reporter molecule) is produced, the occurrence of cleavage of the duplex between the extended strand and the SO may be analyzed by directly detecting the signal from the label linked to the cleaved fragment under more flexible or convenient conditions (*e.g*., highly stringent conditions or post-wash conditions on a solid substrate).

According to an embodiment, one of the interactive dual labels linked to the immobilized SO remains on the solid substrate after the cleavage of the duplex between the extended strand and the SO.

According to an embodiment, when the SO immobilized on the solid substrate has the interactive dual labels and the 5' nuclease is used as a nucleic acid cleavage enzyme, one of the interactive dual labels may certainly remain on the solid substrate after the cleavage of the duplex between the extended strand and the SO by providing conditions suitable for dissociating the SO fragment from the duplex between the extended strand and the SO or by imparting a resistance to 5' nuclease activity an internal nucleotide of the SO (*e.g.,* a nucleotide comprising a backbone having a resistance to 5' to 3' exonuclease activity or a nucleotide having a label on the base thereof).

When the SO comprises a 5'-tagging portion that is non-complementary to the extended strand, the reporter or quencher molecule may be linked to the 5'-tagging portion in consideration of the cleavage site.

### (ii) Single Label

In addition, the present disclosure is excellently implemented using a single label system that provides a signal for the generation of the duplex between the SO and the extended strand indicative of the presence of the target nucleic acid sequence.

The specific types and features of the single label are referred to the disclosure in the PTOCE assay.

In an embodiment, the SO has a single label, cleavage of the SO in the duplex between the extended strand and the SO produces a fragment having the single label, and the occurrence of cleavage of the SO in the duplex between the extended strand and the SO is detected by detecting the release of the single labeled fragment. In this case, the signal provided from the single label before the cleavage of the SO is different from the signal provided from the single label after the cleavage of the SO, and the difference in the signal enables the detection of the occurrence of cleavage of the SO of the duplex between the extended strand and the SO.

According to an embodiment, the single label is linked to the 5'-end or 3'-end of the SO.

When the single label is used in the present disclosure, the present disclosure is efficiently implemented in a solid phase using the immobilized SO. According to an embodiment, the SO is immobilized on the solid substrate through the 5'-end or 3'-end thereof.

According to an embodiment, the SO is immobilized on the surface of the solid substrate through the 5'-end or 3'-end thereof, the SO has a single label, and cleavage of the SO in the duplex between the extended strand and the SO produces a fragment having the single label, and the fragment is released from the solid substrate, whereby a change in the signal is provided on the solid substrate to detect the occurrence of cleavage of the SO in the duplex between the extended strand and the SO.

When the SO is immobilized on the solid substrate through the 3'-end thereof, the single label is linked to the 5'-end of the SO, and the resulting cleavage site is a cleavage site for the 5'-nuclease, restriction enzyme, or ribonuclease.

In a certain embodiment, the single label is linked to the 5'-end of the SO or to a site of 1 to 5 nucleotides apart from the 5'-end of the SO. Alternatively, the single label is located at the 3'-end of the SO or at a site of 1 to 5 nucleotides apart from the 3'-end of the SO.

### E. Detection of Duplex between First Form of FHO and Hybridizing Oligonucleotide

According to an embodiment, the detection in step (c) may be carried out by using the signal provided from the duplex between the first form of FHO and the hybridizing oligonucleotide. For example, it may be carried out according to PCE-NH assay (*see* WO 2014/104818).

According to an embodiment, the method additionally uses a hybridizing oligonucleotide (HO) comprising a hybridizing nucleotide sequence to the first form of FHO and a label, a duplex between the HO and the first form of FHO provides a detectable signal and the presence of the extended strand in step (c) is detected by measuring a signal provided from the duplex between the HO and the first form of FHO, the presence of the signal from the duplex between the HO and the first form of FHO indicates the absence of the extended strand.

In the PCE-NH assay, the detectable signal may be provided by (i) a label linked to the HO, (ii) a label linked to the first form of FHO, (iii) a combination of the label linked to the HO and the label linked to the first form of FHO, or (iv) an intercalating label.

The details on the principles of PCE-NH assay are referred to International Publication WO 2014/104818.

A label system useful for the PCE-NH assay may be described as follows:

### (i) Single Label Linked to HO or First Form of FHO

The present disclosure may provide a signal indicative of the absence of the extended strand by hybridizing the HO having the single label with the first form of FHO or by hybridizing the HO with the first form of FHO having the single label.

According to an embodiment, it is necessary to detect the signal at a temperature at which the hybridization between the HO and the first form of FHO is possible.

### (ii) Interactive Dual Labels Linked to HO or First Form of FHO

According to an embodiment, the detectable signal is provided by the interactive dual labels linked to the HO or the first form of FHO.

Specifically, the target-dependent fragment is hybridized with the first form of FHO. Thereafter, the HO labeled with the interactive dual labels comprising the reporter molecule and the quencher molecule is hybridized with the templating portion of the first form of FHO. Then, the target-dependent fragment is extended, and the extension induces the cleavage of the HO to separate the reporter molecule from the quencher molecule, thereby providing the signal indicative of the presence of the extended strand.

In such an embodiment, when the interactive dual labels linked to the nucleotide are relatively in close proximity to each other, a change in the signal between before and after the HO cleavage may be used for signal detection.

When the interactive dual labels are apart from each other, the hybridization between the HO and the first form of FHO induces a conformational separation of the interactive dual labels, so that the signal from the reporter molecule is unquenched even when the HO is not cleaved, thereby providing a change in the signal. In this case, the signal from the cleaved fragment of the HO may be detected at a higher temperature (*e.g*., 95°C.) at which the hybridization between the HO and the first form of FHO may be prevented.

According to an embodiment, the reporter molecule and the quencher molecule may be located at any site of the HO so long as the cleaved HO and uncleaved HO may provide a distinct signal.

In a certain embodiment, the reporter molecule and the quencher molecule are each linked to bothe ends of the HO.

### (iii) Interactive Dual Labels Linked to HO and First Form of FHO

According to an embodiment, a signal is provided that is detectable by the interactive dual labels comprising the reporter molecule and the quencher molecule, one of which is linked to the HO and the other of which is linked to the first form of FHO.

In a certain embodiment, the reporter molecule and the quencher molecule are each linked to the HO and the first form of FHO such that the signal from the reporter molecule is quenched by the quencher molecule when the HO is hybridized with the first form of FHO. The cleavage of the HO induced by the extension of the target-dependent fragment releases the HO from the first form of FHO and separates the reporter molecule from the quencher molecule, causing the quencher molecule to unquench the signal from the reporter molecule, thereby providing a signal indicative of the presence of the extended strand.

According to an embodiment, it is necessary to detect the signal at a temperature at which the hybridization between the HO and the first form of FHO is possible.

According to an embodiment, the HO may be designed to have a hairpin structure. In a certain embodiment, one of the reporter molecule and the quencher molecule is linked to the 3'-end of the HO and the other is linked to the 5'-end of the first form of FHO.

According to an embodiment, a label system, such as interactive dual labels using two HOs, may be used in the method of the present disclosure using the HO. As long as the interactive dual labels can provide the signal of distinguishing the cleaved HO from the uncleaved HO, the interactive dual labels may be linked to any site of the two HOs. The types and sites of the label may be described with reference to the description of the SO.

According to an embodiment, a label system such as FRET using an intercalating dye may be used in the method of the present disclosure using the HO. As long as the FRET label can provide the signal of distinguishing the cleaved HO from the uncleaved HO, the FRET label may be linked to any site of the HO.

### F. Detection of Duplex between Extended Strand and Immobilized Immobilizing Oligonucleotide

According to an embodiment, the detection in step (c) may be carried out by using the signal provided from the duplex between the extended strand and the immobilizing oligonucleotide immobilized on the solid substrate. For example, it may be carried out according to PCE-IH assay (*see* WO 2015/008985).

According to an embodiment, the method additionally uses an immobilizing oligonucleotide (IO) immobilized on a solid substrate, which comprises a hybridizing nucleotide sequence to the extended strand, the extended strand comprises a label, a duplex between the extended strand and the IO provides a detectable signal on the solid substrate, and the presence of the extended strand in step (c) is detected by measuring a signal provided from the duplex between the extended strand and the IO.

In the PCE-IH assay, the detectable signal may be provided by (i) a label linked to the target-dependent fragment, (ii) a label incorporated into the extended strand during the extension reaction in step (b), or (iii) a combination of the label linked to the target-dependent fragment and the label incorporated into the extended strand during the extension reaction in step (b).

The details on the principles of PCE-IH assay are referred to International Publication WO 2015/008985.

A label system useful for the PCE-IH assay may be described as follows:

### (i) Single Label Linked to Target-dependent Fragment

The present invention may provide a signal for the presence of the extended strand indicative of the presence of the target nucleic acid sequence by using a single label. The target-dependent fragment labeled with the single label is hybridized with the first form of FHO and is extended on the first form of FHO to produce an extended strand, and the single label is incorporated into the extended strand. Subsequently, after the extended strand is hybridized with the IO immobilized on the solid substrate to provide a detectable signal on the solid substrate, a signal indicating the presence of the extended strand is detected.

The types and features of the single label that can be used in the above method are referred to the disclosure in the PTOCE assay section.

According to an embodiment, the single label on the TO may be linked to any site so long as the target-dependent fragment released from the TO comprises the single label. According to an embodiment, the single label on the TO may be linked to any site of the tagging portion, for example, at the 5'-end of the tagging portion.

### (ii) Label Incorporated into Extended Strand during Extension Reaction

According to an embodiment, the label used in the present disclosure is a label which is incorporated into the extended strand, the templating portion of the first form of FHO comprises the nucleotide comprising a synthetic unnatural base, and the extension reaction in step (b) is carried out in the presence of the nucleotide comprising a label and a complementary synthetic unnatural base with a specific binding affinity to the synthetic unnatural base, whereby the label is incorporated into the extended strand. Subsequently, the extended strand is hybridized with the IO immobilized on the solid substrate to provide a detectable signal on the solid substrate, and a signal indicating the presence of the extended strand is detected.

### (iii) Combination of Single Label on Target-dependent Fragment and Label Incorporated into Extended Strand

According to an embodiment, the label used in the present disclosure is a combination of the single label linked to the target-dependent fragment and the label incorporated into the extended strand during the extension reaction, and the single label and the incorporated label are interactive dual labels comprising a pair of acceptor and donor.

As an example, the TO comprising the tagging portion labeled with a fluorescent single label (donor) is hybridized with the target nucleic acid sequence, and then cleaved. The target-dependent fragment comprising the fluorescent single label (donor) is hybridized with the first form of FHO comprising a synthetic unnatural base (*e.g.,* iso-dC) at the templating portion, and a complementary synthetic unnatural base (*e.g.,* iso-dG) to the synthetic unnatural base labeled with the acceptor is incorporated into the opposite side of the iso-dC during the extension reaction of the target-dependent fragment to introduce the acceptor into the extended strand, whereby interactive dual labels are formed on the extended strand. Then, the extended strand is hybridized with the IO immobilized on the solid substrate, irradiated with excitation light for the donor to cause energy transfer from the donor to the acceptor, thereby inducing a change in the signal from the acceptor to provide a detectable signal on the solid substrate.

### (iv) Intercalating Dye

According to an embodiment, the hybridization between the extended strand and the IO is carried out in the presence of an intercalating dye, the label incorporated into in the extended strand is an acceptor receiving a signal from the intercalating dye, and the signal from the intercalating dye intercalated into the duplex between the extended strand and the IO is transferred to the acceptor, and the acceptor provides a detectable signal.

For example, the TO comprising the tagging portion labeled with a fluorescent label (acceptor) is hybridized with the target nucleic acid sequence, and then cleaved. The target-dependent fragment comprising the fluorescent label (acceptor) is hybridized with the first form of FHO and is extended to generate an extended strand. Then, the extended strand is hybridized with the IO immobilized on the solid substrate in the presence of the intercalating dye as a donor, and the intercalating dye is intercalated into the hybrid. After the donor is irradiated with excitation light, energy transfer from the donor to the acceptor is induced, thereby inducing a change in the signal from the acceptor to provide a detectable signal on the solid substrate.

Alternatively, the hybridization between the extended strand and the IO may be carried out in the presence of the intercalating dye without using the label incorporated into in the extended strand, and the presence of the target nucleic acid sequence is analyzed by detecting a signal from the intercalating dye intercalated into the duplex between the extended strand and the IO.

### G. Detection of Third Duplex Using Third Form of FHO

According to an embodiment, the detection in step (c) may be carried out by using the signal provided from the duplex between the extended strand and the additional third form of FHO. For example, it may be carried out according to PTO cleavage and extension-dependent extension (PTOCE-E) assay (*see* WO 2019/0666461).

The third form of FHO and the third duplex of the present disclosure may correspond to the second CTO and the second extended duplex, respectively, in the above-described PTOCE-E method.

According to an embodiment, the method performs the following steps instead of step (c):
(c-1) hybridizing the extended strand with a third form of FHO, wherein the third form of FHO comprises in a 3'to 5' direction (i) a capturing portion comprising a hybridizing nucleotide sequence with the extended strand and (ii) a templating portion comprising a non-hybridizing nucleotide sequence with the extended strand; wherein the extended strand is hybridized with the capturing portion of the third form of FHO;
(c-2) performing an extension reaction using the resultant of the step (c-1) and a template-dependent nucleic acid polymerase, wherein the extended strand hybridized with the capturing portion of the third form of FHO is further extended to generate a second extended strand comprising a second extended sequence complementary to the templating portion of the third form of FHO, thereby forming a third duplex between the second extended strand and the third form of FHO; and
(c-3) detecting the presence of the second extended strand, whereby the presence of the second extended strand indicates the presence of the target nucleic acid sequence.

The PTOCE-E assay may be carried out according to various assay methods such as: A. detection of a third duplex; B. detection of the cleavage of the third duplex; C. detection of a duplex between the second extended strand and a signaling oligonucleotide; D. detection of the cleavage of the duplex between the second extended strand and the signaling oligonucleotide; E. detection of a duplex between the third form of FHO and the hybridizing oligonucleotide; and detection of a duplex between the second extended strand and the immobilizing oligonucleotide.

The details on the principles of PTOCE-E assay are referred to International Publication WO 2019/0666461.

In an embodiment, when the method forms the second duplex, the detection of the presence of the second duplex in step (c) may be carried out by various methods known in the art. For example, when the method forms the second duplex, the detection of the presence of the second duplex in step (c) may be carried out by using a dual quenching assay (WO 2016/101959) in combination with a melting analysis.

In an embodiment, when the method forms the second duplex, the reporter molecule and a first quencher molecule are linked to the TO, and the second form of FHO is linked to a second quencher molecule. The TO may be cleaved dependent on the presence of the target nucleic acid sequence during the reaction of step (a) to release the target-dependent fragment comprising the reporter molecule, and the reporter molecule linked to the target-dependent fragment and the second quencher molecule linked to the second form of FHO may provide a detectable signal by the formation and dissociation of the second duplex between the target-dependent fragment and the second form of FHO depending on temperature. The presence of the second duplex in step (c) may be detected by measuring the signal provided from the second duplex.

In an embodiment, the signal of the second duplex may be measured by a melting analysis or by a post-melting hybridization analysis.

According to an embodiment, the detection of the presence of the second duplex in step (c) is carried out through melting analysis after hybridization. For example, the detection of the second duplex in step (c) is carried out through a melting analysis in which the formed second duplex is melted in a certain range of temperatures to provide a signal indicative of the presence of the second duplex.

According to an embodiment, the detection of the presence of the second duplex in step (c) is carried out through a post-melting hybridization analysis. For example, the detection of the second duplex in step (c) is carried out through a hybridization analysis in which the melted second duplex is hybridized in a certain range of temperatures to provide a signal indicative of the presence of the second duplex.

In an embodiment, when the method forms a second duplex, the reporter molecule on the TO may be linked to a site at which the reporter molecule may be released together with the target-dependent fragment, and the first quencher molecule on the TO may be linked to a site at which the first quencher molecule is in close proximity to the reporter molecule on the TO and quenches a signal from the reporter molecule before the TO is cleaved in step (a), but is not released together with the target-dependent fragment after the TO is cleaved in step (a). For example, the reporter molecule may be linked to the tagging portion of the TO, and the first quencher molecule may be linked to the targeting portion of the TO. The second quencher molecule on the second form of FHO may be linked to a site at which the signal from the reporter molecule may be quenched when the second duplex is formed.

The TO and FHO (the first form of FHO or the second form of FHO) used in the methods of the present disclosure, and optionally the SO, HO and IO may composed of naturally occurring dNMPs. Alternatively, the TO and FHO, and optionally the SO, HO and IO may comprise modified or unnatural nucleotides such as Peptide Nucleic Acid (PNA, *see* WO 92/20702) and Locked Nucleic Acid (LNA, *see* WO 98/22489, WO 98/39352 and WO 99/14226). The TO and FHO, and the third form of FHO, and optionally the SO, HO and IO may comprise universal bases such as deoxyinosine, inosine, 1-(2'-deoxy-beta-D-ribofuranosyl)-3-nitropyrrole, and 5-nitroindole. The term "universal bases" means bases capable of base pairing with natural DNA/RNA bases with little discrimination between them.

As described above, in an embodiment, when the TO comprises the 5'-tagging portion and 3'-targeting portion, the TO may be cleaved at a site spaced apart in the 3'-direction from the 3'-end of the 5'-tagging portion of the TO. The cleavage site may be located at a part of the 5'-end of the 3'-targeting portion of the TO. When the target-dependent fragment comprises a part of the 5'-end of the 3'-targeting portion of the TO, the site at which the FHO is hybridized with a part of the 5'-end of the 3'-targeting portion may comprise a universal base, a degenerate sequence, or a combination thereof. For example, if the TO is cleaved at a site one nucleotide in the 3'-direction apart from the 3'-end of the 5'-tagging portion of the TO, it is advantageous that a part of the 5'-end of the capturing portion of the FHO comprises a universal base for the hybridization with the nucleotide. If the TO is cleaved at a site two nucleotides in the 3'-direction apart from the 3'-end of the 5'-tagging portion of the TO, it is advantageous that the 5'-end of the capturing portion of the FHO comprises a degenerate sequence, and the nucleotide adjacent in the 3'-direction comprises a universal base. As such, when the TO is cleaved at various sites of a part of the 5'-end of the 3'-targeting portion, it is useful to use the universal base and the degenerate sequence in the FHO. In addition, under the induction of upstream primer extension-dependent cleavage, when the TO having the same 5'-tagging portion is used for screening a plurality of target nucleic acid sequences, target-dependent fragments having different parts of the 5'-end of the 3'-targeting portion may be generated. In this case, the universal base and the degenerate sequence are useful for the FHO. The strategy of using the universal base and degenerate sequence in the FHO allows the use of one type of FHO or the least type of FHO for screening a plurality of target nucleic acid sequences.

According to an embodiment, the method of the present disclosure comprises repeating all or some of steps (a)-(c), comprising denaturation. This repetition amplifies the target nucleic acid sequence and/or signal.

According to an embodiment of the present invention, the method of the present disclosure further comprises a step of melting the duplex (the first duplex or the second duplex) or melting the duplex, followed by hybridization after step (c) to provide a detectable signal, and measuring the detectable signal to determine the presence of the extended strand in the first duplex or the presence of the second duplex.

The denaturation may be carried out through known techniques including, but not limited to, heat, alkali, formamide, urea and glycoxal treatment, enzymatic methods (*e.g.*, helicase action), and binding proteins. For example, the denaturation may be achieved by heat treatment in a temperature range of 80-105°C. The general method of denaturation described above is disclosed in Joseph Sambrook, et al., Molecular Cloning, A Laboratory Manual, Cold Spring Harbor Laboratory Press, Cold Spring Harbor, N.Y. (2001).

When the upstream primer is used as the upstream oligonucleotide in the repeating process, the method of the present disclosure is carried out in the presence of a downstream primer, specifically, by a PCR method.

According to an embodiment, steps (a)-(c) above are carried out in one reaction vessel, or some of steps (a)-(c) above are carried out in a separate reaction vessel.

The present disclosure does not require target nucleic acid sequences, which is to be detected and/or amplified, to have any particular sequence or length, comprising all DNA (gDNA and cDNA) and RNA molecules.

When mRNA is used as an initial material, a reverse transcription step is essential prior to performing the annealing step, and details thereof are disclosed in Joseph Sambrook, et al., Molecular Cloning, A Laboratory Manual, Cold Spring Harbor Laboratory Press, Cold Spring Harbor, N.Y. (2001); and Noonan, K. F. et al., Nucleic Acids Res. 16:10366 (1988). For the reverse transcription reaction, an oligonucleotide dT primer hybridizing with a random hexamer or mRNA may be used.

The target nucleic acid sequences that may be detected and/or amplified in the present disclosure comprise any naturally occurring prokaryotic nucleic acid, eukaryotic (*e.g.,* protozoa and parasitic animals, fungi, yeast, higher plants, lower animals, and higher animals comprising mammals and humans) nucleic acid, viral (*e.g*., herpes virus, HIV, influenza virus, Epstein-Barr virus, hepatitis virus, poliovirus, *etc*.) nucleic acid, or viroid nucleic acid.

In addition, the present disclosure is useful for the detection of a nucleotide variation. Specifically, the target nucleic acid sequence comprises a nucleotide variation. As used herein, the term "nucleotide variation" refers to all single or multiple nucleotide substitutions, deletions, or insertions in a DNA sequence at a specific site in consecutive DNA segments or DNA segments having similar sequences. These consecutive DNA fragments comprise one gene or any other site of one chromosome. Such a nucleotide variation may be a mutant or polymorphic allele variations. For example, the nucleotide variation detected in the present disclosure comprises single nucleotide polymorphism (SNP), mutation, deletion, insertion, substitution and translocation. Examples of nucleotide variations comprise various variations in the human genome (*e.g*., variations in the methylenetetrahydrofolate reductase (MTHFR) gene), variations related to drug resistance of the pathogen, and cancer-associated variations. As used herein, the term "nucleotide variation" comprises any variation at a particular site of a DNA molecule. That is, the term "nucleotide variation" comprises wild-type at a particular site of the DNA molecule and any variant thereof.

In the present disclosure for detecting a nucleotide variation of a target nucleic acid sequence, if the primer or probe used has a sequence complementary to the nucleotide variation of the target nucleic acid sequence, the target nucleic acid sequence comprising the nucleotide variation is described herein as a matching template. If the primer or probe used has a sequence that is non-complementary to the nucleotide variation of the target nucleic acid sequence, the target nucleic acid sequence comprising the nucleotide variation is described herein as a mismatching template.

To detect a nucleotide variation, the 3'-end of the upstream primer can be designed to be located opposite the nucleotide variation site in the target nucleic acid sequence. According to an embodiment, the 3'-end of the upstream primer has a sequence complementary to the nucleotide variation of the target nucleic acid sequence. The 3'-end of the upstream primer with a sequence complementary to the nucleotide variation of the target nucleic acid sequence is annealed and extended to the matching template to induce TO cleavage. As a result, the target-dependent fragment is hybridized with the FHO to provide a signal. On the other hand, when the 3'-end of the upstream primer is mismatched to the nucleotide variation in the mismatching template, even if the upstream primer is hybridized with the mismatching template, the upstream primer is not extended under the conditions that the annealing of the 3'-end of the primer for extension reaction is necessary, and thus no signal is provided.

Alternatively, the TO cleavage that is dependent on the hybridization of the TO having a sequence complementary to the nucleotide variation of the target nucleic acid sequence may be used. For example, under controlled conditions, the TO having a sequence complementary to the nucleotide variation of the target nucleic acid sequence is cleaved after hybridized with the matching template. As a result, the target-dependent fragment is hybridized with the FHO to provide a target signal. On the other hand, under controlled conditions, the TO is not hybridized with a mismatching template having a sequence that is non-complementary to the nucleotide variation site and is not cleaved. In this case, specifically, a complementary sequence to the nucleotide variation of the TO is located at the center of the targeting portion of the TO.

Alternatively, for the detection of nucleotide variations, it is preferred that a part of the 5'-end of the 3'-targeting portion of the TO is located at the nucleotide variation of the target nucleic acid sequence, and a part of the 5'-end of the 3'-targeting portion of the TO has a sequence complementary to the nucleotide variation of the target nucleic acid sequence.

In an embodiment for the detection of a single nucleotide variation, the 5'-end of the 3'-targeting portion of the TO has a sequence complementary to the single nucleotide variation of the target nucleic acid sequence. As described above, the cleavage of the TO hybridized with the matching template may be induced at a site immediately adjacent to the 5'-end of the 3'-targeting portion of the TO in the 3'-direction, for example, under the induction of the upstream primer extension-dependent cleavage. The 3'-end of the target-dependent fragment has a nucleotide complementary to the single nucleotide variation. The target-dependent fragment is hybridized with the first form of FHO having a capturing portion comprising a sequence corresponding to the nucleotide variation and is extended to form a first duplex, thereby providing a target signal.

If the same TO is hybridized with a mismatching template having the same sequence for the matching template except for a single nucleotide variation, the TO may be cleaved at a site two nucleotides in the 3'-direction apart from the 5'-end of the 3'-targeting portion of the TO. The 3'-end of the target-dependent fragment has a nucleotide that is further cleaved in addition to the nucleotide complementary to the single nucleotide variation. When the site at which the first form of FHO is hybridized with the additionally cleaved nucleotide is designed to have a sequence that is non-complementary to the additionally cleaved nucleotide, the 3'-end of the target-dependent fragment is not hybridized with the first form of FHO, and under controlled conditions, does not eventually produce an extended strand. Even if the target-dependent fragment is extended to form the first duplex, the duplex has a Tm value different from that of the duplex by the hybridization between the TO and the mismatching template.

According to an embodiment, the cleavage site of the TO having a sequence complementary to the nucleotide variation in a part of the 5'-end of the 3'-targeting portion of the TO varies depending on the hybridization with a matching template or a mismatching template, whereby the target-dependent fragments released from the two hybridization events have different sequences, specifically different sequences at a part of the 3'-end thereof, more preferably at the 3'-end thereof.

According to an embodiment, the matching template and the mismatching template may be distinguished by the selection of the nucleotide sequence of the first form of FHO with taking account of the difference in a part of the 3'-end of the target-dependent fragment.

According to an embodiment, the target nucleic acid sequence used in the present disclosure is a pre-amplified nucleic acid sequence. The use of pre-amplified nucleic acid sequence significantly increases the sensitivity and specificity of the target detection of the present disclosure.

According to an embodiment, the method is carried out in the presence of a downstream primer.

An advantage of the present disclosure is that at least two target nucleic acid sequences may be simultaneously detected (multiplexed).

According to an embodiment, the method is carried out to detect at least two (more specifically, at least three, and even more specifically, at least five) target nucleic acid sequences.

According to an embodiment, the method is carried out to detect at least two (more specifically at least three, and even more specifically at least five) target nucleic acid sequences; the upstream oligonucleotide comprises at least two (more specifically at least three, and even more specifically at least five) oligonucleotides, the TO comprises at least two (more specifically at least three, and even more specifically at least five) TOs, and the FHO comprises at least one (specifically at least two, more specifically at least three, and even more specifically at least five)FHO. Optionally, when an SO, HO, and/or IO are used, the SO, HO, and/or IO may comprise at least two types (more specifically at least three types, and even more specifically at least five types). When at least two target nucleic acid sequences are present, the method provides at least two target signals corresponding to at least two target nucleic acid sequences.

At least two tagging portions of the TO (*e.g.,* 5'-tagging portion or 3'-tagging portion) may have the same sequence as each other. For example, when the present disclosure is implemented on screening of target nucleic acid sequences, the tagging portion of the TO may have the same sequence.

Moreover, one type of FHO (specifically, the first form of FHO) may be used to detect multiple target nucleic acid sequences. For example, when the TOs having the same sequence at the tagging portion are used for screening of target nucleic acid sequences, one type of FHO may be used.

According to an embodiment, duplexes (the first duplex or the second duplex) corresponding to at least two target nucleic acid sequences have different Tm values.

According to an embodiment, at least two signals corresponding to at least two target nucleic acid sequences are provided from different types of labels.

According to an embodiment, at least two signals corresponding to at least two target nucleic acid sequences are provided from the same type of label.

According to an embodiment, at least two target signals corresponding to at least two target nucleic acid sequences are provided from the same type of label; duplexes corresponding to at least two target nucleic acid sequences have different Tm values.

As used herein, the term "different types of labels" refers to labels producing detectable signals of different characteristics. For example, FAM and TAMRA as fluorescent reporter labels are considered as different types of labels because their excitation and emission wavelengths are different from each other.

In order to simultaneously detect at least two target nucleic acid sequences by a melting curve analysis, the present disclosure may be implemented to detect at least two target nucleic acid sequences even if one type of label (*e.g.,* FAM) is used when duplexes corresponding to at least two target nucleic acid sequences have different Tm values.

### Detection of Target Using Immobilized Oligonucleotide in Solid Phase

A remarkable advantage of the present disclosure is that the detection of the target nucleic acid sequence is effective even in a solid phase such as a microarray.

In an embodiment, the first form of FHO or the second form of FHO is immobilized on a solid substrate.

According to an embodiment of the solid phase method, the FHO is immobilized on the solid substrate through the 5'-end or 3'-end thereof.

For the solid phase reaction, the FHO is immobilized either directly or indirectly (specifically, indirectly) to the surface of the solid substrate through the 5'-end or 3'-end (specifically, 3'-end). In addition, the FHO may be immobilized on the surface of the solid substrate in a covalent or non-covalent manner. When the immobilized FHO is indirectly immobilized on the solid substrate, an appropriate linker is used. The linker used in the present disclosure may comprise any linker used to secure the probe on the surface of the solid substrate. For example, an alkyl or aryl compound having an amine group, or an alkyl or aryl compound having a thiol group may be used for FHO immobilization as a linker. In addition, the poly (T) tail or the poly (A) tail may be used as the linker.

According to an embodiment of the solid phase method, the solid substrate used in the present disclosure is a microarray. Any microarray known in the art may be used as the microarray for providing the reaction environment in the present disclosure. All processes of the present disclosure, *i.e*., the hybridization with a target nucleic acid sequence, cleavage, extension, hybridization, extension, fluorescence detection (or fluorescence detection upon melting or post-melting hybridization), are carried out in a microarray. The FHO immobilized on the microarray serves as a hybridizable array element. Solid substrates for fabricating microarrays include, but are not limited to, metals (*e.g.,* gold, an alloy of gold and copper, aluminum,), metal oxides, glass, ceramics, quartz, silicon, semiconductors, Si/SiO₂ wafers, germanium, gallium arsenide, carbon, carbon nanotubes, polymers (*e.g*., polystyrene, polyethylene, polypropylene, and polyacrylamide), Sepharose, agarose, and colloids. The plurality of immobilized FHOs of the present disclosure may be immobilized on one or more addressable portions on the solid substrate, and the solid substrate may comprise 2-1,000,000 addressable portions. The immobilized FHOs may be fabricated to produce an array or array for a particular application through conventional fabrication techniques such as photolithography, ink-jetting, mechanical microspotting and the like.

According to an embodiment of the solid phase method, the FHO immobilized on the surface of the solid substrate comprises interactive dual labels.

In the present disclosure, the target-dependent fragment is generated by the cleavage of the TO hybridized with the target nucleic acid. This generates an extended strand on the first form of FHO through hybridization and an extension reaction, and generates a first duplex, or is hybridized with the second form of FHO to generate a second duplex.

In an embodiment, when the present disclosure uses the first form of FHO, additional fragments that are extendable on the first form of FHO may be provided by additional 5' nuclease cleavage reactions using additional TO in order to increase the number of extended strands. There may be various ways to generate additional fragments using the additional TO.

In an embodiment, the additional TO comprises (i) a 3'-targeting portion comprising a hybridizing nucleotide sequence that is complementary to the templating portion of the first form of FHO and (ii) a 5'-tagging portion comprising a nucleotide sequence that is non-complementary to the templating portion of the first form of FHO and comprising a sequence that is complementary to the capturing portion of the first form of FHO. In the embodiment, the additional TO is located downstream of the target-dependent fragment that is hybridized with the capturing portion of the first form of FHO. The target-dependent fragment cleaves the additional TO while being extended, and the cleavage generates an additional target-dependent fragment.

In an embodiment, the method uses the additional SO, HO or third form of FHO immobilized on the solid substrate.

According to one embodiment of the solid phase method, the present disclosure uses a signaling oligonucleotide (SO), and the SO is immobilized on the solid substrate through the 5'-end or 3'-end thereof.

According to an embodiment of the solid phase method, the present invention uses a hybridizing oligonucleotide (HO), and the HO is immobilized on the solid substrate through the 5'-end or 3'-end thereof.

According to an embodiment of the solid phase method, the present disclosure uses an IO immobilized on the solid substrate and the first form of FHO may be indirectly immobilized on the solid substrate via the IO. In this case, the first form of FHO further comprises an IO-hybridization portion comprising a hybridizing nucleotide sequence with the IO. In the embodiment, the IO-hybridization portion may be located at the 3'- or 5'-end of the first form of FHO. In addition, when the IO-hybridization portion is located at the 5'-end of the first form of FHO, the first form of FHO may further comprise a blocker portion that prevents the extension reaction of the target-dependent fragment between the templating portion of the first form of FHO and the IO-hybridization portion.

The details of the embodiment that use the first form of FHO further comprising the IO-hybridization portion are referred to WO 2015/057008.

In particular, when the present invention is implemented in a solid phase using a single label, the single label does not require specific features. For the solid phase reaction, any single label may be used.

In another embodiment of the present disclosure, the present disclosure provides a composition for detecting a target nucleic acid sequence, the composition comprising:
(a) a tag oligonucleotide (TO),
   wherein the TO comprises (i) a tagging portion comprising a non-hybridizing nucleotide sequence with the target nucleic acid sequence and (ii) a targeting portion comprising a hybridizing nucleotide sequence with the target nucleic acid sequence,
   wherein the tagging portion comprises a first synthetic unnatural base within 5 nucleotides from the 3'-end of the tagging portion,
   wherein the TO is cleaved depending on the presence of the target nucleic acid sequence to release a target-dependent fragment comprising the tagging portion or a part of the tagging portion of the TO, and
   wherein the target-dependent fragment comprises the first synthetic unnatural base;
(b) a fragment-hybridizing oligonucleotide (FHO),
   wherein the FHO is (i) a first form of FHO comprising, in a 3' to 5' direction, a capturing portion having a hybridizing nucleotide sequence with the target-dependent fragment and a templating portion having a non-hybridizing nucleotide sequence with the target-dependent fragment and the targeting portion of the TO or (ii) a second form of FHO comprising a capturing portion having a hybridizing nucleotide sequence with the target-dependent fragment, and
   wherein the capturing portion of the FHO comprises a second synthetic unnatural base capable of base pairing with the first synthetic unnatural base in the target-dependent fragment; and
(c) an enzyme having nuclease activity.

Since the "composition for detecting the target nucleic acid sequence" of the present disclosure is configured to perform the "method for detecting the target nucleic acid sequence," which is the first aspect of the present disclosure as described above, the common contents among them will not be described again in order to avoid the excessive complexity of the present specification.

In an embodiment, the first synthetic unnatural base is located at a site of 2 to 5 nucleotides apart from the 3'-end of the tagging portion of the TO.

In an embodiment, the tagging portion of the TO further comprises one to five additional synthetic unnatural bases.

In an embodiment, the first synthetic unnatural base and one to five additional synthetic unnatural bases are spaced 1 to 10 nucleotides apart from each other.

In an embodiment, the first synthetic unnatural base is selected from the group consisting of S, Z, V, K, Pa. Pn, Px, Q, MMO2, 5FM, NaM, B, P, J, X, Ds, Dss and SSICS.

In an embodiment, the first synthetic unnatural base is any base of a pair selected from the group consisting of S-B base pair, Z-P base pair, V-J base pair, K-X base pair, PaDs base pair, Pa-Q base pair, Pn-Ds base pair, Pn-Dss base pair, Px-Ds base pair, MMO2-SSICS base pair, SFM-SSICS base pair, and NaM-5SICS base pair may.

In an embodiment, the tagging portion of TO is 5 to 50 nucleotides in length.

In an embodiment, the tagging portion of TO is 9 to 14 nucleotides in length.

In an embodiment, the enzyme having nuclease activity is an enzyme having 5' nuclease activity.

In an embodiment, when the FHO is the first form of FHO, the target-dependent fragment is hybridized with the capturing portion of the FHO and is extended to generate an extended strand comprising an extended sequence complementary to the templating portion of the FHO, thereby inducing the formation of a first duplex between the extended strand and the FHO.

In an embodiment, when the FHO is the second form of FHO, the target-dependent fragment is hybridized with the capturing portion of the FHO to form a second duplex.

In an embodiment, the presence of the extended strand in the first duplex or the presence of the second duplex is indicative of the presence of the target nucleic acid sequence.

In an embodiment, the composition further comprises an upstream primer comprising a hybridizing nucleotide sequence with the target nucleic acid sequence, and the upstream primer is located upstream the 5' direction of the TO.

In an embodiment, the composition further comprises a template-dependent nucleic acid polymerase.

In an embodiment, the template-dependent nucleic acid polymerase is identical to the enzyme having the nuclease activity.

In an embodiment, the TO is a probe.

In an embodiment, the composition further comprises a downstream primer.

All of the compositions of the present disclosure as described herein may optionally comprise reagents, such as buffers, DNA polymerase cofactors, and deoxyribonucleotide-5-triphosphate, required to perform target amplification PCR reactions (*e.g*., PCR reactions). Optionally, the compositions of the present disclosure may also comprise various polynucleotide molecules, reverse transcriptase, various buffers and reagents, and antibodies that inhibit DNA polymerase activity. Also, the composition may comprise reagents essential for conducting positive and negative control reactions. The optimal amount of reagents used in a particular reaction may be easily determined by those skilled in the art who have learned the disclosure of the present specification. Typically, the compositions of the present disclosure are made into separate packaging or compartments comprising the aforementioned components.

Unless otherwise defined herein, all technical and scientific terms used herein have the same meaning as those generally understood by those skilled in the art.

### Modes for the Invention

Hereinafter, the present invention will be described in more detail with reference to Examples. These examples are intended to describe the present invention in more detail, and it will be apparent to those skilled in the art that the scope of the present invention presented in the appended claims is not limited by these examples.

### Examples

### Example: Detection of Target Nucleic Acid Sequence Using TO and FHO Comprising Unnatural Base Pairs

It was confirmed whether the method for detecting a target nucleic acid using a TO and FHO comprising synthetic unnatural base pairs according to the present disclosure exhibits excellent sensitivity and specificity compared to the conventional method for detecting a target nucleic acid using TO and FHO comprising natural base pairs.

The detection of the duplex in step (c) was performed using a PTOCE assay (WO 2012/096523), and a Taq DNA polymerase having 5' nuclease activity was used for the extension of a forward primer and a reverse primer, the cleavage of TO, and the extension of target-dependent fragment.

### 1. Preparation of Target Nucleic Acid Sequence and Oligonucleotides

The gene of *Enterococcus faecalis* (EF) was used as the target nucleic acid. The target nucleic acid sequence is as follows:

### Target nucleic acid sequence:

5'-
CCACAAGTACCATTCGTGCCAGTTTTAAGAAGTGACTGGAAAGGAAATCCAAA
AGAAGTCTTTGAAAAATGTGAAGGTTCTTTAATTTATCCGGTCTTTGTTAAACCT
GCCAATATGGGTTCTAGTGTCGGAATTAGCAAAGCGGAAAATCGTGAAGAATTG
CAAGAAGCATTGGAAGAAGCTTTCCGTTATGATGCCCGAGCAATTGTTGAACA-
3'
(SEQ ID NO: 1)

The target nucleic acid sequence (SEQ ID NO: 1) of the gene of *Enterococcus faecalis* (EF) was inserted into a vector plDTSMART-AMP (GenBank: MN689784.1) to prepare a plasmid DNA. Subsequently, 10-fold serial dilution of 2.0×10⁴ copies/µL of the plasmid DNA was carried out using a TE buffer to prepare plasmid DNA templates having a total of four concentrations (concentration 1: 2.0×10⁴ copies/µL, concentration 2: 2.0×10³ copies/µL, concentration 3: 2.0×10² copies/µL, and concentration 4: 2.0×10¹ copies/µL).

The TO was designed such that a 3'-targeting portion has a hybridizing nucleotide sequence with the target nucleic acid sequence, and a 5'-tagging portion has a non-hybridizing nucleotide sequence with the target nucleic acid sequence and 1 to 3 synthetic unnatural bases comprising a first synthetic unnatural base. The number and sites of synthetic unnatural bases included in the 5'-tagging portion of the TO have been variously designed, and the first synthetic unnatural base was designed to be located within 5 nucleotides from the 3'-end of the 5'-tagging portion. As the synthetic unnatural base included in the 5'-tagging portion, 2-amino-8-(2'-deoxy-β-D-erythro-pentofuranosyl)-imidazo[1,2-*a*]-1,3,5-triazin-4(8*H*)-one (hereinafter, P) was used.

The FHO, which is hybridized with the target-dependent fragment, was designed such that the capturing portion has a hybridizing nucleotide sequence with the target-dependent fragment released from the TO, and the templating portion has a non-hybridizing nucleotide sequence with the 5'-tagging portion and the 3'-targeting portion of the TO.

The target-dependent fragment was designed to comprise a synthetic unnatural base derived from the 5'-tagging portion of the TO, the capturing portion of the FHO was designed to comprise a synthetic unnatural base (*i.e.,* 1 to 3 synthetic unnatural bases comprising a second synthetic unnatural base) to be hybridized with the opposite synthetic unnatural base (*i.e.,* 1 to 3 synthetic unnatural bases comprising a first synthetic unnatural base) in the target-dependent fragment, and 6-amino-5-nitro-3-(1'-β-D-2'-deoxyribofuranosyl)-2(1H)-pyridone (hereinafter, Z). In addition, a quencher molecule (BHQ-1) and a fluorescent reporter molecule (FAM) were linked to the templating portion of the FHO.

The TO and FHO were each blocked at the 3'-end with a carbon spacer to prevent extension by DNA polymerase.

The sequences of the forward primer, the reverse primer, and the TO and FHO comprising the synthetic unnatural base pair used in this example are shown in Table 1 below. The number "X-Y.Y" after the TO and FHO means the number and site of synthetic unnatural bases included in the tagging portion of the TO. Specifically, X represents the number of synthetic unnatural bases, and Y represents a site from the 3'-end of the 5'-tagging portion. For example, TO 1-1 means that one synthetic unnatural base is included at a first nucleotide site from the 3'-end. FHO 1-1 refers to FHO to be hybridized with a target-dependent fragment which is released by the cleavage of the TO 1-1. As another example, TO 3-3.7.9 means that three synthetic unnatural bases are included at the third, seventh, and ninth nucleotide sites from the 3'-end. FHO 3-3.7.9 refers to FHO to be hybridized with the target-dependent fragment which is released by the cleavage of the TO 3-3.7.9.

**[Table 1]**

| Sequences of Oligonucleotides | | |
|---|---|---|
| SEQ ID NO | Oligo name | Sequence (5' to 3') |
| 2 | Forward primer | CCACAAGTACCATTCGTGCC |
| 3 | Reverse primer | TGTTCAACAATTGCTCGRGC |
| 4 | TO 1-1 | CGCATTCTCGT**P**CGTGAAGAATTGCAAGAAGCATTGGA |
| 5 | FHO 1-1 | [BHQ-1]ACGTCGATTCGCCGCGTCGA[FAM- T]AGACGZACGAGAATGCG |
| 6 | TO 1-2 | CGCATTCTCG**P**ACGTGAAGAATTGCAAGAAGCATTGGA |
| 7 | FHO 1-2 | [BHQ-1]ACGTCGATTCGCCGCGTCGA[FAM- T]AGACGTZCGAGAATGCG |
| 8 | TO 2-2.7 | CGCAT**P**CTCG**P**ACGTGAAGAATTGCAAGAAGCATTGGA |
| 9 | FHO 2-2.7 | [BHQ-1]ACGTCGATTCGCCGCGTCGA[FAM- T]AGACGTZCGAGZATGCG |
| 10 | TO 2-3.8 | CGCA**P**TCTC**P**TACGTGAAGAATTGCAAGAAGCATTGGA |
| 11 | FHO 2-3.8 | [BHQ-1]ACGTCGATTCGCCGCGTCGA[FAM- T]AGACGTAZGAGAZTGCG |
| 12 | TO 3-2.5.9 | CGC**P**TTC**P**CG**P**ACGTGAAGAATTGCAAGAAGCATTGGA |
| 13 | FHO 3-2.5.9 | [BHQ-1]ACGTCGATTCGCCGCGTCGA[FAM- T]AGACGTZCGZGAAZGCG |
| 14 | TO 3-3.7.9 | CGC**P**T**P**CTC**P**TACGTGAAGAATTGCAAGAAGCATTGGA |
| 15 | FHO 3-3.7.9 | [BHQ-1]ACGTCGATTCGCCGCGTCGA[FAM- |
| | | T]AGACGTAZGAGZAZGCG |
| 16 | TO Natural base | CGCATTCTCGTACGTGAAGAATTGCAAGAAGCATTGGA |
| 17 | FHO Natural base | [BHQ-1]ACGTCGATTCGCCGCGTCGA[FAM- T]AGACGTACGAGAATGCG |

| | | |
|---|---|---|
| (The underlined letters indicate the 5'-tagging portion of TO, and **the bold letters** indicate the synthetic unnatural bases.) | | |

### 2. Real-time PCR

First, 24 tubes were prepared, and a reaction mixture with a total of 20 µL comprising (i) 5 µL of plasmid DNA templates (concentration 1 to concentration 4) prepared in Example 1-1, (ii) 5 pmole each of the forward primer (SEQ ID NO: 2) and reverse primer (SEQ ID NO: 3), (iii) 4 pmole each of the TO (SEQ ID NO: 4, 6, 8, 10, 12, or 14) and FHO (SEQ ID NO: 5, 7, 9, 11, 13, or 15) comprising the unnatural base pair (P-Z), and (iv) 5 µL of EM 1 (Cat No. SD10245Z, Seegene, Inc.) was prepared in each tube. 24 types of reaction mixtures were prepared through a combination of (i) a total of 4 concentrations of plasmid DNA and (iii) a total of 6 types of TO and FHO pairs, and the reaction mixtures were used to carry out a real-time PCR reaction.

The tube containing the reaction mixture was placed in a real-time Thermocycler (CFX96, Bio-Rad). The reaction mixture was denatured at 95°C for 15 minutes and then subjected to 45 repetitions of a cycle of 10 seconds at 95°C, 60 seconds 60°C, and 10 seconds at 72°C. The detection of the signal was measured at 60°C every cycle. Ct values were calculated by Auto calculated single threshold, and all experiments were performed in duplicate to obtain an average Ct value.

A comparative control reaction was also performed under the same conditions as described above by using the TO (SEQ ID NO: 16) and FHO (SEQ ID NO: 17) including natural base pairs instead of the TO and FHO including the unnatural base pairs.

A negative control reaction was also performed under the same conditions as described above by using a reaction mixture in which 5 µL of distilled water was added instead of the plasmid DNA template.

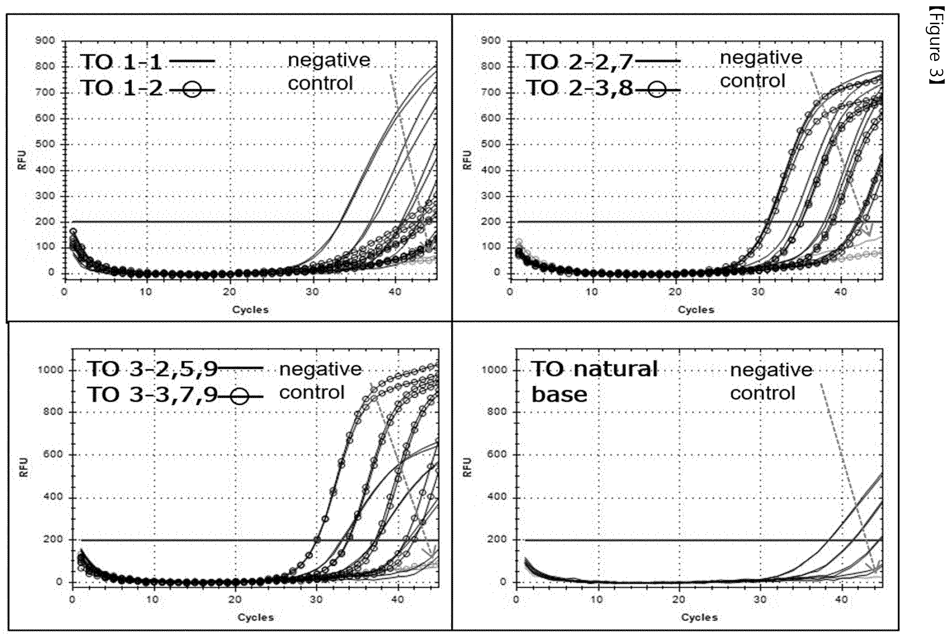

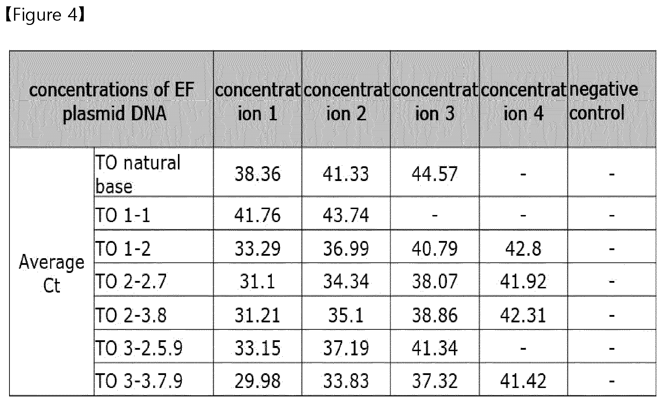

As a result, as shown in FIGS. 3 and 4, it was confirmed that when one or more synthetic unnatural bases are included within the second to fifth nucleotides from the 3'-end of the 5'-tagging portion of the TO, it is possible to detect a target nucleic acid sequence having a lower concentration than that of TO comprising only natural bases. In addition, when the target nucleic acid sequence having the same concentration is detected, it is confirmed that there is an effect of reducing the Ct by 3.2 to 7.2. These results mean that inclusion of one or more synthetic unnatural bases are included within the second to fifth nucleotides from the 3'-end of the TO improves the sensitivity and specificity.

On the other hand, inclusion of the synthetic unnatural base at a site of the first nucleotide from the 3'-end of the TO showed low sensitivity and increased Ct value compared to the TO comprising the natural bases.

These results were estimated to be due to, when the synthetic unnatural base is present at the site of the first nucleotide from the 3'-end, (i) a phenomenon that the cleavage efficiency is reduced due to the unnatural base located at the cleavage site of nuclease, (ii) a phenomenon that the extension efficiency is reduced because the unnatural base located at the 3'-end of the target-dependent fragment hybridized with the FHO, *i.e.,* at the initiation site of the extension of the polymerase is not easily recognized as a general substrate in the extension reaction of the polymerase, and (iii) a phenomenon that the synthetic unnatural base at the site of the first nucleotide from the 3'-end exhibits stronger repulsive force against the opposite natural base in the target nucleic acid sequence compared to the repulsive force between natural bases, and thus the 5'-end of the targeting portion separated from the target nucleic acid sequence together with the 3'-tagging portion (*i.e.,* the synthetic unnatural base at the site of the first nucleotide from the 3'-end is not hybridized with the target nucleic acid sequence), therefore, the TO cleavage site by the enzyme having nuclease activity is changed or the sequence at the 3'-end of the target-dependent fragment is changed, so that the extension efficiency of the target-dependent fragment hybridized with the FHO is reduced.

Meanwhile, it was confirmed that no signal was detected in the negative control reaction.

## Claims

1. A method for detecting a target nucleic acid sequence, comprising the steps of:
(a) reacting the target nucleic acid sequence with a tag oligonucleotide (TO);
wherein the TO comprises (i) a tagging portion comprising a non-hybridizing nucleotide sequence with the target nucleic acid sequence and (ii) a targeting portion comprising a hybridizing nucleotide sequence with the target nucleic acid sequence;
wherein the tagging portion comprises a first synthetic unnatural base within 5 nucleotides from the 3'-end of the tagging portion;
wherein the reacting involves hybridization of the TO with the target nucleic acid sequence and cleavage of the TO depending on the presence of the target nucleic acid sequence to release a target-dependent fragment comprising the tagging portion or a part of the tagging portion of the TO;
wherein the target-dependent fragment comprises the first synthetic unnatural base;
wherein the cleavage of the TO is performed by an enzyme having nuclease activity;
(b) reacting the target-dependent fragment with a fragment-hybridizing oligonucleotide (FHO) to form a duplex;
wherein the FHO is (i) a first form of FHO comprising, in a 3' to 5' direction, a capturing portion having a hybridizing nucleotide sequence with the target-dependent fragment and a templating portion having a non-hybridizing nucleotide sequence with the target-dependent fragment and the targeting portion of the TO or (ii) a second form of FHO comprising a capturing portion having a hybridizing nucleotide sequence with the target-dependent fragment;
wherein the capturing portion of the FHO comprises a second synthetic unnatural base capable of base pairing with the first synthetic unnatural base in the target-dependent fragment,
(i) when the FHO is the first form of FHO, the target-dependent fragment is hybridized with the capturing portion of the FHO and is extended to generate an extended strand comprising an extension sequence complementary to the templating portion of the FHO, thereby inducing the formation of a first duplex between the extended strand and the FHO, or (ii) when the FHO is the second form of FHO, the target-dependent fragment is hybridized with the capturing portion of FHO to form a second duplex; and
(c) detecting the presence of the extended strand in the first duplex or the presence of the second duplex;
wherein the presence of the extended strand in the first duplex or the presence of the second duplex is indicative of the presence of the target nucleic acid sequence.

2. The method of claim 1, wherein the first synthetic unnatural base is located at a site of 2 to 5 nucleotides apart from the 3'-end of the tagging portion of the TO.

3. The method of claim 1, wherein the tagging portion of the TO further comprises 1 to 5 additional synthetic unnatural bases; wherein preferably the first and 1 to 5 additional synthetic unnatural bases are positioned 1 to 10 nucleotides apart from each other.

4. The method of claim 1, wherein the first synthetic unnatural base is selected from the group consisting of S, Z, V, K, Pa. Pn, Px, Q, MMO2, 5FM, NaM, B, P, J, X, Ds, Dss and 5SICS.

5. The method of claim 1, wherein the tagging portion of TO is 5 to 50 nucleotides in length; wherein preferably the tagging portion of the TO is 9 to 14 nucleotides in length.

6. The method of claim 1, wherein the enzyme having nuclease activity is an enzyme having 5' nuclease activity.

7. The method of claim 1, wherein the method is performed in the presence of an upstream primer comprising a hybridizing nucleotide sequence with the target nucleic acid sequence, and the upstream primer is located upstream the 5' direction of the TO; wherein preferably the step (a) comprises a reaction in which the upstream primer is extended by a template-dependent nucleic acid polymerase to generate an extended strand, and the extended strand induces cleavage of the TO by the enzyme having nuclease activity, thereby releasing the target-dependent fragment comprising the tagging portion or a part of the tagging portion of the TO; wherein more preferably the template-dependent polymerase is identical to the enzyme having the nuclease activity.

8. The method of claim 1, wherein the first duplex provides a detectable signal by (i) at least one label linked to the target-dependent fragment and/or the first form of FHO, (ii) a label incorporated into the first duplex during the extension reaction, (iii) a label incorporated into the first duplex during the extension reaction and at least one label linked to the first form of FHO or (iv) an intercalating label, and the presence of the extended strand in step (c) is detected by measuring a signal provided from the first duplex.

9. The method of claim 1, wherein when the method forms the second duplex, a reporter molecule and a first quencher molecule are linked to the TO, and a second quencher molecule is linked to the second form of FHO, the TO is cleaved depending on the presence of the target nucleic acid sequence during the step (a) to release a target-dependent fragment comprising the reporter molecule, the reporter molecule comprised in the target-dependent fragment and the second quencher molecule linked to the second form of FHO provide a signal detectable by the formation and dissociation of a second duplex between the target-dependent fragment and the second form of FHO according to temperature and the presence of the second duplex in step (c) is detected by measuring a signal provided from the second duplex.

10. The method of claim 1, wherein a Tm value of the first or second duplex is 0.5 to 10°C higher than a Tm value of a duplex in which the first and second synthetic unnatural bases comprised in the first or second duplex are substituted with natural bases.

11. A composition for detecting a target nucleic acid sequence, comprising:
(a) a tag oligonucleotide (TO);
wherein the TO comprises (i) a tagging portion comprising a non-hybridizing nucleotide sequence with the target nucleic acid sequence and (ii) a targeting portion comprising a hybridizing nucleotide sequence with the target nucleic acid sequence;
wherein the tagging portion comprises a first synthetic unnatural base within 5 nucleotides from the 3'-end of the tagging portion;
wherein preferably the tagging portion of TO is 5 to 50 nucleotides in length;
wherein the TO is cleaved depending on the presence of the target nucleic acid sequence to release a target-dependent fragment comprising the tagging portion or a part of the tagging portion of the TO;
wherein the target-dependent fragment comprises the first synthetic unnatural base;
(b) a fragment-hybridizing oligonucleotide (FHO);
wherein the FHO is (i) a first form of FHO comprising, in a 3' to 5' direction, a capturing portion having a hybridizing nucleotide sequence with the target-dependent fragment and a templating portion having a non-hybridizing nucleotide sequence with the target-dependent fragment and the targeting portion of the TO or (ii) a second form of FHO comprising a capturing portion having a hybridizing nucleotide sequence with the target-dependent fragment;
wherein the capturing portion of the FHO comprises a second synthetic unnatural base capable of base pairing with the first synthetic unnatural base in the target-dependent fragment; and
(c) an enzyme having nuclease activity;
wherein preferably the enzyme having nuclease activity is an enzyme having 5' nuclease activity.

12. The composition of claim 11, wherein the first synthetic unnatural base is located at a site of 2 to 5 nucleotides apart from the 3'-end of the tagging portion of the TO.

13. The composition of claim 11, wherein the tagging portion of the TO further comprises 1 to 5 additional synthetic unnatural bases; wherein preferably the first and 1 to 5 additional synthetic unnatural bases are positioned 1 to 10 nucleotides apart from each other.

14. The composition of claim 11, wherein the first synthetic unnatural base is selected from the group consisting of S, Z, V, K, Pa. Pn, Px, Q, MMO2, 5FM, NaM, B, P, J, X, Ds, Dss and SSICS.

15. The composition of claim 11, (i) when the FHO is the first form of FHO, the target-dependent fragment is hybridized with the capturing portion of the FHO and is extended to generate an extended strand comprising an extension sequence complementary to the templating portion of the FHO, thereby inducing the formation of a first duplex between the extended strand and the FHO; and (ii) when the FHO is the second form of FHO, the target-dependent fragment is hybridized with the capturing portion of FHO to form a second duplex.

## Patentansprüche

1. Verfahren zum Nachweis einer Zielnukleinsäuresequenz, umfassend die folgenden Schritte:
(a) Umsetzen der Zielnukleinsäuresequenz mit einem Markeroligonukleotid (TO);
wobei das TO (i) einen Markierungsbereich, der eine nicht hybridisierende Nukleotidsequenz mit der Zielnukleinsäuresequenz umfasst, und (ii) einen Zielbereich umfasst, der eine hybridisierende Nukleotidsequenz mit der Zielnukleinsäuresequenz umfasst;
wobei der Markierungsbereich eine erste synthetische nichtnatürliche Base innerhalb von 5 Nukleotiden vom 3'-Ende des Markierungsbereichs umfasst; wobei das Umsetzen eine Hybridisierung des TO mit der Zielnukleinsäuresequenz und eine Spaltung des TO in Abhängigkeit von der Anwesenheit der Zielnukleinsäuresequenz umfasst, um ein zielabhängiges Fragment freizusetzen, das den Markierungsbereich oder einen Teil des Markierungsbereichs des TO umfasst;
wobei das zielabhängige Fragment die erste synthetische nichtnatürliche Base umfasst; wobei die Spaltung des TO durch ein Enzym mit Nukleaseaktivität durchgeführt wird;
(b) Umsetzen des zielabhängigen Fragments mit einem fragmenthybridisierenden Oligonukleotid (FHO) zur Bildung eines Duplex;
wobei das FHO (i) eine erste Form des FHO, die in einer 3'-zu-5'-Richtung einen Capturebereich mit einer hybridisierenden Nukleotidsequenz mit dem zielabhängigen Fragment und einen Templatbereich mit einer nicht hybridisierenden Nukleotidsequenz mit dem zielabhängigen Fragment und dem Zielbereich des TO umfasst, oder (ii) eine zweite Form des FHO ist, die einen Capturebereich mit einer hybridisierenden Nukleotidsequenz mit dem zielabhängigen Fragment umfasst;
wobei der Capturebereich des FHO eine zweite synthetische nichtnatürliche Base umfasst, die zur Basenpaarung mit der ersten synthetischen nichtnatürlichen Base in dem zielabhängigen Fragment fähig ist,
(i) wenn das FHO die erste Form des FHO ist, das zielabhängige Fragment mit dem Capturebereich des FHO hybridisiert wird und verlängert wird, um einen verlängerten Strang zu erzeugen, der eine zum Templatbereich des FHO komplementäre Verlängerungssequenz umfasst, wodurch die Bildung eines ersten Duplex zwischen dem verlängerten Strang und dem FHO induziert wird, oder (ii) wenn das FHO die zweite Form des FHO ist, das zielabhängige Fragment mit dem Capturebereich des FHO hybridisiert wird, um einen zweiten Duplex zu bilden; und
(c) Nachweisen der Anwesenheit des verlängerten Strangs in dem ersten Duplex oder der Anwesenheit des zweiten Duplex; wobei die Anwesenheit des verlängerten Strangs in dem ersten Duplex oder die Anwesenheit des zweiten Duplex auf die Anwesenheit der Zielnukleinsäuresequenz hinweist.

2. Verfahren nach Anspruch 1, wobei die erste synthetische nichtnatürliche Base an einer Stelle angeordnet ist, die 2 bis 5 Nukleotide vom 3'-Ende des Markierungsbereichs des TO entfernt ist.

3. Verfahren nach Anspruch 1, wobei der Markierungsbereich des TO weiter 1 bis 5 zusätzliche synthetische nichtnatürliche Basen umfasst; wobei bevorzugt die erste und 1 bis 5 zusätzliche synthetische nichtnatürliche Basen in einem Abstand von 1 bis 10 Nukleotiden voneinander positioniert sind.

4. Verfahren nach Anspruch 1, wobei die erste synthetische nichtnatürliche Base ausgewählt ist aus der Gruppe bestehend aus S, Z, V, K, Pa. Pn, Px, Q, MMO2, 5FM, NaM, B, P, J, X, Ds, Dss und 5SICS.

5. Verfahren nach Anspruch 1, wobei der Markierungsbereich von TO 5 bis 50 Nukleotide lang ist; wobei bevorzugt der Markierungsbereich des TO 9 bis 14 Nukleotide lang ist.

6. Verfahren nach Anspruch 1, wobei das Enzym mit Nukleaseaktivität ein Enzym mit 5'-Nukleaseaktivität ist.

7. Verfahren nach Anspruch 1, wobei das Verfahren in Gegenwart eines Upstream-Primers durchgeführt wird, der eine hybridisierende Nukleotidsequenz mit der Zielnukleinsäuresequenz umfasst, und der Upstream-Primer stromaufwärts in der 5'-Richtung des TO angeordnet ist; wobei bevorzugt der Schritt (a) eine Reaktion umfasst, in der der Upstream-Primer durch eine templatabhängige Nukleinsäurepolymerase verlängert wird, um einen verlängerten Strang zu erzeugen, und der verlängerte Strang die Spaltung des TO durch das Enzym mit Nukleaseaktivität induziert, wodurch das zielabhängige Fragment freigesetzt wird, das den Markierungsbereich oder einen Teil des Markierungsbereichs des TO umfasst; wobei bevorzugter die templatabhängige Polymerase mit dem Enzym mit Nukleaseaktivität identisch ist.

8. Verfahren nach Anspruch 1, wobei der erste Duplex ein nachweisbares Signal bereitstellt durch (i) mindestens eine Markierung, die mit dem zielabhängigen Fragment und/oder der ersten Form des FHO verknüpft ist, (ii) eine Markierung, die während der Verlängerungsreaktion in den ersten Duplex eingebaut wird, (iii) eine Markierung, die während der Verlängerungsreaktion in den ersten Duplex eingebaut wird, und mindestens eine Markierung, die mit der ersten Form des FHO verknüpft ist, oder (iv) eine interkalierende Markierung, und die Anwesenheit des verlängerten Strangs in Schritt (c) durch Messen eines von dem ersten Duplex bereitgestellten Signals nachgewiesen wird.

9. Verfahren nach Anspruch 1, wobei, wenn das Verfahren den zweiten Duplex bildet, ein Reportermolekül und ein erstes Quenchermolekül mit dem TO verknüpft sind und ein zweites Quenchermolekül mit der zweiten Form des FHO verknüpft ist, das TO während des Schritts (a) in Abhängigkeit von der Anwesenheit der Zielnukleinsäuresequenz gespalten wird, um ein zielabhängiges Fragment freizusetzen, das das Reportermolekül umfasst, das in dem zielabhängigen Fragment umfasste Reportermolekül und das mit der zweiten Form des FHO verknüpfte zweite Quenchermolekül ein durch die Bildung und Dissoziation eines zweiten Duplex zwischen dem zielabhängigen Fragment und der zweiten Form des FHO gemäß der Temperatur nachweisbares Signal bereitstellen und die Anwesenheit des zweiten Duplex in Schritt (c) durch Messen eines von dem zweiten Duplex bereitgestellten Signals nachgewiesen wird.

10. Verfahren nach Anspruch 1, wobei ein Tm-Wert des ersten oder zweiten Duplex 0,5 bis 10 °C höher ist als ein Tm-Wert eines Duplex, in dem die in dem ersten oder zweiten Duplex umfassten ersten und zweiten synthetischen nichtnatürlichen Basen durch natürliche Basen substituiert sind.

11. Zusammensetzung zum Nachweis einer Zielnukleinsäuresequenz, umfassend:
(a) ein Markeroligonukleotid (TO);
wobei das TO (i) einen Markierungsbereich, der eine nicht hybridisierende Nukleotidsequenz mit der Zielnukleinsäuresequenz umfasst, und (ii) einen Zielbereich, der eine hybridisierende Nukleotidsequenz mit der Zielnukleinsäuresequenz umfasst, umfasst;
wobei der Markierungsbereich eine erste synthetische nichtnatürliche Base innerhalb von 5 Nukleotiden vom 3'-Ende des Markierungsbereichs umfasst;
wobei bevorzugt der Markierungsbereich von TO 5 bis 50 Nukleotide lang ist;
wobei das TO in Abhängigkeit von der Anwesenheit der Zielnukleinsäuresequenz gespalten wird, um ein zielabhängiges Fragment freizusetzen, das den Markierungsbereich oder einen Teil des Markierungsbereichs des TO umfasst;
wobei das zielabhängige Fragment die erste synthetische nichtnatürliche Base umfasst;
(b) ein fragmenthybridisierendes Oligonukleotid (FHO);
wobei das FHO (i) eine erste Form des FHO, die in einer 3'-zu-5'-Richtung einen Capturebereich mit einer hybridisierenden Nukleotidsequenz mit dem zielabhängigen Fragment und einen Templatbereich mit einer nicht hybridisierenden Nukleotidsequenz mit dem zielabhängigen Fragment und dem Zielbereich des TO umfasst, oder (ii) eine zweite Form des FHO ist, die einen Capturebereich mit einer hybridisierenden Nukleotidsequenz mit dem zielabhängigen Fragment umfasst;
wobei der Capturebereich des FHO eine zweite synthetische nichtnatürliche Base umfasst, die zur Basenpaarung mit der ersten synthetischen nichtnatürlichen Base in dem zielabhängigen Fragment fähig ist; und
(c) ein Enzym mit Nukleaseaktivität; wobei bevorzugt das Enzym mit Nukleaseaktivität ein Enzym mit 5'-Nukleaseaktivität ist.

12. Zusammensetzung nach Anspruch 11, wobei die erste synthetische nichtnatürliche Base an einer Stelle angeordnet ist, die 2 bis 5 Nukleotide vom 3'-Ende des Markierungsbereichs des TO entfernt ist.

13. Zusammensetzung nach Anspruch 11, wobei der Markierungsbereich des TO weiter 1 bis 5 zusätzliche synthetische nichtnatürliche Basen umfasst; wobei bevorzugt die erste und 1 bis 5 zusätzliche synthetische nichtnatürliche Basen in einem Abstand von 1 bis 10 Nukleotiden voneinander positioniert sind.

14. Zusammensetzung nach Anspruch 11, wobei die erste synthetische nichtnatürliche Base ausgewählt ist aus der Gruppe bestehend aus S, Z, V, K, Pa. Pn, Px, Q, MMO2, 5FM, NaM, B, P, J, X, Ds, Dss und 5SICS.

15. Zusammensetzung nach Anspruch 11, (i) wenn das FHO die erste Form des FHO ist, wird das zielabhängige Fragment mit dem Capturebereich des FHO hybridisiert und verlängert, um einen verlängerten Strang zu erzeugen, der eine zum Templatbereich des FHO komplementäre Verlängerungssequenz umfasst, wodurch die Bildung eines ersten Duplex zwischen dem verlängerten Strang und dem FHO induziert wird; und (ii) wenn das FHO die zweite Form des FHO ist, wird das zielabhängige Fragment mit dem Capturebereich von FHO hybridisiert, um einen zweiten Duplex zu bilden.

## Revendications

1. Procédé de détection d'une séquence d'acide nucléique cible, comprenant les étapes consistant à :
(a) faire réagir la séquence d'acide nucléique cible avec un oligonucléotide marqueur (TO) ;
dans lequel le TO comprend (i) une portion de marquage comprenant une séquence nucléotidique non-hybridante avec la séquence d'acide nucléique cible et (ii) une portion de ciblage comprenant une séquence nucléotidique hybridante avec la séquence d'acide nucléique cible ; dans lequel la portion de marquage comprend une première base non-naturelle synthétique à l'intérieur de 5 nucléotides à partir de l'extrémité 3' de la portion de marquage ;
dans lequel la réaction implique l'hybridation du TO avec la séquence d'acide nucléique cible et le clivage du TO en fonction de la présence de la séquence d'acide nucléique cible pour libérer un fragment dépendant de la cible comprenant la portion de marquage ou une partie de la portion de marquage du TO ;
dans lequel le fragment dépendant de la cible comprend la première base non-naturelle synthétique ; dans lequel le clivage du TO est réalisé par une enzyme présentant une activité nucléase ; (b) faire réagir le fragment dépendant de la cible avec un oligonucléotide s'hybridant au fragment (FHO) pour former un duplex ;
dans lequel le FHO est (i) une première forme de FHO comprenant, dans une direction 3' vers 5', une portion de capture présentant une séquence nucléotidique hybridante avec le fragment dépendant de la cible et une portion matrice présentant une séquence nucléotidique non-hybridante avec le fragment dépendant de la cible et la portion de ciblage du TO ou (ii) une deuxième forme de FHO comprenant une portion de capture présentant une séquence nucléotidique hybridante avec le fragment dépendant de la cible ;
dans lequel la portion de capture du FHO comprend une deuxième base non-naturelle synthétique apte à s'apparier avec la première base non-naturelle synthétique dans le fragment dépendant de la cible,
(i) lorsque le FHO est la première forme de FHO, le fragment dépendant de la cible est hybridé avec la portion de capture du FHO et est étendu de manière à générer un brin étendu comprenant une séquence d'extension complémentaire à la portion matrice du FHO, induisant ainsi la formation d'un premier duplex entre le brin étendu et le FHO, ou (ii) lorsque le FHO est la deuxième forme de FHO, le fragment dépendant de la cible est hybridé avec la portion de capture du FHO pour former un second duplex ; et
(c) détecter la présence du brin étendu dans le premier duplex ou la présence du second duplex ;
dans lequel la présence du brin étendu dans le premier duplex ou la présence du second duplex est indicative de la présence de la séquence d'acide nucléique cible.

2. Procédé selon la revendication 1, dans lequel la première base non-naturelle synthétique est située à un site espacé de 2 à 5 nucléotides de l'extrémité 3' de la portion de marquage du TO.

3. Procédé selon la revendication 1, dans lequel la portion de marquage du TO comprend en outre 1 à 5 bases non-naturelles synthétiques supplémentaires ; dans lequel, de préférence, la première base non-naturelle synthétique et les 1 à 5 bases non-naturelles synthétiques supplémentaires sont positionnées à 1 à 10 nucléotides l'une de l'autre.

4. Procédé selon la revendication 1, dans lequel la première base non-naturelle synthétique est choisie dans le groupe constitué de S, Z, V, K, Pa, Pn, Px, Q, MMO2, 5FM, NaM, B, P, J, X, Ds, Dss et 5SICS.

5. Procédé selon la revendication 1, dans lequel la portion de marquage du TO a une longueur de 5 à 50 nucléotides ; dans lequel, de préférence, la portion de marquage du TO a une longueur de 9 à 14 nucléotides.

6. Procédé selon la revendication 1, dans lequel l'enzyme présentant une activité nucléase est une enzyme présentant une activité nucléase 5'.

7. Procédé selon la revendication 1, dans lequel le procédé est mis en œuvre en présence d'une amorce amont comprenant une séquence nucléotidique hybridante avec la séquence d'acide nucléique cible, et l'amorce amont est située en amont de la direction 5' du TO ; dans lequel, de préférence, l'étape (a) comprend une réaction dans laquelle l'amorce amont est étendue par une polymérase d'acide nucléique dépendante de matrice pour générer un brin étendu, et le brin étendu induit le clivage du TO par l'enzyme présentant une activité nucléase, libérant ainsi le fragment dépendant de la cible comprenant la portion de marquage ou une partie de la portion de marquage du TO ; dans lequel, de manière plus préférée, la polymérase dépendante de matrice est identique à l'enzyme présentant l'activité nucléase.

8. Procédé selon la revendication 1, dans lequel le premier duplex fournit un signal détectable par (i) au moins une étiquette liée au fragment dépendant de la cible et/ou à la première forme de FHO, (ii) une étiquette incorporée dans le premier duplex pendant la réaction d'extension, (iii) une étiquette incorporée dans le premier duplex pendant la réaction d'extension et au moins un marqueur lié à la première forme de FHO ou (iv) une étiquette d'intercalation, et la présence du brin étendu à l'étape (c) est détectée par la mesure d'un signal fourni par le premier duplex.

9. Procédé selon la revendication 1, dans lequel, lorsque le procédé forme le second duplex, une molécule rapporteur et une première molécule extinctrice sont liées au TO, et une deuxième molécule extinctrice est liée à la deuxième forme de FHO, le TO est clivé en fonction de la présence de la séquence d'acide nucléique cible pendant l'étape (a) pour libérer un fragment dépendant de la cible comprenant la molécule rapporteur, la molécule rapporteur comprise dans le fragment dépendant de la cible et la deuxième molécule extinctrice liée à la deuxième forme de FHO fournissent un signal détectable par la formation et la dissociation d'un second duplex entre le fragment dépendant de la cible et la deuxième forme de FHO selon la température et la présence du second duplex à l'étape (c) est détectée par la mesure d'un signal fourni par le second duplex.

10. Procédé selon la revendication 1, dans lequel une valeur de Tm du premier ou du second duplex est supérieure de 0,5 à 10 °C à une valeur de Tm d'un duplex dans lequel les première et deuxième bases non-naturelles synthétiques comprises dans le premier ou le second duplex sont substituées par des bases naturelles.

11. Composition de détection d'une séquence d'acide nucléique cible, comprenant :
(a) un oligonucléotide marqueur (TO) ;
dans laquelle le TO comprend (i) une portion de marquage comprenant une séquence nucléotidique non-hybridante avec la séquence d'acide nucléique cible et (ii) une portion de ciblage comprenant une séquence nucléotidique hybridante avec la séquence d'acide nucléique cible ;
dans laquelle la portion de marquage comprend une première base non-naturelle synthétique à l'intérieur de 5 nucléotides à partir de l'extrémité 3' de la portion de marquage ; dans laquelle, de préférence, la portion de marquage du TO a une longueur de 5 à 50 nucléotides ;
dans laquelle le TO est clivé en fonction de la présence de la séquence d'acide nucléique cible pour libérer un fragment dépendant de la cible comprenant la portion de marquage ou une partie de la portion de marquage du TO ; dans laquelle le fragment dépendant de la cible comprend la première base non-naturelle synthétique ; (b) un oligonucléotide s'hybridant au fragment (FHO) ;
dans laquelle le FHO est (i) une première forme de FHO comprenant, dans une direction 3' vers 5', une portion de capture présentant une séquence nucléotidique hybridante avec le fragment dépendant de la cible et une portion matrice présentant une séquence nucléotidique non-hybridante avec le fragment dépendant de la cible et la portion de ciblage du TO ou (ii) une deuxième forme de FHO comprenant une portion de capture présentant une séquence nucléotidique hybridante avec le fragment dépendant de la cible ;
dans laquelle la portion de capture du FHO comprend une deuxième base non-naturelle synthétique apte à s'apparier avec la première base non-naturelle synthétique dans le fragment dépendant de la cible ; et
(c) une enzyme présentant une activité nucléase ;
dans laquelle, de préférence, l'enzyme présentant une activité nucléase est une enzyme présentant une activité nucléase 5'.

12. Composition selon la revendication 11, dans laquelle la première base non-naturelle synthétique est située à un site espacé de 2 à 5 nucléotides de l'extrémité 3' de la portion de marquage du TO.

13. Composition selon la revendication 11, dans laquelle la portion de marquage du TO comprend en outre 1 à 5 bases non-naturelles synthétiques supplémentaires ; dans laquelle, de préférence, la première base non-naturelle synthétique et les 1 à 5 bases non-naturelles synthétiques supplémentaires sont positionnées à 1 à 10 nucléotides l'une de l'autre.

14. Composition selon la revendication 11, dans laquelle la première base non-naturelle synthétique est choisie dans le groupe constitué de S, Z, V, K, Pa, Pn, Px, Q, MMO2, 5FM, NaM, B, P, J, X, Ds, Dss et 5SICS.

15. Composition selon la revendication 11, (i) lorsque le FHO est la première forme de FHO, le fragment dépendant de la cible est hybridé avec la portion de capture du FHO et est étendu de manière à générer un brin étendu comprenant une séquence d'extension complémentaire à la portion matrice du FHO, induisant ainsi la formation d'un premier duplex entre le brin étendu et le FHO ; et (ii) lorsque le FHO est la deuxième forme de FHO, le fragment dépendant de la cible est hybridé avec la portion de capture du FHO pour former un second duplex.
